(19) Europäisches Patentamt / European Patent Office / Office européen des brevets

(11) **EP 3 981 787 A1**

(12) **EUROPEAN PATENT APPLICATION**
published in accordance with Art. 153(4) EPC

(43) Date of publication:
**13.04.2022 Bulletin 2022/15**

(21) Application number: 20818627.0

(22) Date of filing: **03.06.2020**

(51) International Patent Classification (IPC):
*C07K 16/22* (2006.01)     *C12N 15/13* (2006.01)
*C12N 15/63* (2006.01)     *A61K 39/395* (2006.01)
*G01N 33/53* (2006.01)     *A61P 9/10* (2006.01)
*A61P 9/12* (2006.01)     *A61P 19/02* (2006.01)
*A61P 27/06* (2006.01)     *A61P 35/00* (2006.01)
*A61P 1/16* (2006.01)     *A61P 3/10* (2006.01)
*A61P 11/00* (2006.01)     *A61P 13/12* (2006.01)
*A61P 17/00* (2006.01)

(52) Cooperative Patent Classification (CPC):
**A61K 39/395; A61P 1/16; A61P 3/10; A61P 9/10;
A61P 9/12; A61P 11/00; A61P 13/12; A61P 17/00;
A61P 19/02; A61P 27/06; A61P 35/00;
C07K 16/22; C12N 15/63; G01N 33/53**

(86) International application number:
**PCT/CN2020/094136**

(87) International publication number:
**WO 2020/244540 (10.12.2020 Gazette 2020/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.06.2019 CN 201910480169**

(71) Applicants:
• **Jiangsu Hengrui Medicine Co., Ltd.
Lianyungang, Jiangsu 222047 (CN)**
• **Shanghai Hengrui Pharmaceutical Co., Ltd.
Shanghai 200245 (CN)**

(72) Inventors:
• **FU, Yayuan
Shanghai 200245 (CN)**
• **MA, Xiaoli
Shanghai 200245 (CN)**
• **GE, Hu
Shanghai 200245 (CN)**
• **TAO, Weikang
Shanghai 200245 (CN)**

(74) Representative: **Regimbeau
20, rue de Chazelles
75847 Paris Cedex 17 (FR)**

(54) **ANTI-CONNECTIVE TISSUE GROWTH FACTOR ANTIBODY AND APPLICATION THEREOF**

(57) Provided are an anti-CTGF antibody comprising variable regions of a light chain and a heavy chain and a use thereof as a drug. The antibody may be used to prepare drugs for treating CTGF-related diseases or conditions.

EP 3 981 787 A1

**Description**

The present application claims the priority of the Chinese patent application No.201910480169.4 (title: Anti-Connective Tissue Growth Factor Antibody and Application Thereof, Priority Date: June 4, 2019).

**FIELD OF THE INVENTION**

The present disclosure belongs to the field of biomedicine, and specifically relates to antibodies binding to Connective Tissue Growth Factor (CTGF) and applications thereof.

**BACKGROUND OF THE INVENTION**

The descriptions herein only provide background information about the present disclosure, and do not necessarily constitute prior art.

The expression of CTGF is induced by members of the Transforming Growth Factor beta (TGFβ) superfamily. This superfamily includes TGFβ-1, -2, and -3, Bone Morphogenetic Protein (BMP)-2, and activin. Many regulators (including dexamethasone, thrombin, Vascular Endothelial Growth Factor (VEGF) and angiotensin II) and environmental stress (including hyperglycemia and hypertension) also induce the expression of CTGF (see, for example, Franklin (1997) Int J Biochem Cell Biol 29:79-89; Wunderlich (2000) Graefes Arch Clin Exp Ophthalmol 238: 910-915; Denton and Abraham (2001) Curr Opin Rheumatol 13: 505-511; and Riewald (2001) Blood 97: 3109-3116; Riser et al. (2000) J Am SocNephrol 11: 25-38; and International Publication WO 00/13706).

The stimulating effect of TGFβon the expression of CTGF is rapid and long-term, and it is not necessary to persistently apply TGFβ (Igarashi et al. (1993) Mol BiolCell 4:637-645). TGFβ activates the transcription through the DNA regulatory elements present in the CTGF promoter, resulting in the enhanced expression of CTGF (Grotendorst et al. (1996) Cell Growth Differ 7: 469-480; Grotendorst and Bradham, US Patent No. 6,069,006; Holmes et al. (2001) J Biol Chem 276: 10594-10601).

The expression of CTGF is upregulated in glomerulonephritis, IgA nephropathy, interfocal and segmental glomerulosclerosis, and diabetic nephropathy (see, for example, Riser et al. (2000) J Am Soc Nephrol 11:25 -38). An increase in the number of cells expressing CTGF has also been observed in chronic tubulointerstitial injury sites, and the level of CTGF is correlated with the degree of injury (Ito et al. (1998) Kidney Int 53:853-861). In addition, in various nephropathy associated with renal parenchymal scarring and sclerosis, the expression of CTGF in glomeruli and tubulointerstitium is also increased. The elevated levels of CTGF are also associated with liver fibrosis, myocardial infarction and pulmonary fibrosis. For example, CTGF is strongly upregulated in cells from biopsies and bronchoalveolar lavage fluid in patients with spontaneous pulmonary fibrosis (IPF) (Ujike et al. (2000) Biochem Biophys Res Commun 277:448-454; Abou -Shady et al. (2000) Liver 20: 296-304; Williams et al. (2000) J Hepatol 32: 754-761). Therefore, CTGF represents an effective therapeutic target in the diseases described above.

However, no effective CTGF-targeting antibody agent is currently used in clinical practice, and there is still a need to develop novel safe and effective CTGF antibody agents.

**SUMMARY OF THE INVENTION**

The present disclosure provides an anti-CTGF antibody. The anti-CTGF antibody includes anti-human CTGF full-length antibodies and antigen-binding fragments thereof.

In some embodiments, the anti-CTGF antibody comprises a heavy chain variable region and a light chain variable region, wherein:

> i) the heavy chain variable region comprises the same HCDR1, HCDR2 and HCDR3 sequences as those of the heavy chain variable region shown in SEQ ID NO: 6, and the light chain variable region comprises the same LCDR1, LCDR2 and LCDR3 sequences as those of the light chain variable region shown in SEQ ID NO: 7; or
> ii) the heavy chain variable region comprises the same HCDR1, HCDR2 and HCDR3 sequences as those of the heavy chain variable region shown in SEQ ID NO: 8, and the light chain variable region comprises the same LCDR1, LCDR2 and LCDR3 sequences as those of the light chain variable region shown in SEQ ID NO: 9.

In some embodiments, the anti-CTGF antibody comprises a heavy chain variable region and a light chain variable region, wherein:

> ii-i) the heavy chain variable region comprises the same HCDR1, HCDR2 and HCDR3 sequences as those of the heavy chain variable region shown in SEQ ID NO: 69, and the light chain variable region comprises the same LCDR1, LCDR2 and LCDR3 sequences as those of the light chain variable region shown in SEQ ID NO: 70; or
ii-ii) the heavy chain variable region comprises the same HCDR1, HCDR2 and HCDR3 sequences as those of the heavy chain variable region shown in SEQ ID NO: 85, and the light chain variable region comprises the same LCDR1, LCDR2 and LCDR3 sequences as those of the light chain variable region shown in SEQ ID NO: 70.

In some embodiments, the anti-CTGF antibody comprises a heavy chain variable region and a light chain variable region, wherein:

iii) the heavy chain variable region comprises HCDR1, HCDR2 and HCDR3 as shown in SEQ ID NO: 10, SEQ ID NO: 11 and SEQ ID NO: 12, respectively, and the light chain variable region comprises LCDR1, LCDR2 and LCDR3 as shown in SEQ ID NO: 13, SEQ ID NO: 14 and SEQ ID NO: 15, respectively; or
iv) the heavy chain variable region comprises HCDR1, HCDR2 and HCDR3 as shown in SEQ ID NO: 16, SEQ ID NO: 17 and SEQ ID NO: 18, respectively, and the light chain variable region comprises LCDR1, LCDR2 and LCDR3 as shown in SEQ ID NO: 19, SEQ ID NO: 20 and SEQ ID NO: 21, respectively.

In some embodiments, the anti-CTGF antibody comprises a heavy chain variable region and a light chain variable region, wherein:

iv-i) the heavy chain variable region comprises HCDR1, HCDR2 and HCDR3 as shown in SEQ ID NO: 71, SEQ ID NO: 72 and SEQ ID NO: 73, respectively, and the light chain variable region comprises LCDR1, LCDR2 and LCDR3 as shown in SEQ ID NO: 74, SEQ ID NO: 75 and SEQ ID NO: 76, respectively; or
iv-ii) the heavy chain variable region comprises HCDR1, HCDR2 and HCDR3 as shown in SEQ ID NO: 102, SEQ ID NO: 103 and SEQ ID NO: 104, respectively, and the light chain variable region comprises LCDR1, LCDR2 and LCDR3 as shown in SEQ ID NO: 74, SEQ ID NO: 75 and SEQ ID NO: 76, respectively.

In some embodiments, the anti-CTGF antibody is a murine antibody, a chimeric antibody, or a humanized antibody.

In some embodiments, the anti-CTGF antibody comprises a heavy chain variable region and a light chain variable region, wherein:

(v-1) the amino acid sequence of the heavy chain variable region has at least 90% sequence identity to SEQ ID NO: 6, 27, 28 or 29; and the amino acid sequence of the light chain variable region has at least 90% sequence identity to SEQ ID NO: 7, 22, 23, 24, 25, or 26;
(vi-1) the amino acid sequence of the heavy chain variable region has at least 90% sequence identity to SEQ ID NO: 8, 33, 34, 35 or 36; and the amino acid sequence of the light chain variable region has at least 90% sequence identity to SEQ ID NO: 9, 30, 31 or 32; or
(vi-i) the amino acid sequence of the heavy chain variable region has at least 90% sequence identity to SEQ ID NO: 69, 81, 82, 83, 84 or 85, and the amino acid sequence of the light chain variable region has at least 90% sequence identity to SEQ ID NO: 70, 77, 78, 79, or 80.

In some embodiments, the anti-CTGF antibody comprises a heavy chain variable region and a light chain variable region, wherein:

(v) the heavy chain variable region has at least 90%, 91%, 92%, 93%, 94%, 95%, 96%, 97%, 98%, 99% or 100% identity to the heavy chain variable region as shown in SEQ ID NO: 6, 27, 28 or 29, and the light chain variable region has at least 90%, 91%, 92%, 93%, 94%, 95%, 96%, 97%, 98%, 99% or 100% identity to the light chain variable region as shown in SEQ ID NO: 7, 22, 23, 24, 25 or 26; or
(vi) the heavy chain variable region has at least 90%, 91%, 92%, 93%, 94%, 95%, 96%, 97%, 98%, 99% or 100% identity to the heavy chain variable region as shown in SEQ ID NO: 8, 33, 34, 35 or 36, and the light chain variable region has at least 90%, 91%, 92%, 93%, 94%, 95%, 96%, 97%, 98%, 99% or 100% identity to the light chain variable region as shown in SEQ ID NO: 9, 30, 31 or 32; or
the heavy chain variable region has at least 90%, 91%, 92%, 93%, 94%, 95%, 96%, 97%, 98%, 99% or 100% identity to the heavy chain variable region as shown in SEQ ID NO: 6, and the light chain variable region has at least 90%, 91%, 92%, 93%, 94%, 95%, 96%, 97%, 98%, 99% or 100% identity to the light chain variable region as shown in SEQ ID NO: 7;
the heavy chain variable region has at least 90%, 91%, 92%, 93%, 94%, 95%, 96%, 97%, 98%, 99% or 100% identity to the heavy chain variable region as shown in SEQ ID NO: 27, 28 or 29, and the light chain variable region has at least 90%, 91%, 92%, 93%, 94%, 95%, 96%, 97%, 98%, 99% or 100% identity to the light chain variable region as shown in SEQ ID NO: 22, 23, 24, 25 or 26;
the heavy chain variable region has at least 90%, 91%, 92%, 93%, 94%, 95%, 96%, 97%, 98%, 99% or 100% identity to the heavy chain variable region as shown in SEQ ID NO: 8, and the light chain variable region has at least 90%, 91%, 92%, 93%, 94%, 95%, 96%, 97%, 98%, 99% or 100% identity to the light chain variable region as shown in SEQ ID NO: 9;
the heavy chain variable region has at least 90%, 91%, 92%, 93%, 94%, 95%, 96%, 97%, 98%, 99% or 100% identity to the heavy chain variable region as shown in SEQ ID NO: 33, 34, 35 or 36, and the light chain variable region has at least 90%, 91%, 92%, 93%, 94%, 95%, 96%, 97%, 98%, 99% or 100% identity to the light chain variable region as shown in SEQ ID NO: 30, 31 or 32.

In some embodiments, the anti-CTGF antibody comprises a heavy chain variable region and a light chain variable region, wherein:
(vi-ii) the heavy chain variable region has at least 90%, 91%, 92%, 93%, 94%, 95%, 96%, 97%, 98%, 99% or 100% identity to the heavy chain variable region as shown in SEQ ID NO: 69, 81, 82, 83, 84, or 85, and the light chain variable region has at least 90%, 91%, 92%, 93%, 94%, 95%, 96%, 97%, 98%, 99% or 100% identity to the light chain variable region as shown in SEQ ID NO: 70, 77, 78, 79 or 80.

In some embodiments of the anti-CTGF antibody, wherein the anti-CTGF antibody is a humanized antibody, which comprises a framework region of a human antibody or a framework region variant thereof, and the framework region variant has up to 10 back mutation(s) on each of the human antibody light chain framework region and/or the heavy chain framework region;

In some embodiments, the anti-CTGF antibody comprises a heavy chain variable region and a light chain variable region as described below:

(a) the light chain variable region comprises LCDR1, LCDR2 and LCDR3 as shown in SEQ ID NO: 13, SEQ ID NO: 14 and SEQ ID NO: 15, respectively, and comprises one or more amino acid back mutation(s) selected from the group consisting of 4L, 36F, 43S, 45K, 47W, 58V or 71Y, and/or the heavy chain variable region comprises HCDR1, HCDR2 and HCDR3 as shown in SEQ ID NO: 10, SEQ ID NO: 11 and SEQ ID NO: 12, respectively, and comprises one or more amino acid back mutation(s) selected from the group consisting of 28S, 30N, 49A, 75E, 76S, 93V, 94E or 104D; or

(b) the light chain variable region comprises LCDR1, LCDR2 and LCDR3 as shown in SEQ ID NO: 19, SEQ ID NO: 20 and SEQ ID NO: 21, respectively, and comprises one or more back mutation(s) selected from the group consisting of 36V, 44F, 46G or 49G, and/or the heavy chain variable region comprises HCDR1, HCDR2 and HCDR3 as shown in SEQ ID NO: 16, SEQ ID NO: 17 and SEQ ID NO: 18, respectively, and comprises one or more back mutation(s) selected from the group consisting of 44G, 49G, 27F, 48L, 67L, 71K, 78V or 80F.

In some embodiments, the anti-CTGF antibody comprises a heavy chain variable region and a light chain variable region as described below:

(b-i) the light chain variable region comprises LCDR1, LCDR2 and LCDR3 as shown in SEQ ID NO: 74, SEQ ID NO: 75 and SEQ ID NO: 76, respectively, and one or more back mutation(s) selected from the group consisting of 45P, 46W, 48Y, 69S or 70Y, and the heavy chain variable regions comprises HCDR1, HCDR2 and HCDR3 as shown in SEQ ID NO: 71, SEQ ID NO: 72 and SEQ ID NO: 73, respectively, and one or more back mutation(s) selected from the group consisting of 27F, 38K, 481, 67K, 68A, 70L or 72F; or

(b-ii) the light chain variable region comprises LCDR1, LCDR2 and LCDR3 as shown in SEQ ID NO: 74, SEQ ID NO: 75 and SEQ ID NO: 76, respectively, and comprises one or more back mutation(s) selected from the group consisting of 45P, 46W, 48Y, 69S or 70Y, and the heavy chain variable regions comprises HCDR1, HCDR2 and HCDR3 as shown in SEQ ID NO: 102, SEQ ID NO: 103 and SEQ ID NO: 104, respectively, and comprises one or more back mutation(s) selected from the group consisting of 27F, 38K, 481, 67K, 68A, 70L or 72F.

In some embodiments, the anti-CTGF antibody comprises a heavy chain variable region and a light chain variable region as described below:

(vii) the sequence of the heavy chain variable region is as shown in SEQ ID NO: 6, and the sequence of the light chain variable region is as shown in SEQ ID NO: 7;
(viii) the sequence of the heavy chain variable region is as shown in SEQ ID NO: 27, 28 or 29, and the sequence of the light chain variable region is as shown in SEQ ID NO: 22, 23, 24, 25 or 26;
(ix) the sequence of the heavy chain variable region is as shown in SEQ ID NO: 8, and the sequence of the light chain variable region is as shown in SEQ ID NO: 9; or

(x) the sequence of the heavy chain variable region is as shown in SEQ ID NO: 33, 34, 35 or 36, and the sequence of the light chain variable region is as shown in SEQ ID NO: 30, 31 or 32.

In some embodiments, the anti-CTGF antibody comprises a heavy chain variable region and a light chain variable region as described below:

(xi) the amino acid sequence of the heavy chain variable region is as shown in SEQ ID NO: 69, and the amino acid sequence of the light chain variable region is as shown in SEQ ID NO: 70; or

(xii) the amino acid sequence of the heavy chain variable region is as shown in SEQ ID NO: 81, 82, 83, 84, or 85, and the amino acid sequence of the light chain variable region is as shown in SEQ ID NO: 77, 78, 79 or 80.

In some embodiments, the anti-CTGF antibody comprises a heavy chain variable region and a light chain variable region as shown in Table a, Table b, and Table c below:

Table a: Combinations of the humanized antibody light chain variable region and heavy chain variable region derived from mab 147

|  | Hu147-VL1 SEQ ID NO: 22 | Hu147-VL2 SEQ ID NO: 23 | Hu147-VL3 SEQ ID NO: 24 | Hu147-VL4 SEQ ID NO: 25 | Hu147-VL5 SEQ ID NO: 26 |
|---|---|---|---|---|---|
| Hu147-VH1 SEQ ID NO: 27 | Hu147-11 | Hu147-12 | Hu147-13 | Hu147-14 | Hu147-15 |
| Hu147-VH2 SEQ ID NO: 28 | Hu147-21 | Hu147-22 | Hu147-23 | Hu147-24 | Hu147-25 |
| Hu147-VH3 SEQ ID NO: 29 | Hu147-31 | Hu147-32 | Hu147-33 | Hu147-34 | Hu147-35 |

Table b: Combinations of mab164 humanized antibody light chain variable region and heavy chain variable region

|  | Hu164-VH5 SEQ ID NO: 33 | Hu164-VH6 SEQ ID NO: 34 | Hu164-VH7 SEQ ID NO: 35 | Hu164-VH8 SEQ ID NO: 36 |
|---|---|---|---|---|
| Hu164-VL7 SEQ ID NO: 30 | Hu164-57 | Hu164-67 | Hu164-77 | Hu164-87 |
| Hu164-VL8 SEQ ID NO: 31 | Hu164-58 | Hu164-68 | Hu164-78 | Hu164-88 |
| Hu164-VL9 SEQ ID NO: 32 | Hu164-59 | Hu164-69 | Hu164-79 | Hu164-89 |

Table c: Combinations of mab95 humanized antibody light chain variable region and heavy chain variable region

|  | Hu95-VH1 SEQ ID NO: 81 | Hu95-VH2 SEQ ID NO: 82 | Hu95-VH3 SEQ ID NO: 83 | Hu95-VH4 SEQ ID NO: 84 | Hu95-VH5 SEQ ID NO: 85 |
|---|---|---|---|---|---|
| Hu95-VL1 SEQ ID NO: 77 | Hu95-11 | Hu95-21 | Hu95-31 | Hu95-41 | Hu95-51 |
| Hu95-VL2 SEQ ID NO: 78 | Hu95-12 | Hu95-22 | Hu95-32 | Hu95-42 | Hu95-52 |

(continued)

|  | Hu95-VH1 SEQ ID NO: 81 | Hu95-VH2 SEQ ID NO: 82 | Hu95-VH3 SEQ ID NO: 83 | Hu95-VH4 SEQ ID NO: 84 | Hu95-VH5 SEQ ID NO: 85 |
|---|---|---|---|---|---|
| Hu95-VL3 SEQ ID NO: 79 | Hu95-13 | Hu95-23 | Hu95-33 | Hu95-43 | Hu95-53 |
| Hu95-VL4 SEQ ID NO: 80 | Hu95-14 | Hu95-24 | Hu95-34 | Hu95-44 | Hu95-54 |

In some embodiments, the anti-CTGF antibody comprises a heavy chain variable region and a light chain variable region as described below:

(xiii) the amino acid sequence of the heavy chain variable region is as shown in SEQ ID NO: 27, and the amino acid sequence of the light chain variable region is as shown in SEQ ID NO: 22;
(xiv) the amino acid sequence of the heavy chain variable region is as shown in SEQ ID NO: 34, and the amino acid sequence of the light chain variable region is as shown in SEQ ID NO: 30; or
(xv) the amino acid sequence of the heavy chain variable region is as shown in SEQ ID NO: 85, and the amino acid sequence of the light chain variable region is as shown in SEQ ID NO: 77.

In some embodiments of the anti-CTGF antibody, wherein the antibody further comprises an antibody heavy chain constant region and a light chain constant region; preferably, the heavy chain constant region is selected from the group consisting of the constant regions of human IgG1, IgG2, IgG3 and IgG4 and conventional variants thereof, and the light chain constant region is selected from the group consisting of the constant regions of human antibody κ and λ chains and conventional variants thereof; more preferably, the antibody comprises a heavy chain constant region as shown in SEQ ID NO: 37 or 38 and a light chain constant region as shown in SEQ ID NO: 39 or 40.

In some embodiments, the anti-CTGF antibody comprises:

(c) a heavy chain having at least 85%, 90%, 91%, 92%, 93%, 94%, 95%, 96%, 97%, 98%, 99% or 100% identity to the heavy chain as shown in SEQ ID NO: 41, 43, 44, 45, 46, 47 or 48, and a light chain having at least 85%, 90%, 91%, 92%, 93%, 94%, 95%, 96%, 97%, 98%, 99% or 100% identity to the light chain as shown in SEQ ID NO: 42, 49, 50, 51, 52 or 53;
(d) a heavy chain having at least 85%, 90%, 91%, 92%, 93%, 94%, 95%, 96%, 97%, 98%, 99% or 100% identity to the heavy chain as shown in SEQ ID NO: 54, 56, 57, 58, 59, 60, 61, 62, or 63, and a light chain having at least 85%, 90%, 91%, 92%, 93%, 94%, 95%, 96%, 97%, 98%, 99% or 100% identity to the light chain as shown in SEQ ID NO: 55, 64, 65 or 66;
(e) a heavy chain as shown in SEQ ID NO: 41, 43, 44, 45, 46, 47 or 48, and a light chain as shown in SEQ ID NO: 42, 49, 50, 51, 52 or 53; or
(f) a heavy chain as shown in SEQ ID NO: 54, 56, 57, 58, 59, 60, 61, 62 or 63, and a light chain as shown in SEQ ID NO: 55, 64, 65, or 66.

In some embodiments, the anti-CTGF antibody comprises:

(g) a heavy chain having at least 85%, 90%, 91%, 92%, 93%, 94%, 95%, 96%, 97%, 98%, 99% or 100% sequence identity to SEQ ID NO: 86, 88, 89, 90, 91, 92, 93, 94, 95, 96, or 97, and a light chain having at least 85%, 90%, 91%, 92%, 93%, 94%, 95%, 96%, 97%, 98%, 99%, or 100% sequence identity to SEQ ID NO: 87, 98, 99, 100 or 101; or
(h) a heavy chain as shown in SEQ ID NO: 86, 88, 89, 90, 91, 92, 93, 94, 95, 96 or 97, and a light chain as shown in SEQ ID NO: 87, 98, 99, 100 or 101.

In some embodiments, the anti-CTGF antibody comprises:

(j) a heavy chain as shown in SEQ ID NO: 46, and a light chain as shown in SEQ ID NO: 49;
(k) a heavy chain as shown in SEQ ID NO: 61, and a light chain as shown in SEQ ID NO: 64; or
(1) a heavy chain as shown in SEQ ID NO: 97, and a light chain as shown in SEQ ID NO: 98.

In other aspects of the present disclosure, provided is an anti-CTGF antibody, which competitively with the anti-CTGF antibody or antigen-binding fragments thereof as described above, for the binding to human CTGF.

In other aspects of the present disclosure, provided is a nucleic acid molecule encoding the anti-CTGF antibody as described above.

In other aspects of the present disclosure, provided is a host cell comprising the nucleic acid molecule as described above.

In other aspects of the present disclosure, provided is a pharmaceutical composition, comprising a therapeutically effective amount or a prophylactically effective amount of the anti-CTGF antibody as described above, or the nucleic acid molecule as described above, and one or more pharmaceutically acceptable carriers, diluents, buffers or excipients.

In some specific embodiments, the therapeutically effective amount or prophylactically effective amount means that a unit dose of the composition comprises 0.1-3000 mg or 1-1000 mg of the anti-CTGF antibody as described above.

In other aspects of the present disclosure, provided is a method for the immunoassay or determination of CTGF, the method comprising a step of applying the anti-CTGF antibody as described above.

In other aspects of the present disclosure, provided is a method for the immunoassay or determination of CTGF, the method comprising a step of contacting the anti-CTGF antibody as described above with a subject or a sample thereof.

In other aspects of the present disclosure, provided is a kit comprising the anti-CTGF antibody as described above.

In other aspects of the present disclosure, provided is a method for the treatment of CTGF-related diseases, the method comprising administering to a subject a therapeutically effective amount of the anti-CTGF antibody as described above, or the nucleic acid molecule as described above, or the pharmaceutical composition as described above, wherein the disease is preferably a fibrous disease (the fibrous disease is preferably spontaneous pulmonary fibrosis, diabetic nephropathy, diabetic retinopathy, osteoarthritis, scleroderma, chronic heart failure, liver cirrhosis or renal fibrosis), hypertension, diabetes, myocardial infarction, arthritis, CTGF-related cell proliferative disease, atherosclerosis, glaucoma or cancer (the cancer is preferably acute lymphoblastic leukemia, dermatofibroma, breast cancer, angiolipoma, angioleiomyoma, connective tissue-generating cancer, prostate cancer, ovarian cancer, colorectal cancer, pancreatic cancer, gastrointestinal cancer or liver cancer).

In other aspects of the present disclosure, provided is use of the anti-CTGF antibody as described above, or the nucleic acid molecule as described above, or the pharmaceutical composition as described above, in the preparation of a medicament for the treatment of CTGF-related diseases, the CTGF-related disease includes a fibrous disease (the fibrous disease is preferably spontaneous pulmonary fibrosis, diabetic nephropathy, diabetic retinopathy, osteoarthritis, scleroderma, chronic heart failure, liver cirrhosis or renal fibrosis), hypertension, diabetes, myocardial infarction, arthritis, CTGF-related cell proliferative disease, atherosclerosis, glaucoma or cancer (the cancer is preferably acute lymphoblastic leukemia, dermatofibroma, breast cancer, angiolipoma, angioleiomyoma, connective tissue-generating cancer, prostate cancer, ovarian cancer, colorectal cancer, pancreatic cancer, gastrointestinal cancer or liver cancer).

In other aspects of the present disclosure, provided is the anti-CTGF antibody as described above, or the nucleic acid molecule encoding the anti-CTGF antibody as described above, or the pharmaceutical composition as described above, for use in the treatment of CTGF-related diseases, the CTGF-related disease includes a fibrous disease (the fibrous disease is preferably spontaneous pulmonary fibrosis, diabetic nephropathy, diabetic retinopathy, osteoarthritis, scleroderma, chronic heart failure, liver cirrhosis or renal fibrosis), hypertension, diabetes, myocardial infarction, arthritis, CTGF-related cell proliferative disease, atherosclerosis, glaucoma or cancer (the cancer is preferably acute lymphoblastic leukemia, dermatofibroma, breast cancer, angiolipoma, angioleiomyoma, connective tissue-generating cancer, prostate cancer, ovarian cancer, colorectal cancer, pancreatic cancer, gastrointestinal cancer or liver cancer).

## DESCRIPTION OF THE DRAWINGS

Figure 1: The affinity of humanized antibody derived from mab164 to human CTGF detected by ELISA.

Figure 2: Experimental results of the humanized antibody derived from mab 147 or mab 164 in inhibiting the SU86.86 mouse xenograft tumor.

## DETAILED DESCRIPTION OF THE INVENTION

### Terminology

In order to make the present disclosure be more easily understood, certain technical and scientific terms are specifically defined below. Unless otherwise defined explicitly herein, all other technical and scientific terms used herein have the meaning commonly understood by those skilled in the art to which this disclosure belongs.

Three-letter codes and one-letter codes for amino acids used in the present disclosure are as described in J.biol.chem, 243, p3558 (1968).

Connective Tissue Growth Factor (CTGF) is a 36kD cysteine-rich heparin-binding secreted glycoprotein, which is originally isolated from human umbilical vein endothelial cells (see, for example, Bradham et al. (1991) J Cell Biol114: 1285-1294; Grotendorst and Bradham, US Patent No. 5,408,040). CTGF belongs to the protein CCN (CTGF, Cyr61, Nov) family (secreted glycoproteins), and the family includes serum-induced immediate early gene product Cyr61, putative oncogene Nov, ECM-related protein FISP-12, src-induced gene CEF-10, Wnt-induced secreted protein WISP-3 and antiproliferative protein HICP/rCOP (Brigstock (1999) Endocr Rev 20: 189-206; O'Brian et al. (1990) Mol Cell Biol 10: 3569-3577; Joliot et al. (1992) Mol Cell Biol 12: 10-21; Ryseck et al. (1990) Cell Growth and Diff 2: 225-233; Simmons et al. (1989) Proc Natl Acad Sci USA 86: 1178-1182; Pennica et al. (1998) Proc Natl Acad Sci USA, 95: 14717-14722; and Zhang et al. (1998) Mol Cell Biol 18: 6131-6141. The CCN protein is characterized by the conservative 38 cysteine residues. The 38 cysteine residues constitute over 10% of the total amino acid content and give rise to a modular structure with N-terminal and C-terminal domains. The modular structure of CTGF includes conservative motifs for insulin-like growth factor binding protein (IGF-BP) and von Willebrand factor (VWC) in the N-terminal domain, and thrombospondin (TSP1) and a cysteine-knot motif in the C-terminal domain.

As used herein, "antibody" refers to immunoglobulin, and a full-length antibody is a four-peptide chain structure connected together by interchain disulfide bond(s) between two identical heavy chains and two identical light chains. Immunoglobulin heavy chain constant regions exhibit different amino acid compositions and orders, hence present different antigenicity. Accordingly, immunoglobulins can be divided into five types, or named as immunoglobulin isotypes, namely IgM, IgD, IgG, IgA and IgE, and the corresponding heavy chains are $\mu$, $\delta$, $\gamma$, $\alpha$ and $\varepsilon$, respectively. According to its amino acid composition of hinge region as well as the number and location of heavy chain disulfide bonds, the same type of Ig can further be divided into different sub-types, for example, IgG can be divided into IgG1, IgG2, IgG3 and IgG4. Light chains can be divided into $\kappa$ or $\lambda$ chain based on different constant regions. Each of the five types of Ig can have a kappa chain or a lambda chain.

About 110 amino acid sequences adjacent to the N-terminus of the antibody heavy and light chains are highly variable, known as variable regions (Fv regions); the rest of amino acid sequences close to the C-terminus are relatively stable, known as constant regions. The variable region includes 3 hypervariable regions (HVRs) and 4 framework regions (FRs) with relatively conservative sequences. The three hypervariable regions which determine the specificity of the antibody are also known as complementarity determining regions (CDRs). Each light chain variable region (VL) and each heavy chain variable region (VH) consists of three CDR regions and four FR regions, arranged from the amino terminus to carboxyl terminus in the following order: FR1, CDR1, FR2, CDR2, FR3, CDR3, and FR4. The three CDR regions of the light chain refer to LCDR1, LCDR2, and LCDR3, and the three CDR regions of the heavy chain refer to HCDR1, HCDR2, and HCDR3.

The antibodies of the present disclosure include murine antibodies, chimeric antibodies, and humanized antibodies.

As used herein, the term "murine antibody" refers to monoclonal antibodies against human CTGF prepared according to the knowledge and skills in the art. During the preparation, test subject is injected with CTGF antigen, and then a hybridoma expressing the antibody which possesses desired sequence or functional characteristics is isolated. In a preferred embodiment of the present disclosure, the murine anti-CTGF antibody or antigen-binding fragments thereof can further comprise a light chain constant region of murine kappa, lambda chain or variants thereof, or further comprise a heavy chain constant region of murine IgG1, IgG2, IgG3, or variants thereof.

The term "chimeric antibody", is an antibody by fusing the variable region of murine antibody to the constant region of human antibody, and such antibody can alleviate the murine antibody-induced immune response. To establish a chimeric antibody, first, a hybridoma secreting specific murine monoclonal antibody is established and variable region gene is cloned from the murine hybridoma. Then constant region gene is cloned from human antibody according to the need. The murine variable region gene is connected to the human constant region gene to form a chimeric gene, which can be subsequently inserted into an expression vector. Finally the chimeric antibody molecule will be expressed in eukaryotic or prokaryotic system. In a preferable embodiment of the present disclosure, the antibody light chain of the chimeric antibody further comprises a light chain constant region of human kappa, lambda chain or variants thereof. The antibody heavy chain of the CTGF chimeric antibody further comprises a heavy chain constant region of human IgG1, IgG2, IgG3, IgG4 or variants thereof, preferably comprises a heavy chain constant region of human IgG1, IgG2 or IgG4, or of IgG1, IgG2 or IgG4 variant with amino acid mutation(s) (such as L234A and/or L235A mutation, and/or S228P mutation).

The term "humanized antibody", also known as CDR-grafted antibody, refers to an antibody generated by grafting the murine CDR sequences into human antibody variable region frameworks, i.e., an antibody produced in different types of human germline antibody framework sequences. Humanized antibody can overcome heterologous responses induced by chimeric antibody which carries a large number of murine protein components. Such framework sequences can be obtained from public DNA database covering germline antibody gene sequences or published references. For example, germline DNA sequences of human heavy and light chain variable region genes can be found in "VBase" human germline sequence database (available on www.mrccpe.com.ac.uk/vbase), as well as in Kabat, EA, et al. 1991 Sequences of Proteins of Immunological Interest, 5th Ed. To avoid a decrease in activity caused by the decreased immunogenicity, the framework sequences in human antibody variable region can be subjected to minimal reverse mutation(s) or back mutation(s) to maintain or increase the activity. The humanized antibodies of the present disclosure also comprise humanized antibodies on which CDR affinity maturation is performed by yeast display.

Due to the residues contacted with an antigen, the grafting of CDR can result in a decreased affinity of an antibody or antigen binding fragment thereof to the antigen due to the framework residues contacted with the antigen. Such interactions can be resulted from highly somatic mutations. Therefore, it can still be necessary to graft such donor framework amino acids onto the humanized antibody frameworks. The amino acid residues involved in antigen binding and derived from non-human antibody or antigen binding fragment thereof can be identified by checking the sequence and structure of animal monoclonal antibody variable region. The donor CDR framework amino acid residues which are different from the germ lines can be considered as being related. If it is not possible to determine the most closely related germ line, the sequence can be compared to the common sequence shared by subtypes or the animal antibody sequence with high similarity percentage. Rare framework residues are thought to be the result of a high mutation in somatic cells, and play an important role in binding.

In an embodiment of the present disclosure, the antibody or antigen binding fragment thereof further comprises a light chain constant region derived from human or murine $\kappa$, $\lambda$ chain or variant thereof, or further comprises a heave chain constant region derived from human or murine IgG1, IgG2, IgG3, IgG4 or variant thereof; it can include a heavy chain constant region derived from human IgG1, IgG2 or IgG4, or from IgG1, IgG2 or IgG4 variant with amino acid mutation(s) (such as L234A and/or L235A mutation, and/or S228P mutation).

As used herein, the "conventional variants" of the human antibody heavy chain constant region and the human antibody light chain constant region refer to the human heavy or chain constant region variants disclosed in the prior art which do not change the structure and function of the antibody variable regions. Exemplary variants include IgG1, IgG2, IgG3 or IgG4 heavy chain constant region variants by site-directed modification and amino acid substitutions on the heavy chain constant region. The specific substitutions are, for example, YTE mutation, L234A and/or L235A mutations, S228P mutation, or mutations resulting in a knob-into-hole structure (making the antibody heavy chain have a combination of knob-Fc and hole-Fc), etc. These mutations have been proven to make the antibody have new properties, without changing the function of the antibody variable region.

"Human antibody (HuMAb)", "antibody derived from human ", "fully human antibody" and "completely human antibody" can be used interchangeably, and can be antibodies derived from human or antibodies obtained from a genetically modified organism which has been "engineered" by any method known in the art to produce specific human antibodies in response to antigen stimulation, and can be produced. In some technologies, elements of human heavy and light chain loci are introduced into cell lines of organisms derived from embryonic stem cell lines, and the endogenous heavy and light chain loci in these cell lines are targeted and disrupted. The targeted endogenous heavy and light chain loci included in these cell lines are disrupted. Transgenic organisms can synthesize human antibodies specific for human antigens, and the organisms can be used to produce hybridoma that secretes human antibodies. A human antibody can also be such antibody in which the heavy and light chains are encoded by nucleotide sequences derived from one or more human DNA sources. Fully human antibodies can also be constructed by gene or chromosome transfection methods and phage display technology, or constructed from B cells activated *in vitro,* all of which are known in the art.

The terms "full-length antibody", "full antibody", "whole antibody" and "complete antibody" are used interchangeably herein and refer to an antibody in a substantially intact form, as distinguished from antigen-binding fragments defined below. The term specifically refers to antibodies that contain constant regions in the light and heavy chains.

The "antibody" of the present disclosure includes "full-length antibodies" and antigen-binding fragments thereof.

In some embodiments, the full-length antibody of the present disclosure includes full-length antibodies formed by linking the light chain variable region to the light chain constant region, and linking the heavy chain variable region to the heavy chain constant region, as shown in the combination of light and heavy chain listed in the Tables 1, 2 and 3 below. Those skilled in the art can select the light chain constant region and heavy chain constant region from various antibody sources according to actual needs, such as human antibody-derived light chain constant region and heavy chain constant region. At the same time, the various combinations of the light and heavy chain variable region described in Tables 1, 2 and 3 can form a single chain antibody (scFv), Fab or other forms of antigen-binding fragment comprising scFv or Fab.

Table 1: Combinations of the humanized antibody light chain variable region and heavy chain variable region derived from mab 147

| | Hu147-VL1 | Hu147-VL2 | Hu147-VL3 | Hu147-VL4 | Hu147-VL5 |
|---|---|---|---|---|---|
| Hu147-VH1 | Hu147-11 | Hu147-12 | Hu147-13 | Hu147-14 | Hu147-15 |
| Hu147-VH2 | Hu147-21 | Hu147-22 | Hu147-23 | Hu147-24 | Hu147-25 |
| Hu147-VH3 | Hu147-31 | Hu147-32 | Hu147-33 | Hu147-34 | Hu147-35 |

Table 2. Combinations of the light chain variable region and heavy chain variable region of mab 164 humanized antibody

| | Hu164-VH5 | Hu164-VH6 | Hu164-VH7 | Hu164-VH8 |
|---|---|---|---|---|
| Hu164-VL7 | Hu164-57 | Hu164-67 | Hu164-77 | Hu164-87 |
| Hu164-VL8 | Hu164-58 | Hu164-68 | Hu164-78 | Hu164-88 |
| Hu164-VL9 | Hu164-59 | Hu164-69 | Hu164-79 | Hu164-89 |

Table 3. Combinations of the light chain variable region and heavy chain variable region of mab95 humanized antibody

| | Hu95-VH1 | Hu95-VH2 | Hu95-VH3 | Hu95-VH4 | Hu95-VH5 |
|---|---|---|---|---|---|
| Hu95-VL1 | Hu95-11 | Hu95-21 | Hu95-31 | Hu95-41 | Hu95-51 |
| Hu95-VL2 | Hu95-12 | Hu95-22 | Hu95-32 | Hu95-42 | Hu95-52 |
| Hu95-VL3 | Hu95-13 | Hu95-23 | Hu95-33 | Hu95-43 | Hu95-53 |
| Hu95-VL4 | Hu95-14 | Hu95-24 | Hu95-34 | Hu95-44 | Hu95-54 |
| Note: Hu164-57 means that the humanized antibody Hu164-57 derived from mab164 has a heavy chain variable region as described in Hu164-VH5 and a light chain variable region as described in Hu164-VL7; the others can be interpreted in the same manner. | | | | | |

The term "antigen-binding fragment" or "functional fragment" refers to one or more fragments of the antibody that retain the ability to specifically bind to an antigen (e.g., CTGF). It has been shown that fragments of a full-length antibody can be used to achieve function of binding to a specific antigen. Examples of the binding fragments involved in the term "antigen-binding fragment" of an antibody include (i) Fab fragment, a monovalent fragment composed of VL, VH, CL and CH1 domains; (ii) $F(ab')_2$ fragment, a bivalent fragment comprising two Fab fragments connected by disulfide bridge(s) in the hinge region, (iii) Fd fragment composed of VH and CH1 domains; (iv) Fv fragment composed of the VH and VL domains of one arm of the antibody; (v) dsFv, a stable antigen-binding fragment formed by VH and VL via interchain disulfide bond(s); and (vi) diabody, bispecific antibody and multispecific antibody containing fragments such as scFv, dsFv, and Fab. In addition, the two domains, VL and VH domain, of the Fv fragment are encoded by two separate genes, however, they can be linked by a synthetic linker by using recombinant methods, to generate a single protein chain in which a monovalent molecular is formed by pairing the VL and VH domain (referred to as single chain Fv (scFv); see, e.g., Bird et al. (1988) Science 242: 423-426; and Huston et al (1988) Proc. Natl. Acad. Sci USA85:5879-5883). Such single chain antibodies are also included in the term of "antigen binding fragment" of an antibody. Such fragments of antibodies are obtained using conventional techniques known in the field, and are screened for functional fragments by using the same method as that for an intact antibody. Antigen binding portions can be produced by recombinant DNA technology or by enzymatic or chemical disruption of an intact immunoglobulin. Antibodies can be in the form of different isotypes, e.g., IgG (e.g., IgG1, IgG2, IgG3 or IgG4 subtype), IgA1, IgA2, IgD, IgE or IgM antibody.

Fab is an antibody fragment obtained by treating an IgG antibody molecule with a papain (which cleaves the amino acid residue at position 224 of the H chain), and the antibody fragment has a molecular weight of about 50,000 and has antigen binding activity, in which about a half of the N-terminal side of H chain and the entire L chain are bound together through disulfide bond(s).

"$F(ab')_2$" is an antibody fragment with a molecular weight of about 100,000 and having antigen-binding activity, and it is obtained by digesting the downstream part of the two disulfide bonds in the hinge region of IgG by pepsin. $F(ab')_2$ contains two Fabs connected at the hinge region.

Fab' is an antibody fragment having a molecular weight of about 50,000 and having antigen binding activity, which is obtained by cleaving a disulfide bond at the hinge region of the above-mentioned F(ab')$_2$. The Fab' of the present disclosure can be produced by treating the F(ab')$_2$ of the present disclosure which specifically recognizes CTGF and binds to the extracellular region amino acid sequences or the three-dimensional structure thereof with a reducing agent, such as dithiothreitol.

Further, the Fab' can be produced by inserting DNA encoding Fab' of the antibody into a prokaryotic expression vector or eukaryotic expression vector and introducing the vector into a prokaryote or eukaryote to express the Fab'.

The term "single chain antibody", "single chain Fv" or "scFv" refers to a molecule comprising antibody heavy chain variable domain (or region; VH) connected to antibody light chain variable domain (or region; VL) by a linker. Such scFv molecules have general structure of NH$_2$-VL-linker-VH-COOH or NH$_2$-VH-linker-VL-COOH. A suitable linker in the prior art consists of repeated GGGGS amino acid sequence or variant thereof, for example, variant with 1-4 repeats (Holliger et al. (1993), Proc. Natl. Acad. Sci. USA 90:6444-6448). Other linkers that can be used in the present disclosure are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol.

Diabody is an antibody fragment wherein scFv or Fab is dimerized, and it is an antibody fragment having divalent antigen binding activity. In the bivalent antigen binding activity, the two antigens can be the same or different.

Bispecific and multispecific antibody refer to an antibody that can simultaneously bind to two or more antigens or antigenic determinants, including scFv or Fab fragments that can bind CTGF.

The diabody of the present disclosure can be produced by the following steps: obtaining cDNAs encoding VH and VL of the monoclonal antibody of the present disclosure which specifically recognizes human CTGF and binds to the extracellular region or three-dimensional structure thereof, constructing DNA encoding scFv so that the length of the linker peptide is 8 or less amino acid residues, inserting the DNA into a prokaryotic expression vector or eukaryotic expression vector, and then introducing the expression vector into a prokaryote or eukaryote to express the diabody. dsFv is obtained by substituting one amino acid residue in each of VH and VL with a cysteine residue, and then connecting the substituted polypeptides via a disulfide bond between the two cysteine residues. The amino acid residues to be substituted with a cysteine residue can be selected based on three-dimensional structure prediction of the antibody in accordance with known methods (Protein Engineering, 7, 697 (1994)).

The full-length antibodies or antigen-binding fragments of the present disclosure can be produced by the following steps: obtaining cDNAs encoding the antibody of the present disclosure which specifically recognizes human CTGF and binds to the amino acid sequence of the extracellular region or three-dimensional structure thereof, constructing DNA encoding dsFv, inserting the DNA into a prokaryotic expression vector or eukaryotic expression vector, and then introducing the expression vector into a prokaryote or eukaryote to express the dsFv.

The term "amino acid difference" or "amino acid mutation" refers to the amino acid changes or mutations in a protein or polypeptide variant compared to the original protein or polypeptide, including 1, 2, 3 or more amino acid insertions, deletions or substitutions on the basis of the original protein or polypeptide.

The term "antibody framework" or "FR region" refers to a part of the variable domain, either VL or VH, which serves as a scaffold for the antigen binding loops (CDRs) of this variable domain. Essentially, it is a variable domain without CDRs.

The term "complementarity determining region", "CDR" or "hypervariable region" refers to one of the six hypervariable regions present in the antibody variable domain that mainly contribute to antigen binding. Generally, there are three CDRs (HCDR1, HCDR2, HCDR3) in each heavy chain variable region, and three CDRs (LCDR1, LCDR2, LCDR3) in each light chain variable region. The amino acid sequence boundaries of CDRs can be determined by any of a variety of well-known schemes, including the "Kabat" numbering criteria (see Kabat et al. (1991), "Sequences of Proteins of Immunological Interest", 5th edition, Public Health Service, National Institutes of Health, Bethesda, MD), "Chothia" numbering criteria (see Al-Lazikani et al., (1997) JMB 273:927-948) and ImmunoGenTics (IMGT) numbering criteria (Lefranc MP, Immunologist, 7, 132- 136 (1999); Lefranc, MP, etc., Dev. Comp. Immunol., 27, 55-77 (2003), and the like. For example, for the classical format, following the Kabat criteria, the CDR amino acid residues in the heavy chain variable domain (VH) are numbered as 31-35 (HCDR1), 50-65 (HCDR2) and 95-102 (HCDR3); and the CDR amino acid residues in the light chain variable domain (VL) are numbered as 24-34 (LCDR1), 50-56 (LCDR2), and 89-97 (LCDR3). Following the Chothia criteria, the CDR amino acid residues in VH are numbered as 26-32 (HCDR1), 52-56 (HCDR2) and 95-102 (HCDR3); and the amino acid residues in VL are numbered as 26-32 (LCDR1), 50- 52 (LCDR2) and 91-96 (LCDR3). By combining both Kabat and Chothia to define CDRs, the CDRs are composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. Following IMGT criteria, the CDR amino acid residues in VH are roughly numbered as 26-35 (CDR1), 51-57 (CDR2) and 93-102 (CDR3), and the CDR amino acid residues in VL are roughly numbered as 27-32 (CDR1), 50-52 (CDR2) and 89-97 (CDR3). Following IMGT criteria, the CDR regions of an antibody can be determined by using IMGT/DomainGap Align Program. Unless otherwise specified, the antibody variable regions and CDR sequences involved in the embodiments of the present disclosure are applicable for the "Kabat" numbering criteria.

The term "epitope" or "antigenic determinant" refers to a site on an antigen to which an immunoglobulin or antibody specifically binds (e.g., a specific site on CTGF molecule). Epitopes typically include at least 3, 4, 5, 6, 7, 8, 9, 10, 11, 12, 13, 14 or 15 contiguous or non-contiguous amino acids in a unique tertiary conformation. See, for example, Epitope Mapping Protocols in Methods in Molecular Biology, Vol. 66, G.E. Morris, Ed. (1996).

The term "specifically bind to", "selectively bind to", "selectively binds to" or "specifically binds to" refers to the binding of an antibody to a predetermined epitope on an antigen. Typically, the antibody binds with an affinity (KD) of less than about $10^{-8}$M, for example, less than about $10^{-9}$ M, $10^{-10}$ M, $10^{-11}$ M, $10^{-12}$ M or even less.

The term "KD" refers to the dissociation equilibrium constant for particular antibody-antigen interaction. Generally, the antibody of the present disclosure binds to CTGF with a dissociation equilibrium constant (KD) of less than about $10^{-7}$ M, for example, less than about $10^{-8}$ M or $10^{-9}$ M, for example, the affinity of the antibody in the present disclosure to the cell surface antigen is determined by measuring KD value with FACS method.

When the term "competition" is used in the context of antigen binding proteins (e.g., neutralizing antigen binding proteins or neutralizing antibodies) that compete for the same epitope, it means that competition occurs between the antigen binding proteins, which is determined by the assays in which an antigen binding protein to be tested (e.g., an antibody or immunologically functional fragment thereof) prevents or inhibits (e.g., reduces) the specific binding of a reference antigen binding protein (e.g., a ligand or reference antibody) to a common antigen (e.g., a CTGF antigen or fragments thereof). Numerous types of competitive binding assays are available to determine whether an antigen binding protein competes with another. These assays are, for example, solid phase direct or indirect radioimmunoassay (RIA), solid phase direct or indirect enzyme immunoassay (EIA), Sandwich competition assay (see, e.g., Stahli et al., 1983, Methods in Enzymology 9: 242-253); solid phase direct biotin-avidin EIA (see, e.g., Kirkland et al., 1986, J. Immunol. 137: 3614-3619), solid phase direct labeling assay, solid phase direct labeling sandwich assay (see, e.g., Harlow and Lane, 1988, Antibodies, A Laboratory Manual, Cold Spring Harbor Press); solid phase direct labeling RIA with 1-125 label (see, e.g., Morel et al., 1988, Molec. Immunol. 25: 7-15); solid phase direct biotin-avidin EIA (see, e.g., Cheung, et al., 1990, Virology 176: 546-552); and direct labeling RIA (Moldenhauer et al., 1990, Scand. J. Immunol. 32: 77-82). Typically, the assay involves the use of a purified antigen capable of binding to a solid surface or cell loaded with both an unlabeled test antigen binding protein and a labeled reference antigen binding protein. Competitive inhibition is determined by measuring the amount of label bound to the solid surface or to the cell in the presence of the test antigen binding protein. Usually, the test antigen binding protein is present in excess. Antigen binding proteins identified by competitive assay (competing with the antigen binding protein) includes: antigen binding proteins that bind to the same epitope as the reference antigen binding protein; and antigen binding proteins that bind to an epitope that is sufficiently close to the epitope to which the reference antigen binding protein binds, where the two epitopes spatially interfere with each other to hinder the binding. Additional details regarding methods for determining competitive binding are provided in the Examples herein. Typically, when a competitive antigen binding protein is present in excess, it will inhibit (e.g., reduce) the specific binding of the reference antigen binding protein to the common antigen by at least 40-45%, 45-50%, 50-55%, 55-60%, 60-65%, 65-70%, 70-75% or 75% or even more. In some cases, the binding is inhibited by at least 80-85%, 85-90%, 90-95%, 95-97%, or 97% or even more.

As used herein, the term "nucleic acid molecule" refers to DNA molecules and RNA molecules. The nucleic acid molecule can be single-stranded or double-stranded, and is preferably double-stranded DNA or single-stranded mRNA or modified mRNA. A nucleic acid is "operably linked" when it is placed into a functional relationship with another nucleic acid sequence. For instance, a promoter or enhancer is operably linked to a coding sequence if it affects the transcription of the sequence.

Amino acid sequence "identity" refers to the percentage of the amino acid residues that are identical between the first and the second sequence when the amino acid sequences are aligned (introducing gaps when necessary) to achieve the maximum percentage of sequence identity, and any conservative substitution is not considered as part of the sequence identity. For the purpose of determining the percentage of amino acid sequence identity, the alignment can be achieved in a variety of ways within the scope of the art, for example, using publicly available computer software, such as BLAST, BLAST-2, ALIGN, ALIGN-2 or Megalign (DNASTAR) software. Those skilled in the art can determine the parameters suitable for measuring the alignment, including any algorithm required to achieve the optimum alignment over the entire length of the sequences being compared.

The term "expression vector" refers to a nucleic acid molecule capable of transporting another nucleic acid to which it has been linked. In one embodiment, the vector is a "plasmid," which refers to a circular double stranded DNA loop into which additional DNA segments can be ligated. In another embodiment, the vector is a viral vector, wherein additional DNA segments can be ligated into the viral genome. The vectors disclosed herein are capable of self-replicating in the host cell into which they are introduced (e.g., bacterial vectors having a bacterial replication origin and episomal mammalian vectors), or can be integrated into the genome of a host cell upon introduction into the host cell, and thereby are replicated along with the host genome (e.g., non-episomal mammalian vectors).

Methods for producing and purifying antibodies and antigen-binding fragments are well known in the art, for example, A Laboratory Manual for Antibodies, Cold Spring Harbor Laboratory Press, Cold Spring Harbor, New York, chapters 5-8 and 15. For example, mice can be immunized with human CTGF or fragment thereof, and the resulting antibodies can then be renatured, purified, and sequenced for amino acid sequences by using conventional methods well known in the art. Antigen-binding fragments can also be prepared by conventional methods. The antibodies or antigen binding fragments of the present disclosure are engineered to incorporate one or more human framework regions onto the CDR regions derived from non-human antibody. Human FR germline sequences can be obtained from ImMunoGeneTics (IMGT) via their website http://imgt.cines.fr, or from The Immunoglobulin Facts Book, 2001, ISBN 012441351, by aligning against IMGT human antibody variable germline gene database using MOE software.

The term "host cell" refers to a cell into which an expression vector has been introduced. Host cells can include bacterial, microbial, plant or animal cells. Bacteria that are readily transformed include members of Enterobacteriaceae, such as *Escherichia coli* or *Salmonella* strains; *Bacillaceae* such as *Bacillus subtilis*; *Pneumococcus*; *Streptococcus* and *Haemophilus influenzae*. Suitable microorganisms include *Saccharomyces cerevisiae* and *Pichia pastoris*. Suitable animal host cell lines include CHO (Chinese Hamster Ovary cell line), 293 cells and NS0 cells.

The engineered antibodies or antigen-binding fragments of the present disclosure can be prepared and purified by conventional methods. For example, the cDNA sequences encoding the heavy and light chains can be cloned and recombined into a GS expression vector. The vectors expressing recombinant immunoglobulin can then be stably transfected into CHO cells. As a more recommended method well known in the art, mammalian expression systems will result in glycosylation, typically at highly conservative N-terminal sites in the Fc region. Stable clones can be obtained by expressing an antibody specifically binding to human CTGF. Positive clones can be expanded in serum-free culture medium in bioreactors for antibody production. Culture medium, into which an antibody has been secreted, can be purified by conventional techniques. For example, purification can be performed on Protein A or G Sepharose FF column that has been modified with buffer. The nonspecific binding components are washed out. The bound antibody is eluted by pH gradient and antibody fragments are detected by SDS-PAGE, and then pooled. The antibodies can be filtered and concentrated using common techniques. Soluble mixtures and multimers can be effectively removed by common techniques, such as size exclusion or ion exchange. The resulting product is needed to be frozen immediately, such as at -70°C, or lyophilized.

"Administering", "dosing" or "treating" when applied to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administering", "dosing" or "treating" can refer, e.g., to therapeutic, pharmacokinetic, diagnostic, research, and experimental methods. The treatment of a cell encompasses contacting a reagent with the cell, as well as contacting a reagent with a fluid, where the fluid is in contact with the cell. "Administering", "dosing" or "treating" also means in vitro or *ex vivo* treatments, e.g., of a cell, with a reagent, diagnostic, binding compound, or with another cell. "Treating", as it applies to a human, veterinary, or research subject, refers to therapeutic treatment, prophylactic or preventative measures, research and diagnostic applications.

"Treatment" means to administer a therapeutic agent, such as a composition containing any of binding compounds of the present disclosure, internally or externally to a patient having one or more disease symptoms for which the agent has known therapeutic activity. Typically, the agent is administered in an amount effectively to alleviate one or more disease symptoms in the patient or population to be treated, to induce the regression of or inhibit the progression of such symptom(s) by any clinically measurable degree. The amount of a therapeutic agent that is effective to alleviate any particular disease symptom (also referred to as the "therapeutically effective amount") can vary according to various factors such as the disease state, age, and body weight of the patient, and the ability of the drug to elicit a desired response in the patient. Whether a disease symptom has been alleviated can be assessed by any clinical measurement typically used by physicians or other skilled healthcare providers to assess the severity or progression status of that symptom. While an embodiment of the present disclosure (e.g., a treatment method or article of manufacture) may not be effective in alleviating the target disease symptom(s) in every patient, it should alleviate the target disease symptom(s) in a statistically significant number of patients as determined by any statistical test known in the art such as Student's t-test, chi-square test, U-test according to Mann and Whitney, Kruskal-Wallis test (H-test), Jonckheere-Terpstra-test and Wilcoxon-test.

"Conservative modification" or "conservative substitution or replacement" refers to substitutions of amino acids in a protein with other amino acids having similar characteristics (e.g. charge, side-chain size, hydrophobicity/hydrophilicity, backbone conformation and rigidity, etc.), such that the changes can frequently be made without altering the biological activity of the protein. Those skilled in the art understand that, generally, a single amino acid substitution in a non-essential region of a polypeptide does not substantially change the biological activity (see, for example, Watson et al. (1987) Molecular Biology of the Gene, The Benjamin/Cummings Pub. Co., Page 224, (4th edition)). In addition, substitutions with structurally or functionally similar amino acids are less likely to disrupt biological activity. Exemplary conservative substitutions are set forth below.

| Original residue | Conservative substitution |
|---|---|
| Ala (A) | Gly; Ser |
| Arg(R) | Lys; His |
| Asn (N) | Gln; His; Asp |
| Asp (D) | Glu; Asn |
| Cys (C) | Ser; Ala; Val |
| Gln(Q) | Asn; Glu |

| | |
|---|---|
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro(P) | Ala |
| Ser(S) | Thr |
| Thr(T) | Ser |
| Trp (W) | Tyr; Phe |
| Tyr (Y) | Trp; Phe |
| Val (V) | Ile; Leu |

"Effective amount" or "effective dose" refers to the amount of a medicament, compound, or pharmaceutical composition necessary to obtain any one or more beneficial or desired results. For prophylactic applications, beneficial or desired results include elimination or reduction of risk, reduction of severity, or delay of the onset of the disease, including the biochemical, histological, and behavioral manifestations of the condition, its complications, and intermediate pathological phenotypes during the development of the condition. For therapeutic applications, beneficial or desired results include clinical results, such as reduction of the incidence of various conditions associated with target antigen of the present disclosure or improvement of one or more symptoms of the condition, reduction of the dosage/dose of other agents required to treat the condition, enhancement of the efficacy of another agent, and/or delay of the progression of the condition associated with the target antigen of the present disclosure in patients.

"Exogenous" refers to substances produced outside the organisms, cells, or humans according to circumstances. "Endogenous" refers to substances produced in the cells, organisms, or human bodies according to circumstances.

"Homology" refers to the sequence similarity between two polynucleotide sequences or between two polypeptide sequences. When a position in both of the two sequences to be compared is occupied by the same base or amino acid monomer subunit, e.g., if a position in each of two DNA molecules is occupied by adenine, then the molecules are homologous at that position. The percentage of homology between two sequences is a function of the number of matching or homologous positions shared by the two sequences divided by the number of positions to be compared and then multiplied by 100. For example, when two sequences are optimally aligned, if 6 out of 10 positions in the two sequences are matched or homologous, then the two sequences are 60% homologous; if 95 out of 100 positions in the two sequences are matched or homologous, then the two sequences are 95% homologous. Generally, when two sequences are aligned, comparison is performed to give the maximum homology percentage. For example, the comparison can be performed by the BLAST algorithm, in which the parameters of the algorithm are selected to give the maximum match between each sequence over the entire length of each reference sequence. The following references refer to the BLAST algorithm frequently used for sequence analysis: BLAST algorithm (BLAST ALGORITHMS): Altschul, SF et al., (1990) J. Mol. Biol. 215:403-410; Gish, W. et al., (1993) Nature Genet. 3:266-272; Madden, TL et al., (1996) Meth. Enzymol. 266:131-141; Altschul, SF et al., (1997) Nucleic Acids Res. 25:3389-3402; Zhang, J. et al. (1997) Genome Res. 7:649-656. Other conventional BLAST algorithms such as those available from NCBI BLAST are also well known to those skilled in the art.

As used herein, the expressions "cell," "cell line," and "cell culture" are used interchangeably and all such designations include progeny. Thus, the words "transformant" and "transformed cell" include the primary subject cells and cultures derived therefrom regardless of the number of passages. It should be also understood that all progeny cannot be precisely identical in DNA content, due to intentional or unintentional mutations. Mutant progeny that have the same function or biological activity as that screened in the originally transformed cells are included. Where distinct designations are intended to, it will be clearly understood from the context.

As used herein, "polymerase chain reaction" or "PCR" refers to a procedure or technique in which minute amounts of a specific portion of nucleic acid, RNA and/or DNA, are amplified as described in, e.g., U.S. Pat. No. 4,683,195. Generally, sequence information at the ends of or beyond the region of interest needs to be available, such that oligonucleotide primers can be designed; these primers will be identical or similar in sequence to opposite strands of the template to be amplified. The 5' terminal nucleotides of the two primers can be consistent with the ends of the amplified material. PCR can be used to amplify specific RNA sequences, specific DNA sequences from total genomic DNA, and cDNA transcribed from total cellular RNA, bacteriophage or plasmid sequences, etc. See generally Mullis et al. (1987) Cold Spring Harbor Symp. Ouant. Biol. 51:263; Erlich editors, (1989) PCR TECHNOLOGY (Stockton Press, NY). The PCR used in the present disclosure is considered to be one, but not the only, example of polymerase reaction method for amplifying a test nucleic acid sample. The method comprises use of nucleic acid sequences known as primers together with nucleic acid polymerase to amplify or generate a specific portion of nucleic acid.

"Isolated" refers to a purified state, in which the designated molecule is substantially free of other biological molecules, such as nucleic acids, proteins, lipids, carbohydrates, or other materials, such as cell debris and growth medium. In general, the term "isolated" is not intended to mean the complete absence of these materials or the absence of water, buffers or salts, unless they are present in an amount that significantly interferes with the experimental or therapeutic use of the compound as described herein.

"Optional" or "optionally" means that the event or circumstance that follows may but does not necessarily occur, and the description includes the instances in which the event or circumstance does or does not occur.

"Pharmaceutical composition" refers to a mixture comprising one or more compounds according to the present disclosure or a physiologically/pharmaceutically acceptable salt or prodrug thereof and other chemical components, such as physiologically/pharmaceutically acceptable carriers and excipients. The pharmaceutical composition aims at promoting the administration to an organism, facilitating the absorption of the active ingredient and thereby exerting a biological effect.

The term "pharmaceutically acceptable carrier" refers to any inactive substance suitable for use in a formulation for the delivery of antibodies or antigen-binding fragments. A carrier can be an anti-adhesive agent, adhesive agent, coating agent, disintegrating agent, filler or diluent, preservative (such as antioxidant, antibacterial or antifungal agent), sweetener, absorption delaying agent, wetting agent, emulsifier, buffer, and the like. Examples of suitable pharmaceutically acceptable carriers include water, ethanol, polyol (such as glycerol, propylene glycol, polyethylene glycol, and the like), dextrose, vegetable oil (such as olive oil), saline, buffer, buffered saline, and isotonic agent (such as sugars, polyol, sorbitol and sodium chloride).

In addition, the present disclosure includes an agent for treating a disease associated with target antigen (such as CTGF) positive cells, the agent comprising the anti-CTGF antibody or antibody fragment thereof of the present disclosure as an active ingredient. The active ingredient is administered to the subject in a therapeutically effective amount, and is capable of treating a disease associated with CTGF-positive cells in the subject. The therapeutically effective amount mean that a unit dose of the composition comprises 0.1-3000 mg of the full-length antibody or antigen-binding fragment thereof that specifically binds to human CTGF as described above.

The CTGF-related disease of the present disclosure is not limited, as long as it is a disease related to CTGF. For example, the therapeutic response induced by the molecule of the present disclosure can play the role by binding to human CTGF, and then preventing CTGF from binding to its receptor/ligand, or via killing the tumor cells overexpressing CTGF. Therefore, the molecules of the present disclosure, when present in a preparation or formulation suitable for therapeutic applications, are very useful for such persons suffering from tumor or cancer, optionally including pulmonary fibrosis, renal fibrosis, tumor development and growth, glaucoma, cell proliferative disease, cataract, choroidal neovascularization, amotio retinae, proliferative vitreoretinopathy, macular degeneration, diabetic retinopathy, corneal scarring and corneal opacity, cyst, reduced vascular calcification, pancreatic ductal adenocarcinoma, pancreatic cancer, melanoma, radiation-induced fibrosis (RIF), idiopathic pulmonary fibrosis, pulmonary remodeling disease selected from the group consisting of asthma, chronic bronchitis, chronic obstructive pulmonary disease (COPD), cystic fibrosis, emphysema, etc., preferably pancreatic cancer, pulmonary fibrosis, and renal fibrosis.

In addition, the present disclosure relates to methods for the immunoassay or determination of target antigens (for example, CTGF), reagents for the immunoassay or determination of target antigens (for example, CTGF), methods for the immunoassay or determination of cells expressing target antigens (for example, CTGF), and the diagnostic agents for diagnosing diseases associated with target antigen (for example, CTGF)-positive cells, comprising the antibody or antibody fragment of the present disclosure (as an active ingredient) that specifically recognizes the target antigens (for example, human CTGF) and binds to the extracellular amino acid sequences or to the tertiary structure thereof.

In the present disclosure, the method for detecting or measuring the amount of the target antigen (e.g. CTGF) can be any known method. For example, it includes immunoassay or determination methods.

The immunoassay or determination method is a method of detecting or measuring the amount of antibody or antigen using a labeled antigen or antibody. Examples of the immunoassay or determination methods include radioactive substance-labeled immunoantibody method (RIA), enzyme immunoassay (EIA or ELISA), fluorescence immunoassay (FIA), luminescence immunoassay, western blotting, physicochemical method, and the like.

The above-mentioned diseases associated with CTGF-positive cells can be diagnosed by detecting or measuring the CTGF-expressing cells using the antibodies or antibody fragments of the present disclosure.

Cells expressing the polypeptide can be detected by the known immunodetection methods, preferably by immunoprecipitation, fluorescent cell staining, immunotissue staining, and the like. In addition, the method such as fluorescent antibody staining method with the FMAT8100HTS system (Applied Biosystem) can be used.

In the present disclosure, samples to be detected or measured for the target antigen (e.g. CTGF) are not particularly limited, as long as they are possible to contain cells expressing the target antigen (e.g. CTGF), such as tissue, cells, blood, plasma, serum, pancreatic juice, urine, stool, tissue fluid or culture medium.

Dependent on the required diagnostic method, the diagnostic agent comprising the monoclonal antibody or antibody fragment thereof of the present disclosure can also contain reagents for performing an antigen-antibody reaction or reagents for detecting the reaction. The reagents for performing an antigen-antibody reaction include buffers, salts and the like. The reagents used for detection include agents commonly used in immunoassay or immunodetection methods, for example, a labeled secondary antibody that recognizes the monoclonal antibody, antibody fragment or conjugate thereof, and a substrate corresponding to the label.

The details of one or more embodiments of the present disclosure are set forth in the above specification. The preferred methods and materials are described below, although any method and material similar or identical to those described herein can be used in the practice or testing of the present disclosure. Through the specification and claims, other features, purposes and advantages of the present disclosure will become apparent. In the specification and claims, the singular forms are intented to include plural forms, unless the context clearly dictates otherwise. Unless otherwise defined explicitly herein, all technical and scientific terms used herein have the meaning commonly understood by those skilled in the art to which this disclosure belongs. All patents and publications cited in the specification are incorporated by reference. The following examples are provided to more fully illustrate the preferred embodiments of the present disclosure. These examples should not be construed as limiting the scope of the present disclosure in any way, and the scope of the present disclosure is defined by the claims.

**EXAMPLES**

**Example 1. Preparation of CTGF Antigen and Antibody**

**I. Design and expression of antigen**

Human CTGF protein (Genbank number: NM_001901.2) was used as a template to design the amino acid sequence of the antigen and the protein used for detection. Optionally, the CTGF protein or the fragment thereof was fused with various tags, and separately cloned into pTT5 vector or pXC vector, and transiently expressed in 293 cells or stably expressed in LONZA CHO cells to result in the antigens and proteins used for detection of the present disclosure. The following CTGFantigens refer to human CTGF, unless specifically indicated.

Human CTGF full-length protein (for immunization), SEQ ID NO: 1

QNCSGPCRCPDEPAPRCPAGVSLVLDGCGCCRVCAKQLGELCTERDPCDPHKG
LFCDFGSPANRKIGVCTAKDGAPCIFGGTVYRSGESFQSSCKYQCTCLDGAVGCMPLCS
MDVRLPSPDCPFPRRVKLPGKCCEEWVCDEPKDQTVVGPALAAYRLEDTFGPDPTMIR
ANCLVQTTEWSACSKTCGMGISTRVTNDNASCRLEKQSRLCMVRPCEADLEENIKKG
KKCIRTPKISKPIKFELSGCTSMKTYRAKFCGVCTDGRCCTPHRTTTLPVEFKCPDGEV
MKKNMMFIKTCACHYNCPGDNDIFESLYYRKMYGDMA

CTGF-his (a fusion protein of human CTGF mature protein with his tag) can be used for detection, SEQ ID NO: 2

MEFGLSWLFLVAILKGVQC*QNCSGPCRCPDEPAPRCPAGVSLVLDGCGCCRVCAK QLGELCTERDPCDPHKGLFCDFGSPANRKIGVCTAKDGAPCIFGGTVYRSGESFQSSCKYQ CTCLDGAVGCMPLCSMDVRLPSPDCPFPRRVKLPGKCCEEWVCDEPKDQTVVGPALAAYR LEDTFGPDPTMIRANCLVQTTEWSACSKTCGMGISTRVTNDNASCRLEKQSRLCMVRPCEA DLEENIKKGKKCIRTPKISKPIKFELSGCTSMKTYRAKFCGVCTDGRCCTPHRTTTLPVEFK CPDGEVMKKNMMFIKTCACHYNCPGDNDIFESLYYRKMYGDMA*<u>HHHHHH</u>

(Note: The single underline represents signal peptide, the italic represents the extracellular region of hCTGF, and the double underline represents his tag.)
mCTGF-mFc (a fusion protein of mouse CTGF coding region with mouse Fc tag, which can be used for detection) SEQ ID NO: 3

MEFGLSWLFLVAILKGVQC*QDCSAQCQCAAEAAPHCPAGVSLVLDGCGCCRVCA KQLGELCTERDPCDPHKGLFCDFGSPANRKIGVCTAKDGAPCVFGGSVYRSGESFQSSCKY QCTCLDGAVGCVPLCSMDVRLPSPDCPFPRRVKLPGKCCEEWVCDEPKDRTAVGPALAAY RLEDTFGPDPTMMRANCLVQTTEWSACSKTCGMGISTRVTNDNTFCRLEKQSRLCMVRPC EADLEENIKKGKKCIRTPKIAKPVKFELSGCTSVKTYRAKFCGVCTDGRCCTPHRTTTLPVE FKCPDGEIMKKNMMFIKTCACHYNCPGDNDIFESLYYRKMYGDMA*<u>EPRGPTIKPCPPCKC PAPNLLGGPSVFIFPPKIKDVLMISLSPIVTCVVVDVSEDDPDVQISWFVNNVEVHTAQT QTHREDYNSTLRVVSALPIQHQDWMSGKEFKCKVNNKDLPAPIERTISKPKGSVRAPQ VYVLPPPEEEMTKKQVTLTCMVTDFMPEDIYVEWTNNGKTELNYKNTEPVLDSDGSY FMYSKLRVEKKNWVERNSYSCSVVHEGLHNHHTTKSFSRTPGK</u>

(Note: The single underline represents signal peptide, the italic represents the mouse CTGF coding region, and the double underline represents mFc tag.)
Mouse CTGF protein-His tag, SEQ ID NO: 4

MEFGLSWLFLVAILKGVQC*QDCSAQCQCAAEAAPHCPAGVSLVLDGCGCCRVCA KQLGELCTERDPCDPHKGLFCDFGSPANRKIGVCTAKDGAPCVFGGSVYRSGESFQSSCKY QCTCLDGAVGCVPLCSMDVRLPSPDCPFPRRVKLPGKCCEEWVCDEPKDRTAVGPALAAY RLEDTFGPDPTMMRANCLVQTTEWSACSKTCGMGISTRVTNDNTFCRLEKQSRLCMVRPC EADLEENIKKGKKCIRTPKIAKPVKFELSGCTSVKTYRAKFCGVCTDGRCCTPHRTTTLPVE FKCPDGEIMKKNMMFIKTCACHYNCPGDNDIFESLYYRKMYGDMA*<u>HHHHHH</u>

(Note: The single underline represents signal peptide, the italic represents the mouse CTGF coding region, and the double underline represents his tag.)
Cynomolgus CTGF-Fc (a fusion protein of cynomolgus CTGF coding region with Fc, which can be used for detection), SEQ ID NO: 5

MEFGLSWLFLVAILKGVQC*QNCSGPCRCPAEQAPRCPAGVSLVLDGCGCCRVCAK*

*QLGELCTERDPCDPHKGLFCDFGSPANRKIGVCTAKDGAPCIFGGTVYRSGESFQSSCKYQ*
*CTCLDGAVGCMPLCSMDVRLPSPDCPFPRRVKLPGKCCEEWVCDEPKDQTVVGPALAAYR*
*LEDTFGPDPTMIRANCLVQTTEWSACSKTCGMGISTRVTNDNASCRLEKQSRLCMVRPCEA*
*DLEENIKKGKKCIRTPKISKPIKFELSGCTSVKTYRAKFCGVCTDGRCCTPHRTTTLPVEFKC*
*PDGEVMKKNMMFIKTCACHYNCPGDNDIFESLYYRKMYGDMA*<u>EPKSSDKTHTCPPCPAP</u>
<u>ELLGGPSVFLFPPKPKDTLMISRTPEVTCVVVDVSHEDPEVKFNWYVDGVEVHNAKT</u>
<u>KPREEQYNSTYRVVSVLTVLHQDWLNGKEYKCKVSNKALPAPIEKTISKAKGQPREPQ</u>
<u>VYTLPPSRDELTKNQVSLTCLVKGFYPSDIAVEWESNGQPENNYKTTPPVLDSDGSFFL</u>
<u>YSKLTVDKSRWQQGNVFSCSVMHEALHNHYTQKSLSLSPGK</u>

(Note: The single underline represents signal peptide, the italic represents the cynomolgus CTGF coding region, and the double underline represents Fc tag.)

**II. Purification of CTGF-related recombinant protein, and purification of anti-human CTGF hybridoma antibody and recombinant antibody**

**1. Separation and purification of hybridoma supernatant by Protein G affinity chromatography**

For the purification of mouse hybridoma supernatant, affinity chromatography performed with Protein G was preferable. The resulting hybridoma after culturing was centrifuged and the supernatant was taken out, and based on the volume of the supernatant, 10-15% volume of 1M Tris-HCl (pH 8.0-8.5) was added to adjust pH of the supernatant to neutral. The Protein G column was washed with 3-5×column volume of 6M guanidine hydrochloride, and then washed with 3-5×column volume of pure water; the column was equilibrated with 3-5×column volume of 1×PBS (pH7.4); the cell supernatant was loaded at a low flow rate for binding, and the flow rate was controlled so that the retention time was about 1 min or longer; the column was washed with 3-5×column volume of 1×PBS (pH 7.4) until the UV absorption dropped to the baseline; the samples were eluted with 0.1M acetic acid/sodium acetate (pH3.0) buffer, the elution peaks were pooled according to UV detection. The eluted product was rapidly adjusted to pH 5-6 with 1M Tris-HCl (pH8.0). The eluted product can be subjected to solution replacement according to methods well-known to those skilled in the art, for example, replacing the solution with a desired buffer system by ultrafiltration-concentration with an ultrafiltration tube, or replacing the solution with a desired buffer system by using molecular exclusion such as G-25 desalting, or removing the aggregates from the eluted product to improve the purity of the sample by using high-resolution molecular exclusion column such as Superdex 200.

**2. Purification of antibody with Protein A Affinity Chromatography**

First, the cell culture supernatant expressing the antibody was centrifuged at a high speed to collect the supernatant. The Protein A affinity column was washed with 3-5×column volume of 6M guanidine hydrochloride, and then washed with 3-5×column volume of pure water. The column was equilibrated with 3-5×column volume of, for example, 1×PBS (pH7.4). The cell supernatant was loaded at a low flow rate for binding, and the flow rate was controlled so that the retention time was about 1 min or longer; once the binding was finished, the column was washed with 3-5×column volume of 1×PBS (pH 7.4) until the UV absorption dropped to the baseline The samples were eluted with 0.1M acetic acid/sodium acetate (pH3.0-3.5) buffer, the elution peaks were pooled according to UV detection. The eluted product was rapidly adjusted with 1M Tris-HCl (pH8.0) to pH 5-6. The eluted product can be subjected to solution replacement according to methods well-known to those skilled in the art, for example, replacing the solution with a desired buffer system by ultrafiltration-concentration with an ultrafiltration tube, or replacing the solution with a desired buffer system by using molecular exclusion such as G-25 desalting, or removing the aggregates from the eluted product to improve the purity of the sample by using high-resolution molecular exclusion column such as Superdex 200.

**3. Purification of CTGF-related recombinant proteins (such as human CTGF-his) by nickel column**

The cell expression supernatant sample was centrifuged at high speed to remove impurities, the buffer was replaced with PBS, and imidazole was added to a final concentration of 5mM. The nickel column was equilibrated with PBS solution containing 5mM imidazole, and washed with 2-5×column volume. The supernatant sample after replacement was loaded onto the column for binding, and nickel columns avaliable from different companies can be selected as the media. The column was washed with PBS solution containing 5 mM imidazole until the A280 reading dropped to the baseline. The chromatography column was rinsed with PBS+10mM imidazole to remove the non-specifically bound protein impurities, and the effluent was collected. The target protein was eluted with PBS solution containing 300 mM imidazole, and the elution peak was collected.

The collected elution product was concentrated and then can be further purified by gel chromatography Superdex200 (GE) with PBS as a mobile phase, to remove aggregates and impurity protein peaks, and to collect the elution peak of the target product. The resulting protein was identified by electrophoresis, peptide mapping and LC-MS, and the correct proteins were aliquoted for use.

**4. Purification of CTGF-related recombinant proteins (such as human CTGF-Fc, cynomolgus CTGF-Fc) with Protein A affinity chromatography**

First, the culture supernatant was centrifuged at a high speed to collect the supernatant. The Protein A affinity column was washed with 3-5×column volume of 6M guanidine hydrochloride, and then washed with 3-5×column volume of pure water. The column was equilibrated with 3-5×column volume of, for example, 1×PBS (pH7.4). The cell supernatant was loaded at a low flow rate for binding, and the flow rate was controlled so that the retention time was about 1 min or longer; once the binding was finished, the column was washed with 3-5×column volume of 1×PBS (pH 7.4) until the UV absorption dropped to the baseline The samples were eluted with 0.1M acetic acid/sodium acetate (pH3.0-3.5) buffer, and the elution peaks were pooled according to UV detection.

The collected elution product was concentrated and then can be further purified by gel chromatography Superdex200 (GE) with PBS as a mobile phase, to remove aggregates and impurity protein peaks, and to collect the elution peak of the target product. The resulting protein was identified by electrophoresis, peptide mapping and LC-MS, and the correct proteins were aliquoted for use.

**Example 2. Preparation of anti-human CTGF monoclonal antibody I. Immunization**

The anti-human CTGF monoclonal antibodies were produced by immunizing mice. SJL white mice, female, 6-8 weeks old were used for experiment (Beijing Charles River Experimental Animal Technology Co., Ltd., animal production license number: SCXK (Beijing) 2012-0001). Feeding environment: SPF level. After the mice were purchased, they were kept in the laboratory environment for 1 week, with a light/dark cycle of 12/12 hours, at temperature of 20-25 °C; humidity of 40-60%. The mice adapted to the environment were immunized according to the following schemes. CTGF (R&D) and mCTGF-mFc were used as antigen for immunization.

Immunization scheme: mice were immunized with CTGF protein, 25 micrograms/mouse/time, via intraperitoneal injection. 100 μl/mouse was injected intraperitoneally (IP) on day 0, and then booster immunization was performed once every 14 days. Blood samples were collected on day 21, 35, 49 and 63, the antibody titer in mouse serum was determined by ELISA method. After 7 to 9 immunizations, mice with a high serum antibody titer approaching to the plateau were selected for splenocyte fusion. Three days before the splenocyte fusion, solution of CTGF protein antigen prepared in saline was intraperitoneally injected (IP), 25 μg/mouse, for booster immunization.

**II. Splenocyte fusion**

Hybridoma cells were obtained by fusing splenic lymphocytes with myeloma Sp2/0 (ATCC® CRL-8287™) by using a PEG-mediated fusion procedure. The fused hybridoma cells were resuspended in complete medium (IMDM medium comprising 20% FBS, 1×HAT, 1×OPI) at a density of 0.5-1 × 10^6/ml, seeded in a 96-well plate with 100 μl/well, incubated at 37°C and 5% $CO_2$ for 3-4 days, supplemented with 100μl/well of HAT complete medium, and continued to be cultured for 3-4 days until forming pinpointed clones. The supernatant was removed, 200μl/well of HT complete medium (IMDM medium comprising 20% FBS, 1×HT and 1×OPI) was added, incubated at 37°C, 5% $CO_2$ for 3 days and then subjected to ELISA detection.

**III. Screening of hybridoma cells**

According to the growth density of hybridoma cells, the hybridoma culture supernatant was detected by binding ELISA method. The cell supernatant in positive wells was detected by ELISA assay, for the binding with monkey CTGF/mouse CTGF/human CTGF protein (the specific procedures were as the same as those of Example 3). The cells in the wells positive for protein ELISA binding assay were timely expanded and cryopreserved; and were subcloned 2 to 3 times until a single cell clone was obtained.

Cells after each subcloning were also tested by CTGF binding ELISA. The hybridoma clones were obtained by screening via the above assay. The antibodies were further prepared by serum-free cell culture method. The antibodies were purified according to the example of purification, for use in the test examples.

## IV. Sequencing of the positive hybridoma clones

The procedures of cloning the sequences from the positive hybridoma were as follows. Hybridoma cells in logarithmic growth phase were collected. RNAs were extracted with Trizol (Invitrogen, Cat No. 15596-018) according to the kit's instruction and were reversely transcribed with PrimeScript™ Reverse Transcription kit (Takara, Cat No. 2680A). The cDNAs resulting from reverse transcription were amplified by PCR using mouse Ig-Primer Set (Novagen, TB326 Rev. B 0503), and the amplified products were subjected to sequencing. After sequencing, murine anti-CTGF antibodies mab147, mab 164 and mab95 were obtained. The amino acid sequences of the variable regions are as follows:

mab 147 VH amino acid sequence is as shown in SEQ ID NO: 6, *EVQLVESGGGLVQPEGSLKLSCAASGFSFN*TYAMN*WVRQAPGKGLEWFAR*IRTKSNNYATYY ADSVKD*RFTISRDDSESMLYLQMNNLKTED*TAMYYCVET*GFAY*WDQGTLVTVSA*

mab 147 VL amino acid sequence is as shown in SEQ ID NO: 7, *QIVLTQSPAIMSASPGEKVTITC*SASSSVSYMH-WFQQKPGTSPKLWIY*STSNLAS*GVPARFSGS GSGTSYSLTISRMEAEDAATYYC*QQRSSYPLT*FGAGTKLELK*

mab 164 VH amino acid sequence is as shown in SEQ ID NO: 8, *QVQLKQSGPGLVQPSQSLSITC*TVSGFSLTTF-GVH*WIRQSPGKGLEWLG*VIWRRGGTDYNA　AFMS*RLSITKDNSRSHVFFKMTSLQTDDSAIYYCARD*GGFDY-WGQGTTLTVSS*

mab 164 VL amino acid sequence is as shown in SEQ ID NO: 9, *QAVVTQESALTTSPGGTVILTC*RSSIGAVTTSN-YANW*VQEKPDHLFTGLIG*GTSNRAP*GVPVR　FSGSLIGDKAALTITGAQAEDDAMYFC*ALWYSTHYV*FG-GGTKVTVL*

mab95 VH amino acid sequence is as shown in SEQ ID NO: 69, *EVLLQQSGPVL VKPGPSVKJSCKASGFTFTNY-DIHW*VKQSHGKSLEWIG*LVYPYNGGTAYNQ　KFKD*KATLTFDTSSSTAYMELNSLTSEDSAVYYCAR-WGLIPGTTSYFDV*WGTGTTVTVSS*

mab95 VL amino acid sequence is as shown in SEQ ID NO: 70, *QIVLSQSPPILSASPGEKVTMTC*RASSSVSYIH-WYQQKPRSSPKPWIY*ATSNLAS*GVPARFSGS GSGTSYSLTISRVEAEDAATYYC*QQWNSNPWT*FGGGTRLEIK*

In the above-mentioned mab147, mab164 and mab95 antibody variable region sequences, the italics represents the FR sequence, the underlined italics represents the CDR sequences, and the sequence order is FR1-CDR1-FR2-CDR2-FR3-CDR3-FR4.

Table 4.The amino acid sequences of CDR regions of anti-CTGF antibody heavy chain and light chain*

| Antibo dy | Heavy chain | | | Light chain | |
|---|---|---|---|---|---|
| mab147 | HCDR1 | TYAMN SEQ ID NO: 10 | | LCDR1 | SASSSVSYMH SEQ ID NO: 13 |
| | HCDR2 | RIRTKSNNYATYYA DSVKD SEQ ID NO: 11 | | LCDR2 | STSNLAS SEQ ID NO: 14 |
| | HCDR3 | TGFAY SEQ ID NO: 12 | | LCDR3 | QQRSSYPLT SEQ ID NO: 15 |
| mab164 | HCDR1 | TFGVH SEQ ID NO: 16 | | LCDR1 | RSSIGAVTTSNYAN SEQ ID NO: 19 |
| | HCDR2 | VIWRRGGTDYNAA FMS SEQ ID NO: 17 | | LCDR2 | GTSNRAP SEQ ID NO: 20 |
| | HCDR3 | DGGFDY SEQ ID NO: 18 | | LCDR3 | ALWYSTHYV SEQ ID NO: 21 |
| mab95 | HCDR1 | NYDIH SEQ ID NO: 71 | | LCDR1 | RASSSVSYIH SEQ ID NO: 74 |
| | HCDR2 | LVYPYNGGTAYNQ KFKD SEQ ID NO: 72 | | LCDR2 | ATSNLAS SEQ ID NO: 75 |
| | HCDR3 | WGLIPGTTSYFDV SEQ ID NO: 73 | | LCDR3 | QQWNSNPWT SEQ ID NO: 76 |
| *The CDRs in the table are determined by the Kabat numbering criteria. | | | | | |

**V. Preparation of human IgG1 chimeric antibody**

The candidate molecules mab147, mab 164 and mab95 were amplified and sequenced to obtain the gene sequences encoding the variable regions. Forward and reverse primers were designed on the basis of the sequences obtained by sequencing, the gene to be sequenced was used as a template to construct the VH/VK gene fragment for each antibody via PCR, and then inserted into the expression vector pHr (with a signal peptide and hIgG1/hkappa/hlambda constant region gene (CH1-Fc/CL) fragment) via homologous recombination to construct an expression plasmid for the full-length of recombinant chimeric antibody VH-CH1-Fc-pHr/VL-CL-pHr, resulting in three chimeric antibodies Ch147, Ch164 and Ch95.

**VI. Humanization of murine anti-human CTGF antibody**

The heavy chain and light chain variable region germline genes having high homology with murine antibodies were selected as templates by aligning against IMGT (http://imgt.cines.fr) human antibody heavy and light chain variable germline gene database by using MOE software (Molecular Operating Environment) software. The murine antibody CDRs were grafted into the corresponding human templates to form variable region sequences in an order of FR1-CDR1-FR2-CDR2-FR3-CDR3-FR4.

1. Humanization of mab147

For murine antibody mab147, the light chain templates for humanization were IGKV3-11*01 and IGKJ2*01, or IGKV1-39*01 and IGKJ2*01, and the heavy chain templates for humanization were IGHV3-72*01 and IGHJ1*01. Each of the CDRs of the murine antibody mab 147 were grafted into its human template, and then the amino acids of the FR of the humanized antibody were subjected to back mutation. Among them, the back mutation(s) in the light chain variable region include one or more selected from the group consisting of 4L, 36F, 43S, 45K, 47W, 58V or 71Y, and the back mutation(s) in the heavy chain variable region include one or more selected from the group consisting of 28S, 30N, 49A, 75E, 76S, 93V, 94E, or 104D (the back mutation positions in the light and heavy chain variable regions were determined according to the Kabat numbering criteria), resulting in the light chain variable regions and the heavy chain variable regions with different sequences. The humanized antibodies comprising the CDRs of mab 147 were obtained, and the variable region sequences thereof are as follows:

The specific variable region sequences of the humanized antibodies of mAb147 are as follows:

The amino acid sequence of Hu147-VL1 is as shown in SEQ ID NO: 22

*EIVLTQSPATLSLSPGERATLSCSASSSVSYMHWFQQKPGQSPRLLIYSTSNLASGIPAR FSGSGSGTDYTLTISSLEPEDFAVYYCQQRSSYPLTFGQGTKLEIK*

The amino acid sequence of Hu147-VL2 is as shown in SEQ ID NO: 23

*EIVLTQSPATLSLSPGERATLSCSASSSVSYMHWFQQKPGQSPKLLIYSTSNLASGIPAR FSGSGSGTDYTLTISSLEPEDFAVYYCQQRSSYPLTFGQGTKLEIK*

The amino acid sequence of Hu147-VL3 is as shown in SEQ ID NO: 24

*EIVLTQSPATLSLSPGERATLSCSASSSVSYMHWFQQKPGQSPKLWIYSTSNLASGVPA RFSGSGSGTDYTLTISSLEPEDFAVYYCQQRSSYPLTFGQGTKLEIK*

The amino acid sequence of Hu147-VL4 is as shown in SEQ ID NO: 25

*DIQMTQSPSSLSASVGDRVTITCSASSSVSYMHWFQQKPGKSPKLLIYSTSNLASGVPS RFSGSGSGTDYTLTISSLQPEDFATYYCQQRSSYPLTFGQGTKLEIK*

The amino acid sequence of Hu147-VL5 is as shown in SEQ ID NO: 26

*DIQLTQSPSSLSASVGDRVTITCSASSSVSYMHWFQQKPGKSPKLWIYSTSNLASGVPS
RFSGSGSGTDYTLTISSLQPEDFATYYCQQRSSYPLTFGQGTKLEIK*

The amino acid sequence of Hu147-VH1 is as shown in SEQ ID NO: 27

*EVQLVESGGGLVQPGGSLRLSCAASGFTFSTYAMNWVRQAPGKGLEWVGRIRTKSN
NYATYYADSVKDRFTISRDDSKNSLYLQMNSLKTEDTAVYYCVETGFAYWDQGTLVTVSS*

The amino acid sequence of Hu147-VH2 is as shown in SEQ ID NO: 28

*EVQLVESGGGLVQPGGSLRLSCAASGFTFNTYAMNWVRQAPGKGLEWVARIRTKSN
NYATYYADSVKDRFTISRDDSKNSLYLQMNSLKTEDTAVYYCVETGFAYWDQGTLVTVSS*

The amino acid sequence of Hu147-VH3 is as shown in SEQ ID NO: 29

*EVQLVESGGGLVQPGGSLRLSCAASGFSFNTYAMNWVRQAPGKGLEWVARIRTKSN
NYATYYADSVKDRFTISRDDSESSLYLQMNSLKTEDTAVYYCVETGFAYWDQGTLVTVSS.*

Note: The CDR sequences determined according to Kabat Criteria are underlined, and the sequences are arranged in the order of FR1-CDR1-FR2-CDR2-FR3-CDR3-FR4.

2. Humanization of mab164

For murine antibody mab164, the light chain templates for humanization were composed of IGLV7-43*01 and IGLJ2*01, IGLV8-61*01 and IGLJ2*01, or IGLVI-40*02 and IGLJ2*01 respectively, and the heavy chain templates for humanization were composed of IGHV2-26*01 and IGHJ6*01, or IGHV4-31*02 and IGHJ6*01, respectively. Each of the CDRs of the murine antibody mab 164 was grafted into its human template, and then the amino acids of the FR of the humanized antibody were subjected to back mutation. Among them, the back mutation(s) in the light chain variable region include one or more selected from the group consisting of 36V, 44F, 46G or 49G, and the back mutation(s) in the heavy chain variable region include one or more selected from the group consisting of 44G, 49G, 27F, 48L, 67L, 71K, 78V or 80F (the back mutation positions in the light and heavy chain variable regions were determined according to the Kabat numbering criteria), resulting in the humanized heavy and the light chain of mab164, with variable region sequences as follows:

The specific variable region sequences of the humanized antibodies of mAb164 are as follows:

The amino acid sequence of Hu164-VL7 is as shown in SEQ ID NO: 30

*ETVVTQEPSLTVSPGGTVTLTCRSSIGAVTTSNYANWVQQKPGQAFRGLIGGTSNRAP
WTPARFSGSLLGGKAALTLSGVQPEDEAEYYCALWYSTHYVFGGGTKLTVL*

The amino acid sequence of Hu164-VL8 is as shown in SEQ ID NO: 31

*ETVVTQEPSFSVSPGGTVTLTCRSSIGAVTTSNYANWVQQTPGQAFRGLIGGTSNRAP
GVPDRFSGSILGNKAALTITGAQADDESDYYCALWYSTHYVFGGGTKLTVL*

The amino acid sequence of Hu164-VL9 is as shown in SEQ ID NO: 32

*ESVVTQPPSVSGAPGQRVTISCRSSIGAVTTSNYANWVQQLPGTAFKGLIGGTSNRAP
GVPDRFSGSKSGTSASLAITGLQAEDEADYYCALWYSTHYVFGGGTKLTVL*

The amino acid sequence of Hu164-VH5 is as shown in SEQ ID NO: 33

*EVTLKESGPVLVKPTETLTLTCTVSGFSLS<u>TFGVH</u>WIRQPPGKALEWLA<u>VIWRRGGT DYNAAFMS</u>RLTISKDTSKSQVVLTMTNMDPVDTATYYCAR<u>DGGFDY</u>WGQGTTVTVSS*

The amino acid sequence of Hu164-VH6 is as shown in SEQ ID NO: 34

*EVTLKESGPVLVKPTETLTLTCTVSGFSLS<u>TFGVH</u>WIRQPPGKGLEWLG<u>VIWRRGGT DYNAAFMS</u>RLTISKDTSKSQVVFTMTNMDPVDTATYYCAR<u>DGGFDY</u>WGQGTTVTVSS*

The amino acid sequence of Hu164-VH7 is as shown in SEQ ID NO: 35

*EVQLQESGPGLVKPSQTLSLTCTVSGFSIS<u>TFGVH</u>WIRQHPGKGLEWIG<u>VIWRRGGT DYNAAFMS</u>RVTISKDTSKNQVSLKLSSVTAADTAVYYCAR<u>DGGFDY</u>WGQGTTVTVSS*

The amino acid sequence of Hu164-VH8 is as shown in SEQ ID NO: 36

*EVQLQESGPGLVKPSQTLSLTCTVSGFSIS<u>TFGVH</u>WIRQHPGKGLEWLG<u>VIWRRGGT DYNAAFMS</u>RLTISKDTSKNQVSFKLSSVTAADTAVYYCAR<u>DGGFDY</u>WGQGTTVTVSS.*

Note: In the above mentioned Hu164 antibody sequences, the underline represents CDR sequence, and the sequence order is FR1-CDR1-FR2-CDR2-FR3-CDR3-FR4, wherein the CDR sequences are defined according to Kabat criteria.

3. Humanization of mab95

For murine antibody mab95, the light chain templates for humanization were IGKV3-20*02 and IGKV1-40*01, and the heavy chain template for humanization was IGHV1-3*01. Each of the CDRs of the murine antibody mab95 was grafted into its human template, and then the amino acids of the FR of the humanized antibody were subjected to back mutation. Among them, the back mutation(s) in the light chain variable region include one or more selected from the group consisting of 45P, 46W, 48Y, 69S or 70Y, and the back mutation(s) in the heavy chain variable region include one or more selected from the group consisting of 27F, 38K, 48I, 67K, 68A, 70L or 72F (the back mutation positions in the light and heavy chain variable regions were determined according to the Kabat numbering criteria), resulting in the light chain variable regions and the heavy chain variable regions with different sequences. The humanized antibodies comprising the CDRs of mab95 were obtained, and the variable region sequences thereof are as follows:

The specific variable region sequences of the humanized antibodies of mAb95 are as follows:

The amino acid sequence of Hu95-VL1 is as shown in SEQ ID NO: 77

*EIVLTQSPATLSLSPGERATLSC<u>RASSSVSYIH</u>WYQQKPGQAPRPWIY<u>ATSNLAS</u>GIPAR FSGSGSGTDYTLTISRLEPEDFAVYYC<u>QQWNSNPWT</u>FGGGTKVEIK*

The amino acid sequence of Hu95-VL2 is as shown in SEQ ID NO: 78

*EIVLTQSPATLSLSPGERATLSC<u>RASSSVSYIH</u>WYQQKPGQSPRPWIY<u>ATSNLAS</u>GVPAR FSGSGSGTSYTLTISRLEPEDFAVYYC<u>QQWNSNPWT</u>FGGGTKVEIK*

The amino acid sequence of Hu95-VL3 is as shown in SEQ ID NO: 79

*ESVLTQPPSVSGAPGQRVTISC<u>RASSSVSYIH</u>WYQQLPGTAPKPWIY<u>ATSNLAS</u>GVPDR FSGSKSGTSYSLAITGLQAEDEADYYC<u>QQWNSNPWT</u>FGGGTKVEIK*

The amino acid sequence of Hu95-VL4 is as shown in SEQ ID NO: 80

*EIVLTQPPSVSGAPGQRVTISCRASSSVSYIHWYQQLPGTSPKPWIYATSNLASGVPDR FSGSKSGTSYSLAITGLQAEDEADYYCQQWNSNPWTFGGGTKVEIK*

The amino acid sequence of Hu95-VH1 is as shown in SEQ ID NO: 81

*EVQLVQSGAEVKKPGASVKVSCKASGFTFTNYDIHWVRQAPGQRLEWMGLVYPYN GGTAYNQKFKDRVTLTFDTSASTAYMELSSLRSEDTAVYYCARWGLIPGTTSYFDVWGQGTT VTVSS*

The amino acid sequence of Hu95-VH2 is as shown in SEQ ID NO: 82

*EVQLVQSGAEVKKPGASVKVSCKASGFTFTNYDIHWVRQAPGQRLEWIGLVYPYNG GTAYNQKFKDRATLTFDTSASTAYMELSSLRSEDTAVYYCARWGLIPGTTSYFDVWGQGTTVT VSS*

The amino acid sequence of Hu95-VH3 is as shown in SEQ ID NO: 83

*EVQLVQSGAEVKKPGASVKVSCKASGFTFTNYDIHWVKQAPGQRLEWMGLVYPYN GGTAYNQKFKDKVTLTFDTSASTAYMELSSLRSEDTAVYYCARWGLIPGTTSYFDVWGQGTT VTVSS*

The amino acid sequence of Hu95-VH4 is as shown in SEQ ID NO: 84

*EVQLVQSGAEVKKPGASVKVSCKASGFTFTNYDIHWVKQAPGQRLEWIGLVYPYNG GTAYNQKFKDKATLTFDTSASTAYMELSSLRSEDTAVYYCARWGLIPGTTSYFDVWGQGTTVT VSS*

The amino acid sequence of Hu95-VH5 is as shown in SEQ ID NO: 85

*EVQLVQSGAEVKKPGASVKVSCRASGFTFTNYAIHWVKQAPGQRLEWMGLVYPYTG GTAYNQKFKDKVTLTFDTSASTAYMELSSLRSEDTAVYYCARWGMIPGTNSYFDVWGQGTTV TVSS.*

Note: The CDR sequences determined according to Kabat Criteria are underlined, and the sequences are arranged in the order of FR1-CDR1-FR2-CDR2-FR3-CDR3-FR4.

Among them, the CDR regions of Hu95-VH5 were further modified, wherein HCDR1 as shown in SEQ ID NO: 102 (NYAIH), HCDR2 as shown in SEQ ID NO: 103 (LVYPYTGGTAYNQKFKD), and HCDR3 as shown in SEQ ID NO: 104 (WGMIPGTNSYFDV).

**4. Construction and expression of anti-CTGF humanized antibody**

Primers were designed, VH/VL gene fragment of each humanized antibody was constructed by PCR and then inserted into the expression vector pHr (with a signal peptide and constant region gene (CH/CL) fragment) via homologous recombination to construct an expression vector for a full-length antibody VH-CH -pHr/VL-CL-pHr. For the humanized antibody, the constant region can be selected from the group consisting of the light chain constant region of human κ, λ chain, and can be selected from the group consisting of the heavy chain constant region of IgG1, IgG2, IgG3 or IgG4 or variant thereof. Non-limiting examples include optimizing the constant region of human IgG1, IgG2 or IgG4 to improve antibody's function. For example, the half-life of the antibody can be prolonged by point mutations in the constant region such as M252Y/S254T/T256E (YTE) site mutations, and the mutations such as S228P, F234A and L235A in the constant region.

An exemplary anti-CTGF humanized antibody constant region sequence is as follows:

The amino acid sequence of the heavy chain constant region is as shown in SEQ ID NO: 37 (the full-length antibody heavy chain is named with a suffix: w):

ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEVHNAKT

KPREEQYNSTYRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPPVLDSDGSFFL YSKLTVDKSRWQQGNVFSCSVMHEALHNHYTQKSLSLSPGK.

The amino acid sequence of the heavy chain constant region of the YTE engineered antibody is as shown in SEQ ID NO: 38 (the full-length antibody heavy chain is named with a suffix: y):

ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTCPPCPA PELLGGPSVFLFPPKPKDTLYITREPEVTCVVVDVSHEDPEVKFNWYVDGVEVHNAKT KPREEQYNSTYRVVSVLTVLHQDWLNGKEYKCKVSNKALPAPIEKTISKAKGQPREPQ VYTLPPSRDELTKNQVSLTCLVKGFYPSDIAVEWESNGQPENNYKTTPPVLDSDGSFFL YSKLTVDKSRWQQGNVFSCSVMHEALHNHYTQKSLSLSPGK.

The amino acid sequence of the light chain kappa constant region of the anti-CTGF humanized antibody is as shown in SEQ ID NO: 39: (the full-length antibody heavy chain is named with a suffix: k):

RTVAAPSVFIFPPSDEQLKSGTASVVCLLNNFYPREAKVQWKVDNALQSGNSQE SVTEQDSKDSTYSLSSTLTLSKADYEKHKVYACEVTHQGLSSPVTKSFNRGEC.

The amino acid sequence of the light chain lambda constant region of the anti-CTGF humanized antibody is as shown in SEQ ID NO: 40: (the full-length antibody heavy chain is named with a suffix: 1):

GQPKANPTVTLFPPSSEELQANKATLVCLISDFYPGAVTVAWKADGSPVKAGVE TTKPSKQSNNKYAASSYLSLTPEQWKSHRSYSCQVTHEGSTVEKTVAPTEC.

The heavy chain variable region of the humanized antibody was linked to the heavy chain constant region as shown in SEQ ID NO: 37, and the light chain variable region of the humanized antibody was linked to the light chain kappa constant region as shown in SEQ ID NO: 39, resulting in a full-length humanized antibody derived from mab147, including:

Table 5

|  | Hu147-VL1 | Hu147-VL2 | Hu147-VL3 | Hu147-VL4 | Hu147-VL5 |
|---|---|---|---|---|---|
| Hu147-VH1 | Hu147-11wk | Hu147-12wk | Hu147-13wk | Hu147-14wk | Hu147-15wk |
| Hu147-VH2 | Hu147-21wk | Hu147-22wk | Hu147-23wk | Hu147-24wk | Hu147-25wk |
| Hu147-VH3 | Hu147-31wk | Hu147-32wk | Hu147-33wk | Hu147-34wk | Hu147-35wk |

The heavy chain variable region of the humanized antibody was linked to the heavy chain constant region YTE variant as shown in SEQ ID NO: 38, and the light chain variable region of the humanized antibody was linked to the light chain kappa constant region as shown in SEQ ID NO: 39, resulting in a full-length humanized antibody derived from mab147, including:

Table 6

|  | Hu147-VL1 | Hu147-VL2 | Hu147-VL3 | Hu147-VL4 | Hu147-VL5 |
|---|---|---|---|---|---|
| Hu147-VH1 | Hu147-11yk | Hu147-12yk | Hu147-13yk | Hu147-14yk | Hu147-15yk |
| Hu147-VH2 | Hu147-21yk | Hu147-22yk | Hu147-23yk | Hu147-24yk | Hu147-25yk |
| Hu147-VH3 | Hu147-31yk | Hu147-32yk | Hu147-33yk | Hu147-34yk | Hu147-35yk |

The heavy chain variable region of the humanized antibody was linked to the heavy chain constant region as shown in SEQ ID NO: 37, and the light chain variable region of the humanized antibody was linked to the light chain lambda constant region as shown in SEQ ID NO: 40, resulting in a full-length humanized antibody derived from mab164, including:

Table 7

|  | Hu164-VH5 | Hu164-VH6 | Hu164-VH7 | Hu164-VH8 |
|---|---|---|---|---|
| Hu164-VL7 | Hu164-57wl | Hu164-67wl | Hu164-77wl | Hu164-87wl |
| Hu164-VL8 | Hu164-58wl | Hu164-68wl | Hu164-78wl | Hu164-88wl |
| Hu164-VL9 | Hu164-59wl | Hu164-69wl | Hu164-79wl | Hu164-89wl |

The heavy chain variable region of the humanized antibody was linked to the heavy chain constant region YTE variant as shown in SEQ ID NO: 38, and the light chain variable region of the humanized antibody was linked to the light chain lambda constant region as shown in SEQ ID NO: 40, resulting in a full-length humanized antibody derived from mab164, including:

Table 8

|  | Hu164-VH5 | Hu164-VH6 | Hu164-VH7 | Hu164-VH8 |
|---|---|---|---|---|
| Hu164-VL7 | Hu164-57yl | Hu164-67yl | Hu164-77yl | Hu164-87yl |
| Hu164-VL8 | Hu164-58yl | Hu164-68yl | Hu164-78yl | Hu164-88yl |
| Hu164-VL9 | Hu164-59yl | Hu164-69yl | Hu164-79yl | Hu164-89yl |

The heavy chain variable region of the humanized antibody was linked to the heavy chain constant region as shown in SEQ ID NO: 37, and the light chain variable region of the humanized antibody was linked to the light chain kappa constant region as shown in SEQ ID NO: 39, resulting in a full-length humanized antibody derived from mab95, including:

Table 9

|  | Hu95-VH1 | Hu95-VH2 | Hu95-VH3 | Hu95-VH4 | Hu95-VH5 |
|---|---|---|---|---|---|
| Hu95-VL1 | Hu95-11wk | Hu95-21wk | Hu95- 31wk | Hu95- 41wk | Hu95- 51wk |
| Hu95-VL2 | Hu95-12wk | Hu95-22wk | Hu95- 32wk | Hu95- 42wk | Hu95- 52wk |
| Hu95-VL3 | Hu95-13wk | Hu95-23wk | Hu95- 33wk | Hu95- 43wk | Hu95- 53wk |
| Hu95-VL4 | Hu95-14wk | Hu95-24wk | Hu95- 34wk | Hu95- 44wk | Hu95- 54wk |

The heavy chain variable region of the humanized antibody was linked to the heavy chain constant region YTE variant as shown in SEQ ID NO: 38, and the light chain variable region of the humanized antibody was linked to the light chain kappa constant region as shown in SEQ ID NO: 39, resulting in a full-length humanized antibody derived from mab95, including:

Table 10

|  | Hu95-VH1 | Hu95-VH2 | Hu95-VH3 | Hu95-VH4 | Hu95-VH5 |
|---|---|---|---|---|---|
| Hu95-VL1 | Hu95- 11yk | Hu95- 21yk | Hu95- 31yk | Hu95- 41yk | Hu95- 51yk |
| Hu95-VL2 | Hu95- 12yk | Hu95- 22yk | Hu95- 32yk | Hu95- 42yk | Hu95- 52yk |
| Hu95-VL3 | Hu95- 13yk | Hu95- 23yk | Hu95- 33yk | Hu95- 43yk | Hu95- 53yk |
| Hu95-VL4 | Hu95-14yk | Hu95- 24yk | Hu95- 34yk | Hu95-44yk | Hu95- 54yk |

Exemplary amino acid sequences of full-length anti-CTGF antibody are as follows:

Table 11. The light chain or heavy chain of full-length antibody

| Full-length antibody light chain or heavy chain (SEQ ID NO) | Amino acid sequence |
|---|---|
| Ch147 heavy chain (SEQ ID NO: 41) | *EVQLVESGGGLVQPEGSLKLSCAASGFSFNTYAMNWVRQAPG KGLEWVARIRTKSNNYATYYADSVKDRFTISRDDSESMLYLQMN NLKTEDTAMYYCVETGFAYWDQGTLVTVSA*ASTKGPSVFPLAP SSKSTSGGTAALGCLVKDYFPEPVTVSWNSGALTSGVHTFP AVLQSSGLYSLSSVVTVPSSSLGTQTYICNVNHKPSNTKVD KKVEPKSCDKTHTCPPCPAPELLGGPSVFLFPPKPKDTLMIS RTPEVTCVVVDVSHEDPEVKFNWYVDGVEVHNAKTKPRE EQYNSTYRVVSVLTVLHQDWLNGKEYKCKVSNKALPAPIE KTISKAKGQPREPQVYTLPPSRDELTKNQVSLTCLVKGFYPS DIAVEWESNGQPENNYKTTPPVLDSDGSFFLYSKLTVDKSR WQQGNVFSCSVMHEALHNHYTQKSLSLSPGK |
| Ch147 light chain (SEQ ID NO: 42) | *QIVLTQSPAIMSASPGEKVTITCSASSSVSYMHWFQQKPGTSPKL WIYSTSNLASGVPARFSGSGSGTSYSLTISRMEAEDAATYYCQQR SSYPLTFGAGTKLELK*RTVAAPSVFIFPPSDEQLKSGTASVVC LLNNFYPREAKVQWKVDNALQSGNSQESVTEQDSKDSTYS LSSTLTLSKADYEKHKVYACEVTHQGLSSPVTKSFNRGEC |
| Full-length heavy chain for humanized antibodies Hu147-11wk, Hu147-12wk, Hu147-13wk, Hu147-14wk and Hu147-15wk (SEQ ID NO: 43) | *EVQLVESGGGLVQPGGSLRLSCAASGFTFSTYAMNWVRQAPG KGLEWVGRIRTKSNNYATYYADSVKDRFTISRDDSKNSLYLQMN SLKTEDTAVYYCVETGFAYWDQGTLVTVSS*ASTKGPSVFPLAPS SKSTSGGTAALGCLVKDYFPEPVTVSWNSGALTSGVHTFPA VLQSSGLYSLSSVVTVPSSSLGTQTYICNVNHKPSNTKVDK KVEPKSCDKTHTCPPCPAPELLGGPSVFLFPPKPKDTLMISR TPEVTCVVVDVSHEDPEVKFNWYVDGVEVHNAKTKPREE QYNSTYRVVSVLTVLHQDWLNGKEYKCKVSNKALPAPIEK TISKAKGQPREPQVYTLPPSRDELTKNQVSLTCLVKGFYPSD IAVEWESNGQPENNYKTTPPVLDSDGSFFLYSKLTVDKSRW QQGNVFSCSVMHEALHNHYTQKSLSLSPGK |

(continued)

| Full-length antibody light chain or heavy chain (SEQ ID NO) | Amino acid sequence |
|---|---|
| Full-length heavy chain for humanized antibodies Hu147-21wk, Hu147-22wk, Hu147-23wk, Hu147-24wk and Hu147-24wk (SEQ ID NO: 44) | *EVQLVESGGGLVQPGGSLRLSCAASGFTFNTYAMNWVRQAPG KGLEWVARIRTKSNNYATYYADSVKDRFTISRDDSKNSLYLQMN SLKTEDTAVYYCVETGFAYWDQGTLVTVSS*ASTKGPSVFPLAPS SKSTSGGTAALGCLVKDYFPEPVTVSWNSGALTSGVHTFPA VLQSSGLYSLSSVVTVPSSSLGTQTYICNVNHKPSNTKVDK KVEPKSCDKTHTCPPCPAPELLGGPSVFLFPPKPKDTLMISR TPEVTCVVVDVSHEDPEVKFNWYVDGVEVHNAKTKPREE QYNSTYRVVSVLTVLHQDWLNGKEYKCKVSNKALPAPIEK TISKAKGQPREPQVYTLPPSRDELTKNQVSLTCLVKGFYPSD IAVEWESNGQPENNYKTTPPVLDSDGSFFLYSKLTVDKSRW QQGNVFSCSVMHEALHNHYTQKSLSLSPGK |
| Full-length heavy chain for humanized antibodies Hu147-31wk, Hu147-32wk, Hu147-33wk, Hu147-34wk and Hu147-35wk (SEQ ID NO: 45) | *EVQLVESGGGLVQPGGSLRLSCAASGFSFNTYAMNWVRQAPG KGLEWVARIRTKSNNYATYYADSVKDRFTISRDDSESSLYLQMNS LKTEDTAVYYCVETGFAYWDQGTLVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| Full-length heavy chain for humanized antibodies Hu147-11yk, Hu147-12yk, Hu147-13yk, Hu147-14yk and Hu147-15yk (SEQ ID NO: 46) | *EVQLVESGGGLVQPGGSLRLSCAASGFTFSTYAMNWVRQAPG KGLEWVGRIRTKSNNYATYYADSVKDRFTISRDDSKNSLYLQMN SLKTEDTAVYYCVETGFAYWDQGTLVTVSS*ASTKGPSVFPLAPS SKSTSGGTAALGCLVKDYFPEPVTVSWNSGALTSGVHTFPA VLQSSGLYSLSSVVTVPSSSLGTQTYICNVNHKPSNTKVDK KVEPKSCDKTHTCPPCPAPELLGGPSVFLFPPKPKDTLYITR EPEVTCVVVDVSHEDPEVKFNWYVDGVEVHNAKTKPREE QYNSTYRVVSVLTVLHQDWLNGKEYKCKVSNKALPAPIEK TISKAKGQPREPQVYTLPPSRDELTKNQVSLTCLVKGFYPSD IAVEWESNGQPENNYKTTPPVLDSDGSFFLYSKLTVDKSRW QQGNVFSCSVMHEALHNHYTQKSLSLSPGK |

(continued)

| Full-length antibody light chain or heavy chain (SEQ ID NO) | Amino acid sequence |
|---|---|
| Full-length heavy chain for humanized antibodies Hu147-21yk, Hu147-22yk, Hu147-23yk, Hu147-24yk and Hu147-24yk (SEQ ID NO: 47) | *EVQLVESGGGLVQPGGSLRLSCAASGFTFNTYAMNWVRQAPG KGLEWVARIRTKSNNYATYYADSVKDRFTISRDDSKNSLYLQMN SLKTEDTAVYYCVETGFAYWDQGTLVTVSS*ASTKGPSVFPLAPS SKSTSGGTAALGCLVKDYFPEPVTVSWNSGALTSGVHTFPA VLQSSGLYSLSSVVTVPSSSLGTQTYICNVNHKPSNTKVDK KVEPKSCDKTHTCPPCPAPELLGGPSVFLFPPKPKDTLYITR EPEVTCVVVDVSHEDPEVKFNWYVDGVEVHNAKTKPREE QYNSTYRVVSVLTVLHQDWLNGKEYKCKVSNKALPAPIEK TISKAKGQPREPQVYTLPPSRDELTKNQVSLTCLVKGFYPSD IAVEWESNGQPENNYKTTPPVLDSDGSFFLYSKLTVDKSRW QQGNVFSCSVMHEALHNHYTQKSLSLSPGK |
| Full-length heavy chain for humanized antibodies Hu147-31yk, Hu147-32yk, Hu147-33yk, Hu147-34yk and Hu147-35yk (SEQ ID NO: 48) | *EVQLVESGGGLVQPGGSLRLSCAASGFSFNTYAMNWVRQAPG KGLEWVARIRTKSNNYATYYADSVKDRFTISRDDSESSLYLQMNS LKTEDTAVYYCVETGFAYWDQGTLVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLYITREP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| Full-length light chain for humanized antibodies Hu147-11wk, Hu147-21wk and Hu147-31wk (SEQ ID NO: 49) | *EIVLTQSPATLSLSPGERATLSCSASSSVSYMHWFQQKPGQSPRL LIYSTSNLASGIPARFSGSGSGTDYTLTISSLEPEDFAVYYCQQRSS YPLTFGQGTKLEIK*RTVAAPSVFIFPPSDEQLKSGTASVVCLL NNFYPREAKVQWKVDNALQSGNSQESVTEQDSKDSTYSLS STLTLSKADYEKHKVYACEVTHQGLSSPVTKSFNRGEC (It is also the full-length light chain for Hu147-11yk, Hu147-21yk and Hu147-31yk) |
| Full-length light chain for humanized antibodies Hu147-12wk, Hu147-22wk and Hu147-32wk (SEQ ID NO: 50) a | *EIVLTQSPATLSLSPGERATLSCSASSSVSYMHWFQQKPGQSPKL LIYSTSNLASGIPARFSGSGSGTDYTLTISSLEPEDFAVYYCQQRSS YPLTFGQGTKLEIK*RTVAAPSVFIFPPSDEQLKSGTASVVCLL NNFYPREAKVQWKVDNALQSGNSQESVTEQDSKDSTYSLS STLTLSKADYEKHKVYACEVTHQGLSSPVTKSFNRGEC (It is also the full-length light chain for Hu147-12yk, Hu147-22yk and Hu147-32yk) |
| Full-length light chain for humanized antibodies Hu147-13wk, Hu147-23wk and Hu147-33wk (SEQ ID NO: 51) | *EIVLTQSPATLSLSPGERATLSCSASSSVSYMHWFQQKPGQSPKL WIYSTSNLASGVPARFSGSGSGTDYTLTISSLEPEDFAVYYCQQRS SYPLTFGQGTKLEIK*RTVAAPSVFIFPPSDEQLKSGTASVVCL LNNFYPREAKVQWKVDNALQSGNSQESVTEQDSKDSTYSL SSTLTLSKADYEKHKVYACEVTHQGLSSPVTKSFNRGEC (It is also the full-length light chain for Hu147-13yk, Hu147-23yk and Hu147-33yk) |

(continued)

| Full-length antibody light chain or heavy chain (SEQ ID NO) | Amino acid sequence |
|---|---|
| Full-length light chain for humanized antibodies Hu147-14wk, Hu147-24wk and Hu147-34wk (SEQ ID NO: 52) | *DIQMTQSPSSLSASVGDRVTITCSASSSVSYMHWFQQKPGKSPK LLIYSTSNLASGVPSRFSGSGSGTDYTLTISSLQPEDFATYYCQQR SSYPL*TFGQGTKLEIKRTVAAPSVFIFPPSDEQLKSGTASVVCL LNNFYPREAKVQWKVDNALQSGNSQESVTEQDSKDSTYSL SSTLTLSKADYEKHKVYACEVTHQGLSSPVTKSFNRGEC<br><br>(It is also the full-length light chain for Hu147-14yk, Hu147-24yk and Hu147-34k) |
| Full-length light chain for humanized antibodies Hu147-15wk, Hu147-25wk and Hu147-35wk (SEQ ID NO: 53) | *DIQLTQSPSSLSASVGDRVTITCSASSSVSYMHWFQQKPGKSPK LWIYSTSNLASGVPSRFSGSGSGTDYTLTISSLQPEDFATYYCQQ RSSYPL*TFGQGTKLEIKRTVAAPSVFIFPPSDEQLKSGTASVVC LLNNFYPREAKVQWKVDNALQSGNSQESVTEQDSKDSTYS LSSTLTLSKADYEKHKVYACEVTHQGLSSPVTKSFNRGEC<br><br>(It is also the full-length light chain for Hu147-15yk, Hu147-25yk and Hu147-35yk) |
| Ch164 heavy chain (SEQ ID NO: 54) | *QVQLKQSGPGLVQPSQSLSITCTVSGFSLTTFGVHWIRQSPGK GLEWLGVIWRRGGTDYNAAFMSRLSITKDNSRSHVFFKMTSLQ TDDSAIYYCARDGGFDYWGQGTTLTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| Ch164 light chain (SEQ ID NO: 55) | *QAVVTQESALTTSPGGTVILTCRSSIGAVTTSNYANWVQEKPDH LFTGLIGGTSNRAPGVPVRFSGSLIGDKAALTITGAQAEDDAMY FCALWYSTHYVFGGGTKVTVL*GQPKANPTVTLFPPSSEELQA NKATLVCLISDFYPGAVTVAWKADGSPVKAGVETTKPSKQS NNKYAASSYLSLTPEQWKSHRSYSCQVTHEGSTVEKTVAPT EC |
| Full-length heavy chain for Hu164-57wl, Hu164-58wl and Hu164-59wl (SEQ ID NO: 56) | *EVTLKESGPVLVKPTETLTLTCTVSGFSLSTFGVHWIRQPPGKA LEWLAVIWRRGGTDYNAAFMSRLTISKDTSKSQVVLTMTNMDP VDTATYYCARDGGFDYWGQGTTVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |

(continued)

| Full-length antibody light chain or heavy chain (SEQ ID NO) | Amino acid sequence |
|---|---|
| Full-length heavy chain for Hu164-67wl, Hu164-68wl and Hu164-69wl (SEQ ID NO: 57) | *EVTLKESGPVLVKPTETLTLTCTVSGFSLSTFGVHWIRQPPGKG LEWLGVIWRRGGTDYNAAFMSRLTISKDTSKSQVVFTMTNMD PVDTATYYCARDGGFDYWGQGTTVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| Full-length heavy chain for Hu164-77wl, Hu164-78wl and Hu164-79wl (SEQ ID NO: 58) | *EVQLQESGPGLVKPSQTLSLTCTVSGFSISTFGVHWIRQHPGK GLEWIGVIWRRGGTDYNAAFMSRVTISKDTSKNQVSLKLSSVTA ADTAVYYCARDGGFDYWGQGTTVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| Full-length heavy chain for Hu164-87wl, Hu164-88wl and Hu164-89wl (SEQ ID NO: 59) | *EVQLQESGPGLVKPSQTLSLTCTVSGFSISTFGVHWIRQHPGK GLEWLGVIWRRGGTDYNAAFMSRLTISKDTSKNQVSFKLSSVTA ADTAVYYCARDGGFDYWGQGTTVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLMISRTP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |

(continued)

| Full-length antibody light chain or heavy chain (SEQ ID NO) | Amino acid sequence |
|---|---|
| Full-length heavy chain for Hu164-57yl, Hu164-58yl and Hu164-59yl (SEQ ID NO: 60) | *EVTLKESGPVLVKPTETLTLTCTVSGFSLSTFGVHWIRQPPGKA LEWLAVIWRRGGTDYNAAFMSRLTISKDTSKSQVVLTMTNMDP VDTATYYCARDGGFDYWGQGTTVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLYITREP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| Full-length heavy chain for Hu164-67yl, Hu164-68yl and Hu164-69yl (SEQ ID NO: 61) | *EVTLKESGPVLVKPTETLTLTCTVSGFSLSTFGVHWIRQPPGKG LEWLGVIWRRGGTDYNAAFMSRLTISKDTSKSQVVFTMTNMD PVDTATYYCARDGGFDYWGQGTTVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLYITREP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| Full-length heavy chain for Hu164-77yl, Hu164-78yl and Hu164-79yl (SEQ ID NO: 62) | *EVQLQESGPGLVKPSQTLSLTCTVSGFSISTFGVHWIRQHPGK GLEWIGVIWRRGGTDYNAAFMSRVTISKDTSKNQVSLKLSSVTA ADTAVYYCARDGGFDYWGQGTTVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLYITREP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |

(continued)

| Full-length antibody light chain or heavy chain (SEQ ID NO) | Amino acid sequence |
|---|---|
| Full-length heavy chain for Hu164-87yl, Hu164-88yl and Hu164-89yl (SEQ ID NO: 63) | *EVQLQESGPGLVKPSQTLSLTCTVSGFSISTFGVHWIRQHPGK GLEWLGVIWRRGGTDYNAAFMSRLTISKDTSKNQVSFKLSSVTA ADTAVYYCARDGGFDYWGQGTTVTVSS*ASTKGPSVFPLAPSS KSTSGGTAALGCLVKDYFPEPVTVSWNSGALTSGVHTFPAV LQSSGLYSLSSVVTVPSSSLGTQTYICNVNHKPSNTKVDKK VEPKSCDKTHTCPPCPAPELLGGPSVFLFPPKPKDTLYITREP EVTCVVVDVSHEDPEVKFNWYVDGVEVHNAKTKPREEQY NSTYRVVSVLTVLHQDWLNGKEYKCKVSNKALPAPIEKTIS KAKGQPREPQVYTLPPSRDELTKNQVSLTCLVKGFYPSDIA VEWESNGQPENNYKTTPPVLDSDGSFFLYSKLTVDKSRWQ QGNVFSCSVMHEALHNHYTQKSLSLSPGK |
| Full-length light chain for Hu164-57wl, Hu164-67wl, Hu164-77wl and Hu164-87wl (SEQ ID NO: 64) | *ETVVTQEPSLTVSPGGTVTLTCRSSIGAVTTSNYANWVQQKPGQ AFRGLIGGTSNRAPWTPARFSGSLLGGKAALTLSGVQPEDEAEY YCALWYSTHYVFGGGTKLTVL*GQPKANPTVTLFPPSSEELQA NKATLVCLISDFYPGAVTVAWKADGSPVKAGVETTKPSKQS NNKYAASSYLSLTPEQWKSHRSYSCQVTHEGSTVEKTVAPT EC (It is also the full-length light chain for Hu164-57yl, Hu164-67yl, Hu164-77yl and Hu164-87yl) |
| Full-length light chain for Hu164-58wl, Hu164-68wl, Hu164-78wl and Hu164-88wl (SEQ ID NO: 65) | *ETVVTQEPSFSVSPGGTVTLTCRSSIGAVTTSNYANWVQQTPGQ AFRGLIGGTSNRAPGVPDRFSGSILGNKAALTITGAQADDESDY YCALWYSTHYVFGGGTKLTVL*GQPKANPTVTLFPPSSEELQA NKATLVCLISDFYPGAVTVAWKADGSPVKAGVETTKPSKQS NNKYAASSYLSLTPEQWKSHRSYSCQVTHEGSTVEKTVAPT EC (It is also the full-length light chain for Hu164-58yl, Hu164-68yl, Hu164-78vl and Hu164-88yl) |
| Full-length light chain for Hu164-59wl, Hu164-69wl, Hu164-79wl and Hu164-89wl (SEQ ID NO: 66) | *ESVVTQPPSVSGAPGQRVTISCRSSIGAVTTSNYANWVQQLPGTA FKGLIGGTSNRAPGVPDRFSGSKSGTSASLAITGLQAEDEADYY CALWYSTHYVFGGGTKLTVL*GQPKANPTVTLFPPSSEELQAN KATLVCLISDFYPGAVTVAWKADGSPVKAGVETTKPSKQSN NKYAASSYLSLTPEQWKSHRSYSCQVTHEGSTVEKTVAPTE C (It is also the full-length light chain for Hu164-59yl, Hu164-69yl, Hu164-79yl and Hu164-89yl) |

(continued)

| Full-length antibody light chain or heavy chain (SEQ ID NO) | Amino acid sequence |
|---|---|
| Ch95 heavy chain (SEQ ID NO: 86) | *EVLLQQSGPVLVKPGPSVKISCKASGFTFT<u>NYDIH</u>WVKQSHGK SLEWIG<u>LVYPYNGGTAYNQKFKD</u>KATLTFDTSSSTAYMELNSLTS EDSAVYYCAR<u>WGLIPGTTSYFDV</u>WGTGTTVTVSS*ASTKGPSVF PLAPSSKSTSGGTAALGCLVKDYFPEPVTVSWNSGALTSGV HTFPAVLQSSGLYSLSSVVTVPSSSLGTQTYICNVNHKPSNT KVDKKVEPKSCDKTHTCPPCPAPELLGGPSVFLFPPKPKDT LMISRTPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGK |
| Ch95 light chain (SEQ ID NO: 87) | *QIVLSQSPPILSASPGEKVTMTC<u>RASSSVSYIH</u>WYQQKPRSSPKP WIY<u>ATSNLAS</u>GVPARFSGSGSGTSYSLTISRVEAEDAATYYC<u>QQW NSNPWT</u>FGGGTRLEIK*RTVAAPSVFIFPPSDEQLKSGTASVVC LLNNFYPREAKVQWKVDNALQSGNSQESVTEQDSKDSTYS LSSTLTLSKADYEKHKVYACEVTHQGLSSPVTKSFNRGEC |
| Full-length heavy chain for Hu95-11wk, Hu95-12wk, Hu95-13wk and Hu95-14wk (SEQ ID NO: 88) | *EVQLVQSGAEVKKPGASVKVSCKASGFTFT<u>NYDIH</u>WVRQAPG QRLEWMG<u>LVYPYNGGTAYNQKFKD</u>RVTLTFDTSASTAYMELSS LRSEDTAVYYCAR<u>WGLIPGTTSYFDV</u>WGQGTTVTVSS*ASTKGP SVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKP KDTLMISRTPEVTCVVVDVSHEDPEVKFNWYVDGVEVHN AKTKPREEQYNSTYRVVSVLTVLHQDWLNGKEYKCKVSN KALPAPIEKTISKAKGQPREPQVYTLPPSRDELTKNQVSLTC LVKGFYPSDIAVEWESNGQPENNYKTTPPVLDSDGSFFLYS KLTVDKSRWQQGNVFSCSVMHEALHNHYTQKSLSLSPGK |
| Full-length heavy chain for Hu95-21wk, Hu95-22wk, Hu95-23wk and Hu95-24wk (SEQ ID NO: 89) | *EVQLVQSGAEVKKPGASVKVSCKASGFTFT<u>NYDIH</u>WVRQAPG QRLEWIG<u>LVYPYNGGTAYNQKFKD</u>RATLTFDTSASTAYMELSSL RSEDTAVYYCAR<u>WGLIPGTTSYFDV</u>WGQGTTVTVSS*ASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPK DTLMISRTPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK |

(continued)

| Full-length antibody light chain or heavy chain (SEQ ID NO) | Amino acid sequence |
|---|---|
| Full-length heavy chain for Hu95-31wk, Hu95-32wk, Hu95-33wk and Hu95-34wk (SEQ ID NO: 90) | *EVQLVQSGAEVKKPGASVKVSCKASGFTFTNYDIHWVKQAPGQRLEWMGLVYPYNGGTAYNQKFKDKVTLTFDTSASTAYMELSSLRSEDTAVYYCARWGLIPGTTSYFDVWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| Full-length heavy chain for Hu95-41wk, Hu95-42wk, Hu95-43wk and Hu95-44wk (SEQ ID NO: 91) | *EVQLVQSGAEVKKPGASVKVSCKASGFTFTNYDIHWVKQAPGQRLEWIGLVYPYNGGTAYNQKFKDKATLTFDTSASTAYMELSSLRSEDTAVYYCARWGLIPGTTSYFDVWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| Full-length heavy chain for Hu95-51wk, Hu95-52wk, Hu95-53wk and Hu95-54wk (SEQ ID NO: 92) | *EVQLVQSGAEVKKPGASVKVSCRASGFTFTNYAIHWVKQAPGQRLEWMGLVYPYTGGTAYNQKFKDKVTLTFDTSASTAYMELSSLRSEDTAVYYCARWGMIPGTNSYFDVWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| Full-length antibody light chain or heavy chain (SEQ ID NO) | Amino acid sequence |
|---|---|
| Full-length heavy chain for Hu95-11yk, Hu95-12yk, Hu95-13yk and Hu95-14yk (SEQ ID NO: 93) | *EVQLVQSGAEVKKPGASVKVSCKASGFTFTNYDIHWVRQAPG QRLEWMGLVYPYNGGTAYNQKFKDRVTLTFDTSASTAYMELSS LRSEDTAVYYCARWGLIPGTTSYFDVWGQGTTVTVSS*ASTKGP SVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKP KDTLYITREPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK |
| Full-length heavy chain for Hu95-21yk, Hu95-22yk, Hu95-23yk and Hu95-24yk (SEQ ID NO: 94) | *EVQLVQSGAEVKKPGASVKVSCKASGFTFTNYDIHWVRQAPG QRLEWIGLVYPYNGGTAYNQKFKDRATLTFDTSASTAYMELSSL RSEDTAVYYCARWGLIPGTTSYFDVWGQGTTVTVSS*ASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPK DTLYITREPEVTCVVVDVSHEDPEVKFNWYVDGVEVHNAK TKPREEQYNSTYRVVSVLTVLHQDWLNGKEYKCKVSNKA LPAPIEKTISKAKGQPREPQVYTLPPSRDELTKNQVSLTCLV KGFYPSDIAVEWESNGQPENNYKTTPPVLDSDGSFFLYSKLT VDKSRWQQGNVFSCSVMHEALHNHYTQKSLSLSPGK |
| Full-length heavy chain for Hu95-31yk, Hu95-32yk, Hu95-33yk and Hu95-34yk (SEQ ID NO: 95) | *EVQLVQSGAEVKKPGASVKVSCKASGFTFTNYDIHWVKQAPG QRLEWMGLVYPYNGGTAYNQKFKDKVTLTFDTSASTAYMELSS LRSEDTAVYYCARWGLIPGTTSYFDVWGQGTTVTVSS*ASTKGP SVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKP KDTLYITREPEVTCVVVDVSHEDPEVKFNWYVDGVEVHNA KTKPREEQYNSTYRVVSVLTVLHQDWLNGKEYKCKVSNK ALPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK |

(continued)

| Full-length antibody light chain or heavy chain (SEQ ID NO) | Amino acid sequence |
|---|---|
| Full-length heavy chain for Hu95-41yk, Hu95-42yk, Hu95-43yk and Hu95-44yk (SEQ ID NO: 96) | *EVQLVQSGAEVKKPGASVKVSCKASGFTFTNYDIHWVKQAPG QRLEWIGLVYPYNGGTAYNQKFKDKATLTFDTSASTAYMELSSL RSEDTAVYYCARWGLIPGTTSYFDVWGQGTTVTVSS*ASTKGPS VFPLAPSSKSTSGGTAALGCLVKDYFPEPVTVSWNSGALTS GVHTFPAVLQSSGLYSLSSVVTVPSSSLGTQTYICNVNHKPS NTKVDKKVEPKSCDKTHTCPPCPAPELLGGPSVFLFPPKPK DTLYITREPEVTCVVVDVSHEDPEVKFNWYVDGVEVHNAK TKPREEQYNSTYRVVSVLTVLHQDWLNGKEYKCKVSNKA LPAPIEKTISKAKGQPREPQVYTLPPSRDELTKNQVSLTCLV KGFYPSDIAVEWESNGQPENNYKTTPPVLDSDGSFFLYSKLT VDKSRWQQGNVFSCSVMHEALHNHYTQKSLSLSPGK |
| Full-length heavy chain for Hu95-51yk, Hu95-52yk, Hu95-53yk and Hu95-54yk (SEQ ID NO: 97) | *EVQLVQSGAEVKKPGASVKVSCRASGFTFTNYAIHWVKQAPGQ RLEWMGLVYPYTGGTAYNQKFKDKVTLTFDTSASTAYMELSSLR SEDTAVYYCARWGMIPGTNSYFDVWGQGTTVTVSS*ASTKGPSV FPLAPSSKSTSGGTAALGCLVKDYFPEPVTVSWNSGALTSG VHTFPAVLQSSGLYSLSSVVTVPSSSLGTQTYICNVNHKPSN TKVDKKVEPKSCDKTHTCPPCPAPELLGGPSVFLFPPKPKD TLYITREPEVTCVVVDVSHEDPEVKFNWYVDGVEVHNAKT KPREEQYNSTYRVVSVLTVLHQDWLNGKEYKCKVSNKALP APIEKTISKAKGQPREPQVYTLPPSRDELTKNQVSLTCLVKG FYPSDIAVEWESNGQPENNYKTTPPVLDSDGSFFLYSKLTVD KSRWQQGNVFSCSVMHEALHNHYTQKSLSLSPGK |
| Full-length light chain for Hu95-11wk, Hu95-21wk, Hu95-31wk, Hu95-41wk and Hu95-51wk (SEQ ID NO: 98) | *EIVLTQSPATLSLSPGERATLSCRASSSVSYIHWYQQKPGQAPRP WIYATSNLASGIPARFSGSGSGTDYTLTISRLEPEDFAVYYCQQW NSNPWTFGGGTKVEIK*RTVAAPSVFIFPPSDEQLKSGTASVVC LLNNFYPREAKVQWKVDNALQSGNSQESVTEQDSKDSTYS LSSTLTLSKADYEKHKVYACEVTHQGLSSPVTKSFNRGEC (It is also the full-length light chain for Hu95-11yk, Hu95-21yk, Hu95-31yk, Hu95-41yk and Hu95-51yk) |
| Full-length light chain for Hu95-12wk, Hu95-22wk, Hu95-32wk, Hu95-42wk and Hu95-52wk (SEQ ID NO: 99) | *EIVLTQSPATLSLSPGERATLSCRASSSVSYIHWYQQKPGQSPRP WIYATSNLASGVPARFSGSGSGTSYTLTISRLEPEDFAVYYCQQW NSNPWTFGGGTKVEIK*RTVAAPSVFIFPPSDEQLKSGTASVVC LLNNFYPREAKVQWKVDNALQSGNSQESVTEQDSKDSTYS LSSTLTLSKADYEKHKVYACEVTHQGLSSPVTKSFNRGEC (It is also the full-length light chain for Hu95-12yk, Hu95-22yk, Hu95-32yk, Hu95-42yk and Hu95-52yk) |

(continued)

| Full-length antibody light chain or heavy chain (SEQ ID NO) | Amino acid sequence |
|---|---|
| Full-length light chain for Hu95-13wk, Hu95-23wk, Hu95-33wk, Hu95-43wk and Hu95-53wk (SEQ ID NO: 100) | *ESVLTQPPSVSGAPGQRVTISCRASSSVSYIHWYQQLPGTAPKP WIYATSNLASGVPDRFSGSKSGTSYSLAITGLQAEDEADYYCQQ WNSNPWTFGGGTKVEIK*RTVAAPSVFIFPPSDEQLKSGTASV VCLLNNFYPREAKVQWKVDNALQSGNSQESVTEQDSKDS TYSLSSTLTLSKADYEKHKVYACEVTHQGLSSPVTKSFNRG EC<br>(It is also the full-length light chain for Hu95-13yk, Hu95-23yk, Hu95-33yk, Hu95-43yk and Hu95-53yk) |
| Full-length light chain for Hu95-14wk, Hu95-24wk, Hu95-34wk, Hu95-44wk and Hu95-54wk (SEQ ID NO: 101) | *EIVLTQPPSVSGAPGQRVTISCRASSSVSYIHWYQQLPGTSPKPW IYATSNLASGVPDRFSGSKSGTSYSLAITGLQAEDEADYYCQQW NSNPWTFGGGTKVEIK*RTVAAPSVFIFPPSDEQLKSGTASVVC LLNNFYPREAKVQWKVDNALQSGNSQESVTEQDSKDSTYS LSSTLTLSKADYEKHKVYACEVTHQGLSSPVTKSFNRGEC<br>(It is also the full-length light chain for Hu95-14yk, Hu95-24yk, Hu95-34yk, Hu95-44yk and Hu95-54yk) |

The sequences of the positive control CTGF antibody mAb1 (from CN1829740B, with the antibody name of pamrevlumab, also known as FG-3019) are as follows:

Heavy chain: (SEQ ID NO: 67)

EGQLVQSGGGLVHPGGSLRLSCAGSGFTFSSYGMHWVRQAPGKGLEWVSGIGTGGG
TYSTDSVKGRFTISRDNAKNSLYLQMNSLRAEDMAVYYCARGDYYGSGSFFDCWGQ
GTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKRVEPKSCDKTHTC
PPCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLD
SDGSFFLYSKLTVDKSRWQQGNVFSCSVMHEALHNHYTQKSLSLSPGK;

Light chain: (SEQ ID NO: 68)

DIQMTQSPSSLSASVGDRVTITCRASQGISSWLAWYQQKPEKAPKSLIYAASSL
QSGVPSRFSGSGSGTDFTLTISSLQPEDFATYYCQQYNSYPPTFGQGTKLEIKRTVAAPS
VFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEC.

**Example 3. Detection of binding activity of CTGF antibody**

**I. ELISA assay of the binding of CTGF antibody to human CTGF protein**

The binding activity of anti-human CTGF antibody to human CTGF protein was tested by ELISA assay and Biacore. A 96-well microtiter plate was directly coated with the CTGF recombinant protein. The signal strength upon the addition of the antibody was used to determine the binding activity of the antibody to CTGF. The specific experimental procedures were as follows:

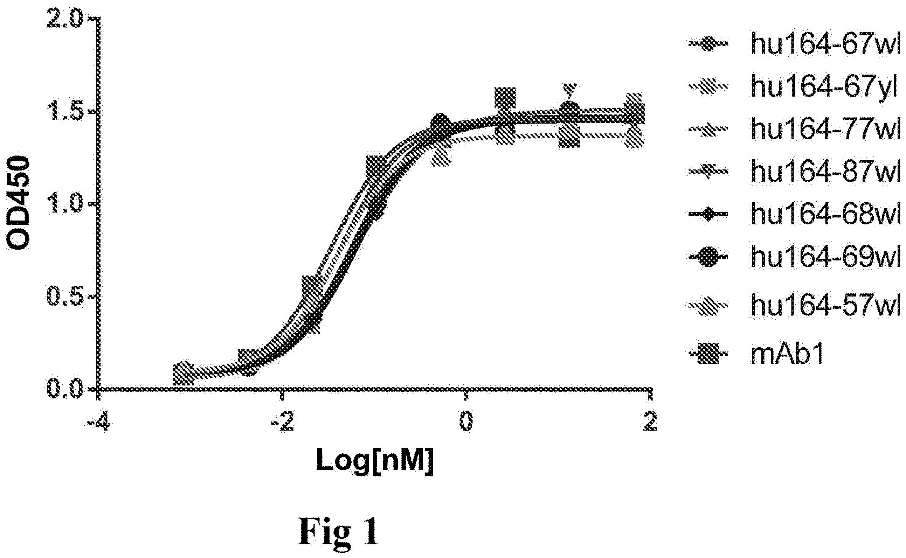

The CTGF protein (R&D, Cat No. 9190-CC) was diluted with PBS, pH7.4 (Shanghai Yuanpei, Cat No. B320) buffer to a concentration of 0.2μg/ml, 50μl/well of which was added into the 96-well microtiter plate (Corning, Cat No. CLS3590-100EA), and incubated in an incubator at 37°C for 2 hours or at 4 °C overnight (16-18 hours). The liquid was removed, 250μl/well blocking solution (5% skim milk (BD skim milk, Cat No.232100) diluted in PBS) was added, and incubated in an incubator at 37°C for 3 hours or at 4°C overnight (16-18 hours) for blocking. After the blocking was finished, the blocking solution was removed, the plate was washed for 5 times with PBST buffer (PBS containing 0.1% Tween-20, pH 7.4), 50μl/well of various concentrations of each of the test antibodies (antibodies purified from hybridoma or humanized antibodies) diluted with sample dilution solution were added, and incubated in an incubator at 37°C for 1 hours. After the incubation was finished, the plate was washed for 3 times with PBST, 50μl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample dilution solution was added, and incubated at 37°C for 1 hour. The plate was washed for 3 times with PBST, 50μl/well of TMB chromogenic substrate (KPL, Cat No. 52-00-03) was added, incubated at room temperature for 5-10min, and 50μl/well of 1M $H_2SO_4$ was added to stop the reaction. The absorbance value was read by a microplate reader (Molecular Devices, VERSA max) at a wavelength of 450 nm, and the data was analyzed with GraphPad Prism 5. The binding of CTGF antibody to human CTGF protein was calculated as EC50 value. The experimental results are shown in Figure 1 and Table 12.

Table 12: ELISA results of the binding of CTGF antibody to human CTGF protein

| Test sample | EC50 (nM) representing the binding to human CTGF protein, in ELISA assay | Test sample | EC50 (nM) representing the binding to human CTGF protein, in ELISA assay |
|---|---|---|---|
| Hu147-11wk | 0.045 | Hu164-57wl | 0.044 |
| Hu147-12wk | 0.046 | Hu164-67wl | 0.044 |
| Hu147-13wk | 0.043 | Hu164-67yl | 0.060 |
| Hu147-14wk | 0.073 | Hu164-77wl | 0.055 |
| Hu147-15wk | 0.034 | Hu164-87wl | 0.055 |
| Hu147-21wk | 0.067 | Hu164-68wl | 0.069 |
| Hu147-22wk | 0.056 | Hu164-69wl | 0.063 |
| Hu147-23wk | 0.058 | mAb1 | 0.067 |
| Hu147-24wk | 0.048 | Hu95-51yk | 0.080 |
| Hu147-31wk | 0.056 | | |
| Hu147-32wk | 0.063 | | |
| Hu147-33wk | 0.054 | | |
| Hu147-34wk | 0.134 | | |

The experimental results show that the humanized full-length anti-CTGF antibodies of the present disclosure all have excellent binding effect with the human CTGF protein.

**II. ELISA assay of the binding of CTGF antibody to cynomolgus monkey CTGF protein**

The binding activity of anti-monkey CTGF antibody to monkey CTGF protein was tested by ELISA assay. A 96-well microtiter plate was directly coated with anti-mFc protein, and then the cyno-CTGF protein with Fc tag was bound to the plate, and then the antibody was added. The signal strength upon the addition of the antibody was used to determine the binding activity of the antibody to cyno-CTGF. The specific experimental procedures were as follows:
The anti-mFc Protein (Sigma, Cat No. M4280) was diluted with PBS, pH7.4 (Shanghai Yuanpei, Cat No. B320) buffer to a concentration of 2μg/ml, which was added into the 96-well microtiter plate (Corning, Cat No. CLS3590-100EA) at 50μl/well, and incubated in an incubator at 37°C for 2 hours. The liquid was removed, 250μl/well blocking solution (5% skim milk (BD skim milk, Cat No.232100) diluted in PBS) was added, and incubated in an incubator at 37°C for 3 hours or at 4°C overnight (16-18 hours) for blocking. After the blocking was finished, the blocking solution was removed, the plate was washed for 3 times with PBST buffer (PBS containing 0.1% Tween-20, pH 7.4), 50μl of 1ug/ml cyno-CTGF-mFc protein was added into each well, incubated in an incubator at 37°C for 1 hour, and then the plate was washed for 3 times with PBST buffer (PBS containing 0.1% Tween-20, pH 7.4). 50μl/well of various concentrations of each of the test antibodies (antibodies purified from hybridoma or humanized antibodies) diluted with sample dilution solution were added, and incubated in an incubator at 37°C for 1 hours. After the incubation was finished, the plate was washed for 3 times with PBST, 50μl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample dilution solution was added, and incubated at 37°C for 1 hour. The plate was washed for 5 times with PBST, 50μl/well of TMB chromogenic substrate (KPL, Cat No. 52-00-03) was added, incubated at room temperature for 5-10min, and 50μl/well of 1M $H_2SO_4$ was added to stop the reaction. The absorbance value was read by a microplate reader (Molecular Devices, VERSA max) at a wavelength of 450 nm, and the data was analyzed with GraphPad Prism 5. The binding of CTGF antibody to cyno CTGF protein was calculated as EC50 value. The experimental results are shown in Table 13.

Table 13: ELISA results of the binding of CTGF antibody to cyno CTGF protein

| Test sample | EC50 (nM) | Test sample | EC50 (nM) | Test sample | EC50 (nM) |
|---|---|---|---|---|---|
| Hu147-11wk | 0.052 | Hu147-23wk | 0.063 | Hu164-57wl | 0.029 |
| Hu147-12wk | 0.062 | Hu147-24wk | 0.080 | Hu164-67wl | 0.028 |
| Hu147-13wk | 0.043 | Hu147-31wk | 0.070 | Hu164-77wl | 0.038 |
| Hu147-14wk | 0.077 | Hu147-32wk | 0.079 | Hu164-87wl | 0.034 |
| Hu147-15wk | 0.053 | Hu147-33wk | 0.044 | Hu164-68wl | 0.036 |
| Hu147-21wk | 0.053 | Hu147-34wk | 0.144 | Hu164-69wl | 0.032 |
| Hu147-22wk | 0.073 | Hu95-51yk | 0.059 | mAb1 | 0.086 |

The experimental results show that the humanized anti-CTGF antibodies of the present disclosure all have excellent binding effect with the monkey CTGF protein.

## III. ELISA assay of the binding of CTGF antibody to mouse CTGF protein

The binding activity of anti-mouse CTGF antibody to mouse CTGF protein was tested by ELISA assay. A 96-well microtiter plate was directly coated with the mouse CTGF fusion protein. The signal strength upon the addition of the antibody was used to determine the binding activity of the antibody to mouse CTGF. The specific experimental procedures were as follows:

The mouse CTGF-his protein was diluted with PBS, pH7.4 (Shanghai Yuanpei, Cat No. B320) buffer to a concentration of 0.5μg/ml, added into the 96-well microtiter plate (Corning, Cat No. CLS3590-100EA) at 50μl/well, and incubated in an incubator at 37°C for 2 hours. The liquid was removed, 250μl/well blocking solution (5% skim milk (BD skim milk, Cat No.232100) diluted in PBS) was added, and incubated in an incubator at 37°C for 3 hours or at 4°C overnight (16-18 hours) for blocking. After the blocking was finished, the blocking solution was removed, the plate was washed for 3 times with PBST buffer (PBS containing 0.1% Tween-20, pH 7.4), 50μl/well of various concentrations of each of the test antibodies (antibodies purified from hybridoma or humanized antibodies) diluted with sample dilution solution were added, and incubated in an incubator at 37°C for 1 hours. After the incubation was finished, the plate was washed for 3 times with PBST, 50μl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample dilution solution was added, and incubated at 37°C for 1 hour. The plate was washed for 5 times with PBST, 50μl/well of TMB chromogenic substrate (KPL, Cat No. 52-00-03) was added, incubated at room temperature for 5-10min, and 50μl/well of 1M $H_2SO_4$ was added to stop the reaction. The absorbance value was read by a microplate reader (Molecular Devices, VERSA max) at a wavelength of 450 nm, and the data was analyzed with GraphPad Prism 5. The binding of CTGF antibody to mouse CTGF protein was calculated as EC50 value. The experimental results are shown in Table 14.

Table 14: ELISA results of the binding of CTGF antibody to mouse CTGF protein

| Test sample | EC50 (nM) | Test sample | EC50 (nM) | Test sample | EC50 (nM) |
|---|---|---|---|---|---|
| Hu147-11wk | 0.228 | Hu147-23wk | 0.200 | Hu164-57wl | 0.041 |
| Hu147-12wk | 0.249 | Hu147-24wk | 0.263 | Hu164-67wl | 0.049 |

(continued)

| Test sample | EC50 (nM) | Test sample | EC50 (nM) | Test sample | EC50 (nM) |
|---|---|---|---|---|---|
| Hu147-13wk | 0.127 | Hu147-31wk | 0.320 | Hu164-77wl | 0.058 |
| Hu147-14wk | 0.431 | Hu147-32wk | 0.349 | Hu164-87wl | 0.067 |
| Hu147-15wk | 0.159 | Hu147-33wk | 0.258 | Hu164-68wl | 0.065 |
| Hu147-21wk | 0.247 | Hu147-34wk | 0.664 | Hu164-69wl | 0.059 |
| Hu147-22wk | 0.336 | | | mAb1 | 0.077 |

The experimental results show that the humanized full-length anti-CTGF antibodies of the present disclosure all have excellent binding effect with the mouse CTGF protein.

## Example 4. Function-blocking assay of CTGF antibody

In this assay, Biacore instrument was used to detect the activity of the humanized anti-CTGF antibody to be tested to block the function of human TGF-β1.

First, the antigen human CTGF was immobilized onto a CM5 biosensor chip (Cat. # BR-1005-30, GE) by amino coupling, with an immobilization level of 1500 RU, and then a high concentration of CTGF antibody (150 μg/ml) was allowed to flow through the surface of the chip for 150s; 50 nM TGF-β1 protein was injected for 120s, and the reaction signals were detected in real time with the Biacore T200 instrument to generate a binding and dissociation curve. After the dissociation was completed in each of the experimental cycles, the biosensor chip was washed and regenerated with regeneration buffer.

The resulting experimental data were statistically analyzed with GE Biacore T200 Evaluation version 3.0 software.

Blocking efficiency=[1-(response value of the sample /response value of the blank control)]×100%. The specific results are shown in Table 15.

Table 15. Function-blocking assay of CTGF antibody

| Test sample | Function-blocking percentage (%) | Test sample | Function-blocking percentage (%) |
|---|---|---|---|
| Hu147-11wk | 50.8% | Hu147-24wk | 52.8% |
| Hu147-12wk | 51.6% | Hu147-31wk | 55.0% |
| Hu147-13wk | 53.3% | Hu147-32wk | 55.2% |
| Hu147-14wk | 50.9% | Hu147-33wk | 57.3% |
| Hu147-15wk | 52.3% | Hu147-34wk | 56.2% |
| Hu147-21wk | 51.6% | Hu164-67wl | 55.5% |
| Hu147-22wk | 52.3% | Hu164-67yl | 53.9% |
| Hu147-23wk | 54.6% | mAb1 | 41.3% |

The experimental results show that the humanized anti-CTGF antibodies of the present disclosure all have high function-blocking activity.

## Determination of the affinity of anti-CTGF antibody by Biacore

In this experiment, Biacore instrument was used to determine the affinity of the humanized anti-CTGF antibodies to be tested with human CTGF and mouse CTGF.

A certain amount of each of the test antibodies was affinity-captured with Protein A biosensor chip (Cat. # 29127556, GE), and then a certain concentration of human or mouse CTGF was allowed to flow through the surface of the chip. The reaction signals were detected in real time with Biacore instrument to generate a binding and dissociation curve. After the dissociation was completed in each cycle, the biosensor chip was washed and regenerated with Glycine-HCl regeneration solution pH 1.5 (Cat. # BR-1003-54, GE). The running buffer for the assay was 1×HBS-EP buffer solution (Cat. # BR-1001-88, GE).

The resulting experimental data were fitted against the (1:1) Langmuir model using GE Biacore T200 Evaluation version 3.0 software, and the affinity values were obtained, as shown in Table 16 and 17 for details.

Table 16. The reaction affinity of CTGF antibody to human CTGF protein

| Antibody | ka (1/Ms) | kd (1/s) | KD (M) |
|---|---|---|---|
| Ch147 | 1.49E+07 | 6.93E-03 | 4.66E-10 |
| Ch164 | 6.62E+07 | 8.83E-03 | 1.33E-10 |
| Ch95 | 7.85E+06 | 3.02E-02 | 3.85E-09 |
| Hu147-11wk | 2.98E+07 | 3.36E-02 | 1.13E-09 |
| Hu147-12wk | 3.41E+07 | 3.30E-02 | 9.69E-10 |
| Hu147-13wk | 2.97E+07 | 2.40E-02 | 8.08E-10 |
| Hu147-14wk | 4.89E+07 | 5.54E-02 | 1.13E-09 |
| Hu147-21wk | 1.66E+07 | 1.62E-02 | 9.74E-10 |
| Hu147-22wk | 1.40E+07 | 1.42E-02 | 1.01E-09 |
| Hu147-23wk | 1.37E+07 | 1.12E-02 | 8.18E-10 |
| Hu147-31wk | 1.78E+07 | 1.59E-02 | 8.94E-10 |
| Hu147-32wk | 1.46E+07 | 1.33E-02 | 9.10E-10 |
| Hu147-33wk | 1.45E+07 | 1.04E-02 | 7.13E-10 |
| Hu147-34wk | 1.47E+07 | 1.55E-02 | 1.05E-09 |
| Hu164-57wl | 4.26E+07 | 2.94E-03 | 6.91E-11 |
| Hu164-67wl | 5.17E+07 | 1.96E-03 | 3.79E-11 |
| Hu164-67yl | 3.43E+07 | 2.27E-03 | 6.62E-11 |
| Hu164-77wl | 5.15E+07 | 1.76E-02 | 3.43E-10 |
| Hu164-87wl | 1.05E+08 | 2.04E-02 | 1.94E-10 |
| Hu164-68wl | 3.84E+07 | 1.16E-02 | 3.02E-10 |
| Hu164-69wl | 8.71E+07 | 2.80E-02 | 3.22E-10 |
| Hu95-21wk | 4.09E+06 | 3.03E-02 | 7.39E-09 |
| Hu95-31wk | 5.59E+06 | 3.26E-02 | 5.84E-09 |
| Hu95-41wk | 7.70E+06 | 4.08E-02 | 5.29E-09 |
| Hu95-42wk | 3.32E+06 | 1.91E-02 | 5.74E-09 |
| Hu95-51yk | 2.84E+07 | 1.02E-03 | 3.58E-11 |

Table 17. The reaction affinity of CTGF antibody to mouse CTGF protein

| Antibody | ka (1/Ms) | kd (1/s) | KD (M) |
|---|---|---|---|
| Ch147 | 3.12E+07 | 3.84E-02 | 1.23E-09 |
| Ch164 | 1.23E+08 | 1.01E-02 | 8.22E-11 |
| Ch95 | 1.38E+07 | 2.35E-02 | 1.71E-09 |
| Hu147-11wk | 1.20E+08 | 3.74E-01 | 3.11E-09 |
| Hu147-12wk | 3.75E+07 | 1.16E-01 | 3.09E-09 |
| Hu147-13wk | 5.73E+07 | 1.46E-01 | 2.55E-09 |
| Hu147-23wk | 8.18E+07 | 1.73E-01 | 2.12E-09 |

(continued)

| Antibody | ka (1/Ms) | kd (1/s) | KD (M) |
|---|---|---|---|
| Hu147-31wk | 3.83E+07 | 1.07E-02 | 2.79E-09 |
| Hu147-32wk | 1.37E+08 | 3.43E-01 | 2.49E-09 |
| Hu147-33wk | 9.01E+07 | 1.74E-01 | 1.93E-09 |
| Hu164-57wl | 7.35E+07 | 2.37E-03 | 3.22E-11 |
| Hu164-67wl | 6.36E+07 | 1.17E-03 | 1.84E-11 |
| Hu164-67yl | 5.34E+07 | 1.94E-03 | 3.63E-11 |
| Hu164-77wl | 5.98E+07 | 8.83E-03 | 1.48E-10 |
| Hu164-87wl | 5.24E+07 | 4.40E-03 | 8.40E-11 |
| Hu164-68wl | 5.70E+07 | 7.60E-03 | 1.33E-10 |
| Hu164-69wl | 6.17E+07 | 7.74E-03 | 1.26E-10 |
| Hu95-21wk | 5.29E+06 | 2.11E-02 | 3.99E-09 |
| Hu95-31wk | 7.84E+06 | 2.25E-02 | 2.87E-09 |
| Hu95-41wk | 1.39E+07 | 3.39E-02 | 2.44E-09 |
| Hu95-42wk | 5.66E+06 | 1.37E-02 | 2.42E-09 |
| Hu95-51yk | 2.56E+07 | 5.16E-04 | 2.02E-11 |

The experimental results show that the chimeric antibodies Ch147 and Ch164 and the humanized anti-CTGF antibodies derived from the murine antibodies mAb147 and mAb164 all can bind to human and mouse CTGF with high affinity. Neither the replacements nor the back mutation(s) present in the human germline antibody FR regions interferes with the affinity of the humanized antibody, and some modifications can even enhance the affinity of the antibody to the antigen.

**Example 5. The assay of CTGF antibody for *in vitro* inhibiting the migration of C2C12 cells induced by TGFβ**

C2C12 cells (mouse myoblasts, Cell Bank of Chinese Academy of Sciences, #GNM26) were digested with 0.25% trypsin (Gibco, #25200-072), centrifuged and resuspended in serum-free DMEM medium (Gibco, #10564-029). The cell-antibody mixture and TGFβ1-antibody mixture were prepared with serum-free DMEM medium, in which the cell density was $2 \times 10^5$/ml, the concentration of the recombinant human TGFβ1 (Cell signaling Technology, #8915LC) was 10 ng/ml, and the concentration of the antibody to be tested was 30µg/ml.

The cover and filter membrane of the ChemoTx ® Disposable Chemotaxis System (Neuro Probe, #106-8) were opened, and the TGFβ-antibody mixture was added into the lower chamber, 30 µl per well, 4 pairs of wells for each group. The filter membrane was covered, and the cell-antibody mixture was added onto the membrane, 50 µl per well, 4 pairs of wells for each group. The cover was closed and placed in an incubator (37 °C, 5% $CO_2$).

48 hours after incubation, the liquid on the filter membrane was removed with a clean paper towel, the filter membrane was opened, 10µl of pre-cooled 0.25% trypsin was added to each well into the lower chamber, and the filter membrane was covered. 5 minutes after digestion, the samples were centrifuged at 1000 rpm for 1 minute. The filter membrane was opened, 20 µl Cell Titer-Glo solution (Promega, #G7573) was added to each well into the lower chamber, and incubated at room temperature for 10 minutes. The liquid was transfered to a 384-well white bottom plate (Thermo Scientific, #267462), and the plate was read with a microplate reader (BMG, #PheraStar) by chemiluminescence. Data were analyzed and processed with Graphpad Prism 6.

$$\text{Inhibition rate} = [(\text{average value of TGF}\beta - \text{average value of sample group})/(\text{average value of TGF}\beta \text{ group} - \text{average value of untreated group})] \times 100\%$$

The experimental results are shown in Table 18.

Table 18. Results showing the inhibitory effect of CTGF antibody on the migration of C2C12 cells *in vitro*

| Test sample | Inhibition percentage of C2C12 cell migration *in vitro* (%) |
|---|---|
| Hu147-11wk | 72.2% |
| Hu147-33wk | 63.8% |
| Hu164-57wl | 75.4% |
| Hu164-67wl | 78.8% |
| Hu164-67yl | 76.5% |
| Hu164-77wl | 72.8% |
| Hu164-87wl | 76.4% |
| Hu164-68wl | 76.3% |
| Hu164-69wl | 74.2% |
| Hu95-51yk | 80.0% |
| mAb1 | 63.0% |

The results show that the humanized anti-CTGF antibodies of the present disclosure can inhibit the migration ability of C2C12 cells *in vitro* induced by TGFβ1, and show stronger inhibitory effect on the migration ability of C2C12 cells when compared to that of the positive control antibody mAb1.

**Example 6. The assay showing the *in vitro* inhibitory effect of CTGF antibody on the migration of PANC1 cells induced by TGFβ**

PANC-1 cells (human pancreatic cancer cells, ATCC, #CRL-1469) were digested with 0.25% trypsin (Gibco, #25200-072), centrifuged and resuspended with DMEM medium (Gibco, #10564-029) containing 0.1% fetal bovine serum (Gibco, #10099-141). The cell-antibody mixture and TGFβ-antibody mixture were prepared with DMEM medium containing 0.1% fetal bovine serum, in which the cell density was $4 \times 10^5$/ml, the concentration of the recombinant human TGFβ (Cell signaling Technology, #8915LC) was 10 ng/ml, and the concentration of the antibody to be tested was 30μg/ml.
The cover and filter membrane of the ChemoTx ® Disposable Chemotaxis System (Neuro Probe, #106-8) were opened, and the TGFβ-antibody mixture was added into the lower chamber, 30 μl per well, 4 pairs of wells for each group. The filter membrane was covered, and the cell-antibody mixture was added onto the membrane, 50 μl per well, 4 pairs of wells for each group. The cover was closed and placed in an incubator (37 °C, 5% $CO_2$).
48 hours after incubation, the liquid on the filter membrane was removed with a clean paper towel, the filter membrane was opened, 10μl of pre-cooled 0.25% trypsin was added to each well into the lower chamber, and the filter membrane was covered. 5 minutes after digestion, the samples were centrifuged at 1000 rpm for 1 minute. The cells which were adherent to the bottom of the plate were counted under an inverted microscope (Leica, #DMIL LED). Data were analyzed and processed with Graphpad Prism 6. Inhibition rate = [(average value of TGFβ - average value of sample group)/(average value of TGFβ group - average value of untreated group)]×100%
The experimental results are shown in Table 19.

Table 19. Results showing the *in vitro* inhibitory effect of CTGF antibody on migration of PANC1 cells

| Test sample | Inhibition percentage of PANC1 cell migration *in vitro* (%) | Test sample | Inhibition percentage of PANC1 cell migration *in vitro* (%) |
|---|---|---|---|
| Hu147-11wk | 73.9% | Hu164-77wl | 74.3% |
| Hu147-33wk | 77.0% | Hu164-87wl | 82.1% |
| Hu164-57wl | 81.2% | Hu164-68wl | 76.9% |
| Hu164-67wl | 75.7% | Hu164-69wl | 76.9% |
| Hu164-67yl | 78.0% | mAb1 | 71.7% |
| Hu95-51yk | 80.0% | | |

# EP 3 981 787 A1

The results show that the humanized anti-CTGF antibodies of the present disclosure all can inhibit the migration ability of PANC1 cells *in vitro* induced by TGFβ1.

**Example 7. Assay of CTGF antibody for *in vitro* inhibitory effect on the proliferation of PANC1 on soft agar**

The deionized water was added into the agar (BD, #214220) and heated to prepare a 1.4% gel solution. 2×DMEM medium (Thermo, #12100046) was mixed with the gel solution in a ratio of 1:1 by volume, 500 μl was added to each well of a 24-well plate (Costar, #3524), and placed at 4 degrees for solidification.

PANC-1 cells (human pancreatic cancer cells, ATCC, #CRL-1469) were digested with 0.25% trypsin (Gibco, #25200-072), centrifuged and adjusted with DMEM medium (GE, #SH30243.01) containing 4% fetal bovine serum (Gibco, #10099-141) to make the cell density $5\times10^4$ cells/ml. 2 mg/ml antibody was prepared with 2×DMEM medium. The cells, antibody and 1.4% gel solution were mixed at a ratio of 2:1:1 by volume, and 200 μl was added to each well of a 24-well plate covered with a lower layer of gel. After the upper gel has solidified, 200 μl of DMEM medium containing 2% fetal bovine serum was added to the 24-well plate. 28 days after incubation in an incubator (37 °C, 5% $CO_2$), a high-content analysis system (Molecular Devices, ImageXpress) was used to develop imaging, and the area of the formed cell clone was analyzed. Inhibition rate=(1-average value of sample group/average value of untreated group)×100%. The experimental results are shown in Table 20.

Table 20. Results showing the inhibitory effect of CTGF antibody on the proliferation of PANC1 cells on soft agar *in vitro*

| Test sample | Inhibition percentage of the *in vitro* proliferation of PANC1 cells (%) |
|---|---|
| Hu147-11wk | 23.76% |
| Hu147-33wk | 22.30% |
| Hu164-67wl | 24.2% |
| Hu164-67yl | 21.7% |
| Hu95-51yk | 20.24% |

The results show that the humanized antibodies derived from mAb147 and mAb164 clones have a significant inhibitory effect on the proliferation of human pancreatic cancer cells (PANC-1 cells) *in vitro*.

**Example 8: *In vivo* biological activity of CTGF antibody in animals**

**I. Assay of CTGF antibody for its inhibitory effect on mouse pulmonary fibrosis induced by bleomycin**

Experimental protocol: 40 mice were randomly divided into the following 4 groups according to the body weight: normal control group (PBS, i.p., qod), mAb1 group (10mg/kg, i.p., qod), Hu164-67wl (10mg/kg, i.p., qod) group and Hu164-67yl (10mg/kg, i.p., qod) group. There are 10 animals in each group, and the specific grouping is shown in Table 21 below. On day 1 of the experiment, each of the solvents and antibodies was intraperitoneally injected according to the body weight (10ml/kg, normal control and model groups were provided with PBS); 2 hours later, aerosol bleomycin was used (50mg/8ml nebulization for 30 minutes and holding for 5 minutes) to establish models. Then, solvent or antibody was intraperitoneally injected every other day according to the above groups. The experimental period was 21 days, with a total of 11 intraperitoneal administrations. On day 22 of the experiment, the mice were anesthetized by intraperitoneal injection of 1% sodium pentobarbital (10ml/kg), and then were fixed on the operating table on supine position. A 1cm skin incision was made along the midline of the neck, and the lower end of the incision reached the entrance of chest. Forceps was used to bluntly separate the subcutaneous connective tissues and muscles to expose the trachea. The connective tissues on both sides of the trachea and between the trachea and the esophagus were separated to isolate the trachea free. A venous indwelling needle was inserted, and fixed with sutures by tying at a place where the cannula entering into the trachea. A 1ml syringe filled with 0.8ml PBS was connected to the inlet end of the venous indwelling needle, the PBS was slowly injected into the trachea, stayed for 30 seconds, and then the PBS was slowly withdrawn back. A milky foamy liquid could be observed during withdrawal. The lavage was repeated for three times, the lavage fluid was transfered into EP tubes, and then 0.5ml PBS was taken, the above steps were repeated. The two lavage fluids were mixed and centrifuged at 1500rpm for 5min, and the supernatant was stored at -20°C for testing. The BALF supernatant was centrifuged again at 10000rpm for 10min and the supernatant was taken to detect the soluble collagen (kit: Biocolor, Batch No. AA883).

Table 21. Test grouping and dosing regimen

| Group | Number of animals | Agent | Dose (mg/ml) | Route of administration | Frequency of administration |
|---|---|---|---|---|---|
| 1 | 10 | PBS | - | i.p. | Once every other day |
| 2 | 10 | mAb1 | 10 | i.p. | Once every other day |
| 3 | 10 | Hu164-67wl | 10 | i.p. | Once every other day |
| 4 | 10 | Hu164-67yl | 10 | i.p. | Once every other day |

Excel statistical software was used: the average value was calculated as avg (Average); SD (Standard Diviation) value was calculated as STDEV (Standard Diviation); SEM (Standard Error of Mean; Standard Deviation of the Sample Mean) value was calculated as STDEV/SQRT (Square root) (number of animals in each group); GraphPad Prism software was used for plotting, Two-way ANOVA (two-way analysis of variance) or One-way ANOVA (one-way analysis of variance) was used for statistical analysis of data.

The experimental results are shown in Table 22.

Table 22. Experimental result showing the inhibitory effect of CTGF antibody on mouse pulmonary fibrosis induced by bleomycin

| Agent | mAb1 (10mpk) | Hu164-67wl (10mpk) | Hu164-67yl (10mpk) |
|---|---|---|---|
| Increased collagen inhibition rate | 53.2% | 72.8% (p<0.05) | 80.7% (p<0.05) |

The results show that Hu164-67wl or Hu164-67yl show a strong inhibitory effect on pulmonary fibrosis in mice.

**II. Assay of CTGF antibody for inhibiting the subcutaneously transplanted tumor of Su86.86 human pancreatic cancer cell**

Experimental protocol: 200 $\mu$l of SU86.86 cells (ATCC, CRL-1837, $3.0 \times 10^6$ cells) were inoculated subcutaneously into the right ribs of Nu/Nu nude mice. 11 days after the inoculation, when the tumor volume was about 140mm$^3$, the mice with too high or low small body weight or tumor volume were excluded. The mice were randomly divided into 5 groups according to the tumor volume, 10 mice per group, and the administration was started on the same day. The antibody was injected intraperitoneally, twice a week, for a total of 18 days. The tumor volume and body weight were measured 1-2 times per week, and the data were recorded. Grouping and dosing regimen are shown in Table 23.

Table 23. Test grouping and dosing regimen

| Group | Number of animals | Agent | Dose (mg/kg) | Route of administration | Frequency of administration |
|---|---|---|---|---|---|
| 1 | 10 | Human IgG | 40 | i.p. | twice a week |
| 2 | 10 | mAb1 | 40 | i.p. | twice a week |
| 3 | 10 | Hu164-67yl | 40 | i.p. | twice a week |
| 4 | 10 | Hu164-67yl | 20 | i.p. | twice a week |
| 5 | 10 | Hu147-33wk | 40 | i.p. | twice a week |

Excel statistical software was used: the average value was calculated as avg; SD value was calculated as STDEV; SEM value was calculated as STDEV/SQRT (number of animals in each group); GraphPad Prism software was used for plotting, Two-way ANOVA or One-way ANOVA was used for statistical analysis of data.

The tumor volume (V) was calculated according to the following formula:

$$V = 1/2 \times L_{long} \times L_{short}^2$$

Relative tumor proliferation rate T/C (%)=(Tt -To)/(Ct -C$_0$)×100, where Tt and Ct are the tumor volumes of the treatment group and the control group at the end of the experiment; To and Co are the tumor volume at the beginning of the experiment.

$$\text{Tumor inhibition rate TGI (\%)} = 1 - T/C\ (\%).$$

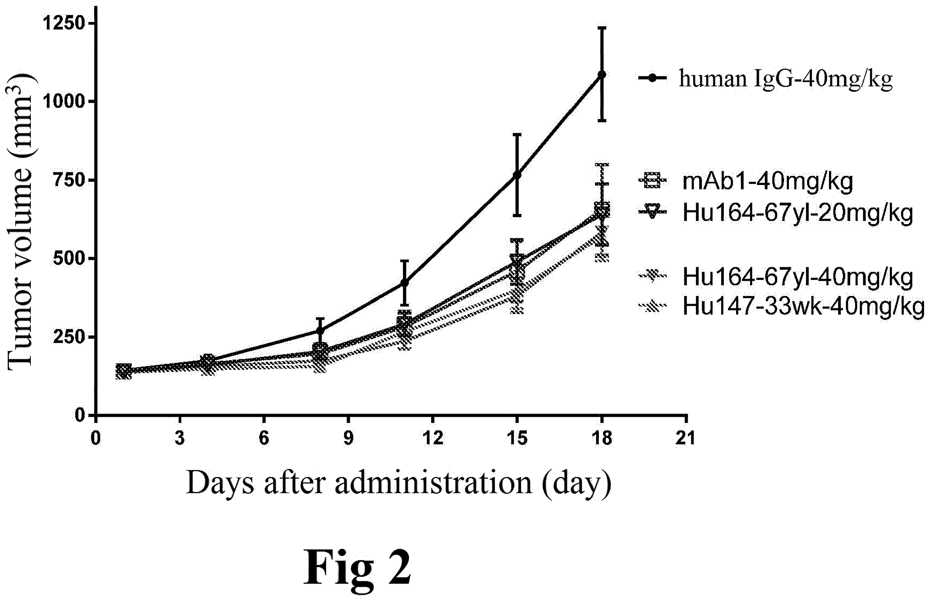

Experimental results:
From D1 to D18, subcutaneous injection was performed twice a week, and the effect of CTGF antibodies on inhibiting the growth of SU86.86 tumor was detected. The experimental results are shown in Table 24 and Figure 2. Compared with the blank control group of the same isotype human IgG, the tumor inhibition rates of mAb1-40mg/kg, Hu164-67yl-40mg/kg, Hu164-67yl-20mg/kg and Hu147-33wk (40mg/kg) group were 45%, 53%, 47% and 54%, respectively. mAb1-40mg/kg, Hu164-67yl -40mg/kg, Hu164-67yl-20mg/kg and Hu147-33wk (40mg/kg) groups can significantly inhibit the growth of Su86.86 tumors (p<0.001), and the inhibitory effect on SU86.86 tumor is dose-dependent. In addition, the body weight of mice in each group did not change significantly.

Table 24. Experimental results showing the inhibitory effect of CTGF antibody on SU86.86 xenograft in mice

| Grouping | Average tumor volume (mm$^3$) | | Average tumor volume (mm$^3$) | | % Tumor inhibitio n rate | P (vs blank control) |
|---|---|---|---|---|---|---|
| | D0 | SEM | D18 | SEM | D18 | |
| Human IgG | 144.35 | 12.30 | 1087.1 0 | 148.21 | - | |
| mAb1 (40mg/kg) | 141.18 | 11.72 | 655.75 | 144.46 | 45 | p<0.001 |
| Hu164-67yl (40mg/kg) | 138.75 | 8.71 | 582.95 | 76.14 | 53 | p<0.001 |
| Hu164-67yl (20mg/kg) | 139.87 | 9.33 | 640.26 | 96.70 | 47 | p<0.001 |
| Hu147-33wk (40mg/kg) | 138.00 | 10.11 | 569.66 | 77.40 | 54 | p<0.001 |

**Claims**

1. An anti-CTGF antibody comprising a heavy chain variable region and a light chain variable region, wherein:

i) the heavy chain variable region comprises the same HCDR1, HCDR2 and HCDR3 sequences as those of the heavy chain variable region shown in SEQ ID NO: 6, and the light chain variable region comprises the same LCDR1, LCDR2 and LCDR3 sequences as those of the light chain variable region shown in SEQ ID NO: 7;
ii) the heavy chain variable region comprises the same HCDR1, HCDR2 and HCDR3 sequences as those of the heavy chain variable region shown in SEQ ID NO: 8, and the light chain variable region comprises the same LCDR1, LCDR2 and LCDR3 sequences as those of the light chain variable region shown in SEQ ID NO: 9;
ii-i) the heavy chain variable region comprises the same HCDR1, HCDR2 and HCDR3 sequences as those of the heavy chain variable region shown in SEQ ID NO: 69, and the light chain variable region comprises the same LCDR1, LCDR2 and LCDR3 sequences as those of the light chain variable region shown in SEQ ID NO: 70; or
ii-ii) the heavy chain variable region comprises the same HCDR1, HCDR2 and HCDR3 sequences as those of the heavy chain variable region shown in SEQ ID NO: 85, and the light chain variable region comprises the same LCDR1, LCDR2 and LCDR3 sequences as those of the light chain variable region shown in SEQ ID NO: 70.

2. The anti-CTGF antibody according to claim 1, comprising a heavy chain variable region and a light chain variable region, wherein:

iii) the heavy chain variable region comprises HCDR1, HCDR2 and HCDR3 as shown in SEQ ID NO: 10, SEQ ID NO: 11 and SEQ ID NO: 12, respectively, and the light chain variable region comprises LCDR1, LCDR2 and LCDR3 as shown in SEQ ID NO: 13, SEQ ID NO: 14 and SEQ ID NO: 15, respectively;
iv) the heavy chain variable region comprises HCDR1, HCDR2 and HCDR3 as shown in SEQ ID NO: 16, SEQ ID NO: 17 and SEQ ID NO: 18, respectively, and the light chain variable region comprises LCDR1, LCDR2 and LCDR3 as shown in SEQ ID NO: 19, SEQ ID NO: 20 and SEQ ID NO: 21, respectively;
iv-i) the heavy chain variable region comprises HCDR1, HCDR2 and HCDR3 as shown in SEQ ID NO: 71, SEQ ID NO: 72 and SEQ ID NO: 73, respectively, and the light chain variable region comprises LCDR1, LCDR2 and LCDR3 as shown in SEQ ID NO: 74, SEQ ID NO: 75 and SEQ ID NO: 76, respectively; or
iv-ii) the heavy chain variable region comprises HCDR1, HCDR2 and HCDR3 as shown in SEQ ID NO: 102, SEQ ID NO: 103 and SEQ ID NO: 104, respectively, and the light chain variable region comprises LCDR1, LCDR2 and LCDR3 as shown in SEQ ID NO: 74, SEQ ID NO: 75 and SEQ ID NO: 76, respectively.

3. The anti-CTGF antibody according to claim 1 or 2, wherein the anti-CTGF antibody is a murine antibody, a chimeric antibody or a humanized antibody.

4. The anti-CTGF antibody according to any one of claims 1 to 3, comprising a heavy chain variable region and a light chain variable region, wherein:

(v) the amino acid sequence of the heavy chain variable region has at least 90% sequence identity to SEQ ID NO: 6, 27, 28, or 29, and the amino acid sequence of the light chain variable region has at least 90% sequence identity to SEQ ID NO: 7, 22, 23, 24, 25 or 26;
(vi) the amino acid sequence of the heavy chain variable region has at least 90% sequence identity to SEQ ID NO: 8, 33, 34, 35, or 36, and the amino acid sequence of the light chain variable region has at least 90% sequence identity to SEQ ID NO: 9, 30, 31 or 32; or
(vi-i) the amino acid sequence of the heavy chain variable region has at least 90% sequence identity to SEQ ID NO: 69, 81, 82, 83, 84, or 85, and the amino acid sequence of the light chain variable region has at least 90% sequence identity to SEQ ID NO: 70, 77, 78, 79, or 80.

5. The anti-CTGF antibody according to claim 2, wherein the anti-CTGF antibody is a humanized antibody, and the humanized antibody comprises a framework region of a human antibody or a framework region variant thereof, and the framework region variant has up to 10 back mutation(s) on each of the human antibody light chain framework region and/or the heavy chain framework region; preferably, the humanized antibody comprises a light chain variable region and a heavy chain variable region selected from the group consisting of the following (a), (b) or (b-i):

(a) a light chain variable region comprising: LCDR1, LCDR2 and LCDR3 as shown in SEQ ID NO: 13, SEQ ID NO: 14 and SEQ ID NO: 15, respectively, and one or more back mutation(s) selected from the group consisting of 4L, 36F, 43S, 45K, 47W, 58V and 71Y, and
a heavy chain variable region comprising:
HCDR1, HCDR2 and HCDR3 as shown in SEQ ID NO: 10, SEQ ID NO: 11 and SEQ ID NO: 12, respectively, and one or more back mutation(s) selected from the group consisting of 28S, 30N, 49A, 75E, 76S, 93V, 94E and 104D;
(b) a light chain variable region comprising:
LCDR1, LCDR2 and LCDR3 as shown in SEQ ID NO: 19, SEQ ID NO: 20 and SEQ ID NO: 21, respectively, and one or more back mutation(s) selected from the group consisting of 36V, 44F, 46G and 49G, and
a heavy chain variable region comprising:
HCDR1, HCDR2 and HCDR3 as shown in SEQ ID NO: 16, SEQ ID NO: 17 and SEQ ID NO: 18, respectively, and one or more back mutation(s) selected from the group consisting of 44G, 49G, 27F, 48L, 67L, 71K, 78V and 80F;
(b-i) a light chain variable region comprising:
LCDR1, LCDR2 and LCDR3 as shown in SEQ ID NO: 74, SEQ ID NO: 75 and SEQ ID NO: 76, respectively, and one or more back mutation(s) selected from the group consisting of 45P, 46W, 48Y, 69S and 70Y, and
a heavy chain variable region comprising:
HCDR1, HCDR2 and HCDR3 as shown in SEQ ID NO: 71, SEQ ID NO: 72 and SEQ ID NO: 73, respectively, and one or more back mutation(s) selected from the group consisting of 27F, 38K, 48I, 67K, 68A, 70L and 72F; or
(b-ii) a light chain variable region comprising:
LCDR1, LCDR2 and LCDR3 as shown in SEQ ID NO: 74, SEQ ID NO: 75 and SEQ ID NO: 76, respectively, and one or more back mutation(s) selected from the group consisting of 45P, 46W, 48Y, 69S and 70Y, and
a heavy chain variable region comprising:
HCDR1, HCDR2 and HCDR3 as shown in SEQ ID NO: 102, SEQ ID NO: 103 and SEQ ID NO: 104, respectively, and one or more back mutation(s) selected from the group consisting of 27F, 38K, 48I, 67K, 68A, 70L and 72F.

6. The anti-CTGF antibody according to claim 5, comprising a heavy chain variable region and a light chain variable region, wherein:

(vii) the amino acid sequence of the heavy chain variable region is as shown in SEQ ID NO: 6, and the amino acid sequence of the light chain variable region is as shown in SEQ ID NO: 7;
(viii) the amino acid sequence of the heavy chain variable region is as shown in SEQ ID NO: 27, 28 or 29, and the amino acid sequence of the light chain variable region is as shown in SEQ ID NO: 22, 23, 24, 25 or 26;
(ix) the amino acid sequence of the heavy chain variable region is as shown in SEQ ID NO: 8, and the amino acid sequence of the light chain variable region is as shown in SEQ ID NO: 9;
(x) the amino acid sequence of the heavy chain variable region is as shown in SEQ ID NO: 33, 34, 35 or 36, and the amino acid sequence of the light chain variable region is as shown in SEQ ID NO: 30, 31 or 32;
(xi) the amino acid sequence of the heavy chain variable region is as shown in SEQ ID NO: 69, and the amino acid sequence of the light chain variable region is as shown in SEQ ID NO: 70; or
(xii) the amino acid sequence of the heavy chain variable region is as shown in SEQ ID NO: 81, 82, 83, 84, or 85, and the amino acid sequence of the light chain variable region is as shown in SEQ ID NO: 77, 78, 79 or 80; preferably, it comprises a heavy chain variable region and a light chain variable region as shown below:
(xiii) the amino acid sequence of the heavy chain variable region is as shown in SEQ ID NO: 27, and the amino acid sequence of the light chain variable region is as shown in SEQ ID NO: 22;
(xiv) the amino acid sequence of the heavy chain variable region is as shown in SEQ ID NO: 34, and the amino acid sequence of the light chain variable region is as shown in SEQ ID NO: 30; or
(xv) the amino acid sequence of the heavy chain variable region is as shown in SEQ ID NO: 85, and the amino acid sequence of the light chain variable region is as shown in SEQ ID NO: 77.

7. The anti-CTGF antibody according to any one of claims 1 to 6, wherein the antibody further comprises an antibody heavy chain constant region and a light chain constant region; preferably, the heavy chain constant region is selected from the group consisting of the constant regions of human IgG1, IgG2, IgG3 and IgG4 and variants thereof, and the light chain constant region is selected from the group consisting of the constant regions of human antibody κ and λ chains and variants thereof; more preferably, the antibody comprises a heavy chain constant region as shown in SEQ ID NO: 37 or 38 and a light chain constant region as shown in SEQ ID NO: 39 or 40.

8. The anti-CTGF antibody according to any one of claims 1 to 7, comprising:

(c) a heavy chain having at least 85% sequence identity to SEQ ID NO: 41, 43, 44, 45, 46, 47, or 48, and a light chain having at least 85% sequence identity to SEQ ID NO: 42, 49, 50, 51, 52 or 53;

(d) a heavy chain having at least 85% sequence identity to SEQ ID NO: 54, 56, 57, 58, 59, 60, 61, 62, or 63, and a light chain having at least 85% sequence identity to SEQ ID NO: 55, 64, 65 or 66;

(e) a heavy chain as shown in SEQ ID NO: 41, 43, 44, 45, 46, 47 or 48, and a light chain as shown in SEQ ID NO: 42, 49, 50, 51, 52 or 53;

(f) a heavy chain as shown in SEQ ID NO: 54, 56, 57, 58, 59, 60, 61, 62 or 63, and a light chain as shown in SEQ ID NO: 55, 64, 65 or 66;

(g) a heavy chain having at least 85% sequence identity to SEQ ID NO: 86, 88, 89, 90, 91, 92, 93, 94, 95, 96, or 97, and a light chain having at least 85% sequence identity to SEQ ID NO: 87, 98, 99, 100 or 101; or

(h) a heavy chain as shown in SEQ ID NO: 86, 88, 89, 90, 91, 92, 93, 94, 95, 96 or 97, and a light chain as shown in SEQ ID NO: 87, 98, 99, 100 or 101;

preferably, comprising:

(j) a heavy chain as shown in SEQ ID NO: 46, and a light chain as shown in SEQ ID NO: 49;

(k) a heavy chain as shown in SEQ ID NO: 61, and a light chain as shown in SEQ ID NO: 64; or

(l) a heavy chain as shown in SEQ ID NO: 97, and a light chain as shown in SEQ ID NO: 98.

9. An isolated anti-CTGF antibody, wherein the isolated anti-CTGF antibody competitively with the anti-CTGF antibody of any one of claims 1 to 8, for the binding to human CTGF.

10. A nucleic acid molecule encoding the anti-CTGF antibody of any one of claims 1 to 9.

11. A host cell comprising the nucleic acid molecule of claim 10.

12. A pharmaceutical composition comprising a therapeutically effective amount of the anti-CTGF antibody according to any one of claims 1 to 9, or the nucleic acid molecule according to claim 10, and one or more pharmaceutically acceptable carriers, diluents, buffers or excipients.

13. A method for the immunoassay or determination of CTGF, the method comprising a step of contacting the anti-CTGF antibody of any one of claims 1 to 9 with a subject or a sample thereof.

14. A kit comprising the anti-CTGF antibody according to any one of claims 1 to 9.

15. A method for the treatment or prevention of CTGF-related disease, the method comprising administering to a subject a therapeutically effective amount or a prophylactically effective amount of the anti-CTGF antibody according to any one of claims 1 to 9, or the nucleic acid molecule of claim 10, or the pharmaceutical composition of claim 12, wherein the CTGF-related disease is preferably fibrous diseases, hypertension, diabetes, myocardial infarction, arthritis, CTGF-related cell proliferative disease, atherosclerosis, glaucoma or cancer;

preferably, the fibrous disease is selected from the group consisting of the group consisting of:

spontaneous pulmonary fibrosis, diabetic nephropathy, diabetic retinopathy, osteoarthritis, scleroderma, chronic heart failure, liver cirrhosis or renal fibrosis;

preferably, the cancer is selected from the group consisting of the group consisting of: acute lymphoblastic leukemia, dermatofibroma, breast cancer, angiolipoma, angioleiomyoma, connective tissue-generating cancer, prostate cancer, ovarian cancer, colorectal cancer, pancreatic cancer, gastrointestinal cancer or liver cancer.

Fig 1

**Fig 2**

EP 3 981 787 A1

**INTERNATIONAL SEARCH REPORT**

| International application No. |
| --- |
| **PCT/CN2020/094136** |

| A. | CLASSIFICATION OF SUBJECT MATTER |
| --- | --- |

C07K 16/22(2006.01)i; C12N 15/13(2006.01)i; C12N 15/63(2006.01)i; A61K 39/395(2006.01)i; G01N 33/53(2006.01)i; A61P 9/10(2006.01)i; A61P 9/12(2006.01)i; A61P 19/02(2006.01)i; A61P 27/06(2006.01)i; A61P 35/00(2006.01)i; A61P 1/16(2006.01)i; A61P 3/10(2006.01)i; A61P 11/00(2006.01)i; A61P 13/12(2006.01)i; A61P 17/00(2006.01)i

According to International Patent Classification (IPC) or to both national classification and IPC

| B. | FIELDS SEARCHED |
| --- | --- |

Minimum documentation searched (classification system followed by classification symbols)

C07K; C12N; A61K; G01N; A61P

Documentation searched other than minimum documentation to the extent that such documents are included in the fields searched

Electronic data base consulted during the international search (name of data base and, where practicable, search terms used)

CNMED; CNABS; CPEA; TWMED; DWPI; SIPOABS; EPOQUE; CNKI; ISI; ELESEVER; NCBI; PUBMED; GOOGLE; GenBank; EMBL; STN; 万方; 中国专利生物序列检索系统数据库: 结缔组织生长因子, connective tissue growth factor, CTGF, 抗体, antibody, SEQ ID NOS: 6-7, 22-29

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 104011206 A (ASTELLAS PHARMA INC.) 27 August 2014 (2014-08-27) see entire document | 1-14 (in part) |
| A | CN 1829740 A (FIBROGEN, INC.) 06 September 2006 (2006-09-06) see entire document | 1-14 (in part) |
| A | CN 101884789 A (FIBROGEN INC.) 17 November 2010 (2010-11-17) see entire document | 1-14 (in part) |

☐ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **24 August 2020** | **09 September 2020** |

| Name and mailing address of the ISA/CN | Authorized officer |
| --- | --- |
| **China National Intellectual Property Administration (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/094136**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.

**PCT/CN2020/094136**

---

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **15**
   because they relate to subject matter not required to be searched by this Authority, namely:

   [1]   Disease treatment methods (PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III      Observations where unity of invention is lacking (Continuation of item 3 of first sheet)**

This International Searching Authority found multiple inventions in this international application, as follows:

[1]   Group 1: claims 1-14 (in part), setting forth technical solutions relating to an antibody having heavy chain variable region and light chain variable region sequences as shown respectively in SEQ ID NOs: 6 and 7, or an antibody having a heavy chain variable region sequence as shown in SEQ ID NO: 27, 28 or 29 and a light chain variable region sequence as shown in SEQ ID NO: 22, 23, 24, 25 or 26.

[2]   Group 2: claims 1-14 (in part), setting forth technical solutions relating to an antibody having heavy chain variable region and light chain variable region sequences as shown respectively in SEQ ID NOs: 8 and 9, or an antibody having a heavy chain variable region sequence as shown in SEQ ID NO: 33, 34, 35 or 36 and a light chain variable region sequence as shown in SEQ ID NO: 30, 31 or 32.

[3]   Group 3: claims 1-14 (in part), setting forth technical solutions relating to an antibody having heavy chain variable region and light chain variable region sequences as shown respectively in SEQ ID NOs: 69 and 70, or an antibody having a heavy chain variable region sequence as shown in SEQ ID NO: 81, 82, 83, 84 or 85 and a light chain variable region sequence as shown in SEQ ID NO: 77, 78, 79 or 80.

[4]   Group 4: claims 1-14 (in part), setting forth technical solutions relating to antibodies having heavy chain variable region and light chain variable region sequences different from the groups above.

[5]   Since the description neither provides embodiments of the antibodies of group 4, nor verifies that said antibodies have the same functions as the antibodies of groups 1-3, the inventions of groups 1-3 and 4 do not share a same or corresponding special technical feature. Groups 1-3 relate to different anti-CTGF antibodies. However, prior art document CN 104011206 A (publication date 27 August 2014) discloses anti-CTGF antibodies, and it is well-known in the prior art that, with the exception of single-domain antibodies, the recognition and binding of an antibody to an antigen generally depends on the interaction of the three CDRs of the heavy chain and the three CDRs of the light chain, the six CDRs together constituting the functional unit of the antibody. The different antibodies of groups 1-3 do not share six completely identical CDR sequences. Therefore, the three groups also do not share a special technical feature which makes a contribution over the prior art, are not so linked as to form a single general inventive step, and do not satisfy the criteria of PCT Rule 13.1 and 13.2.

---

Form PCT/ISA/210 (continuation of first sheet) (January 2015)

**INTERNATIONAL SEARCH REPORT**

International application No.

**PCT/CN2020/094136**

| Box No. III | Observations where unity of invention is lacking (Continuation of item 3 of first sheet) |

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-14 (in part), setting forth technical solutions relating to an antibody having heavy chain variable region and light chain variable region sequences as shown respectively in SEQ ID NOs: 6 and 7, or an antibody having a heavy chain variable region sequence as shown in SEQ ID NO: 27, 28 or 29 and a light chain variable region sequence as shown in SEQ ID NO: 22, 23, 24, 25 or 26.**

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/CN2020/094136**

| Patent document cited in search report | | | Publication date (day/month/year) | Patent family member(s) | | | Publication date (day/month/year) |
|---|---|---|---|---|---|---|---|
| CN | 104011206 | A | 27 August 2014 | PL | 2796550 | T3 | 31 August 2018 |
| | | | | EP | 2796550 | A1 | 29 October 2014 |
| | | | | IN | 4615CHN2014 | A | 18 September 2015 |
| | | | | MX | 2014007681 | A | 25 November 2014 |
| | | | | WO | 2013094723 | A1 | 27 June 2013 |
| | | | | MX | 345019 | B | 11 January 2017 |
| | | | | JP | 6040943 | B2 | 07 December 2016 |
| | | | | CN | 104011206 | B | 08 March 2017 |
| | | | | EP | 2796550 | B1 | 28 February 2018 |
| | | | | EA | 029290 | B1 | 30 March 2018 |
| | | | | AR | 089425 | A1 | 20 August 2014 |
| | | | | JP | WO2013094723 | A1 | 27 April 2015 |
| | | | | ES | 2665851 | T3 | 27 April 2018 |
| | | | | US | 2014343258 | A1 | 20 November 2014 |
| | | | | EP | 2796550 | A4 | 19 August 2015 |
| | | | | PT | 2796550 | T | 18 April 2018 |
| | | | | BR | 112014015405 | A2 | 13 June 2017 |
| | | | | US | 9587015 | B2 | 07 March 2017 |
| | | | | EA | 201491240 | A1 | 28 November 2014 |
| | | | | TW | 201333034 | A | 16 August 2013 |
| | | | | CA | 2859627 | A1 | 27 June 2013 |
| | | | | KR | 20140107507 | A | 04 September 2014 |
| CN | 1829740 | A | 06 September 2006 | CN | 1829740 | B | 29 May 2013 |
| | | | | EP | 1631591 | A2 | 08 March 2006 |
| | | | | RU | 2005141492 | A | 20 July 2007 |
| | | | | US | 7405274 | B2 | 29 July 2008 |
| | | | | US | 2016024198 | A1 | 28 January 2016 |
| | | | | RU | 2330861 | C2 | 10 August 2008 |
| | | | | BR | PI0410963 | A | 04 July 2006 |
| | | | | KR | 20060030032 | A | 07 April 2006 |
| | | | | NO | 336451 | B1 | 24 August 2015 |
| | | | | EP | 2322549 | A1 | 18 May 2011 |
| | | | | JP | 2007525194 | A | 06 September 2007 |
| | | | | IL | 172328 | A | 31 January 2012 |
| | | | | KR | 101244113 | B1 | 18 March 2013 |
| | | | | JP | 5753506 | B2 | 22 July 2015 |
| | | | | ES | 2379662 | T3 | 30 April 2012 |
| | | | | CN | 103539858 | A | 29 January 2014 |
| | | | | US | 7871617 | B2 | 18 January 2011 |
| | | | | US | 2004248206 | A1 | 09 December 2004 |
| | | | | NO | 20060027 | L | 03 January 2006 |
| | | | | US | 2011091468 | A1 | 21 April 2011 |
| | | | | US | 2014073047 | A1 | 13 March 2014 |
| | | | | CA | 2526509 | C | 06 August 2013 |
| | | | | EP | 1631591 | B1 | 21 December 2011 |
| | | | | AU | 2004245514 | B2 | 01 October 2009 |
| | | | | EP | 2338914 | A1 | 29 June 2011 |
| | | | | BR | PI0410963 | B1 | 08 October 2019 |
| | | | | NO | 20060027 | A | 03 January 2006 |
| | | | | US | 2016257740 | A1 | 08 September 2016 |

Form PCT/ISA/210 (patent family annex) (January 2015)

**INTERNATIONAL SEARCH REPORT**
Information on patent family members

International application No.

**PCT/CN2020/094136**

| Patent document cited in search report | | | Publication date (day/month/year) | Patent family member(s) | | | Publication date (day/month/year) |
|---|---|---|---|---|---|---|---|
| | | | | JP | 2012143239 | A | 02 August 2012 |
| | | | | NZ | 543522 | A | 30 June 2008 |
| | | | | WO | 2004108764 | A3 | 03 March 2005 |
| | | | | HK | 1094336 | A1 | 06 December 2013 |
| | | | | WO | 2004108764 | A2 | 16 December 2004 |
| | | | | CA | 2526509 | A1 | 16 December 2004 |
| | | | | US | 2019023778 | A1 | 24 January 2019 |
| | | | | DK | 1631591 | T3 | 10 April 2012 |
| | | | | US | 2009017043 | A1 | 15 January 2009 |
| | | | | JP | 5624707 | B2 | 12 November 2014 |
| | | | | AT | 538136 | T | 15 January 2012 |
| | | | | US | 9034643 | B2 | 19 May 2015 |
| | | | | AU | 2004245514 | A1 | 16 December 2004 |
| CN | 101884789 | A | 17 November 2010 | US | 8314059 | B2 | 20 November 2012 |
| | | | | NZ | 549784 | A | 30 June 2008 |
| | | | | PL | 1715890 | T3 | 30 January 2009 |
| | | | | ES | 2311973 | T3 | 16 February 2009 |
| | | | | AU | 2005212371 | A1 | 25 August 2005 |
| | | | | HK | 1095762 | A1 | 09 January 2009 |
| | | | | CA | 2555789 | A1 | 25 August 2005 |
| | | | | US | 2005214294 | A1 | 29 September 2005 |
| | | | | DE | 602005008013 | D1 | 21 August 2008 |
| | | | | EP | 1715890 | A1 | 02 November 2006 |
| | | | | WO | 2005077413 | A1 | 25 August 2005 |
| | | | | US | 2010291098 | A1 | 18 November 2010 |
| | | | | IL | 177437 | D0 | 10 December 2006 |
| | | | | EP | 1715890 | B1 | 09 July 2008 |
| | | | | PT | 1715890 | E | 17 October 2008 |
| | | | | EP | 1977762 | A1 | 08 October 2008 |
| | | | | DK | 1715890 | T3 | 17 November 2008 |
| | | | | IL | 177437 | A | 27 June 2013 |
| | | | | AU | 2005212371 | B2 | 13 May 2010 |
| | | | | AT | 400297 | T | 15 July 2008 |

Form PCT/ISA/210 (patent family annex) (January 2015)

## REFERENCES CITED IN THE DESCRIPTION

**Patent documents cited in the description**

- CN 201910480169
- WO 0013706 A
- US 6069006 A, Grotendorst and Bradham
- US 5408040 A, Grotendorst and Bradham
- US 4683195 A

**Non-patent literature cited in the description**

- **FRANKLIN.** *Int J Biochem Cell Biol,* 1997, vol. 29, 79-89
- **WUNDERLICH.** Graefes Arch Clin Exp Ophthalmol. 2000, vol. 238, 910-915
- **DENTON ; ABRAHAM.** *Curr Opin Rheumatol,* 2001, vol. 13, 505-511
- **RIEWALD.** *Blood,* 2001, vol. 97, 3109-3116
- **RISER et al.** *J Am SocNephrol,* 2000, vol. 11, 25-38
- **IGARASHI et al.** *Mol BiolCell,* 1993, vol. 4, 637-645
- **GROTENDORST et al.** *Cell Growth Differ,* 1996, vol. 7, 469-480
- **HOLMES et al.** *J Biol Chem,* 2001, vol. 276, 10594-10601
- **RISER et al.** *J Am Soc Nephrol,* 2000, vol. 11, 25-38
- **ITO et al.** *Kidney Int,* 1998, vol. 53, 853-861
- **UJIKE et al.** *Biochem Biophys Res Commun,* 2000, vol. 277, 448-454
- **ABOU -SHADY et al.** *Liver,* 2000, vol. 20, 296-304
- **WILLIAMS et al.** *J Hepatol,* 2000, vol. 32, 754-761
- *J.biol.chem,* 1968, vol. 243, 3558
- **BRADHAM et al.** *J Cell Biol,* 1991, vol. 114, 1285-1294
- **BRIGSTOCK.** *Endocr Rev,* 1999, vol. 20, 189-206
- **O'BRIAN et al.** *Mol Cell Biol,* 1990, vol. 10, 3569-3577
- **JOLIOT et al.** *Mol Cell Biol,* 1992, vol. 12, 10-21
- **RYSECK et al.** *Cell Growth and Diff,* 1990, vol. 2, 225-233
- **SIMMONS et al.** *Proc Natl Acad Sci USA,* 1989, vol. 86, 1178-1182
- **PENNICA et al.** *Proc Natl Acad Sci USA,* 1998, vol. 95, 14717-14722
- **ZHANG et al.** *Mol Cell Biol,* 1998, vol. 18, 6131-6141
- **KABAT, EA et al.** Sequences of Proteins of Immunological Interest. 1991
- **BIRD et al.** *Science,* 1988, vol. 242, 423-426
- **HUSTON et al.** *Proc. Natl. Acad. Sci USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res,* 1996, vol. 56, 3055-3061
- **KIPRIYANOV et al.** *J. Mol. Biol.,* 1999, vol. 293, 41-56
- **ROOVERS et al.** *Cancer Immunol,* 2001
- *Protein Engineering,* 1994, vol. 7, 697
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC MP.** *Immunologist,* 1999, vol. 7, 132-136
- **LEFRANC, MP.** *Dev. Comp. Immunol.,* 2003, vol. 27, 55-77
- Epitope Mapping Protocols in Methods in Molecular Biology. 1996, vol. 66
- **STAHLI et al.** *Methods in Enzymology,* 1983, vol. 9, 242-253
- **KIRKLAND et al.** *J. Immunol.,* 1986, vol. 137, 3614-3619
- **HARLOW ; LANE.** Antibodies, A Laboratory Manual. Cold Spring Harbor Press, 1988
- **MOREL et al.** *Molec. Immunol.,* 1988, vol. 25, 7-15
- **CHEUNG et al.** *Virology,* 1990, vol. 176, 546-552
- **MOLDENHAUER et al.** *Scand. J. Immunol.,* 1990, vol. 32, 77-82
- A Laboratory Manual for Antibodies. Cold Spring Harbor Laboratory Press

- **WATSON et al.** Molecular Biology of the Gene. The Benjamin/Cummings Pub. Co, 1987, 224
- **ALTSCHUL, SF et al.** *J. Mol. Biol.,* 1990, vol. 215, 403-410
- **GISH, W. et al.** *Nature Genet,* 1993, vol. 3, 266-272
- **MADDEN, TL et al.** *Meth. Enzymol.,* 1996, vol. 266, 131-141
- **ALTSCHUL, SF et al.** *Nucleic Acids Res,* 1997, vol. 25, 3389-3402
- **ZHANG, J. et al.** *Genome Res,* 1997, vol. 7, 649-656
- **MULLIS et al.** Cold Spring Harbor Symp. Ouant. Biol. 1987, vol. 51, 263
- PCR TECHNOLOGY. Stockton Press, 1989

Sequence listing

<110> JIANGSU HENGRUI MEDICINE CO., LTD.;
      SHANGHAI HENGRUI PHARMACEUTICAL CO., LTD.

<120> Anti-connective tissue growth factor antibody and application thereof

<130> B77229EP D42091
<140> 20818627.0

<141> 2020-06-03

<150> 201910480169.4
<151> 2019-06-04

<160> 104

<170> SIPOSequenceListing 1.0

<210> 1
<211> 323
<212> PRT
<213> Homo sapiens


<400> 1
Gln Asn Cys Ser Gly Pro Cys Arg Cys Pro Asp Glu Pro Ala Pro Arg
1               5                   10                  15
Cys Pro Ala Gly Val Ser Leu Val Leu Asp Gly Cys Gly Cys Cys Arg
            20                  25                  30
Val Cys Ala Lys Gln Leu Gly Glu Leu Cys Thr Glu Arg Asp Pro Cys
        35                  40                  45
Asp Pro His Lys Gly Leu Phe Cys Asp Phe Gly Ser Pro Ala Asn Arg
    50                  55                  60
Lys Ile Gly Val Cys Thr Ala Lys Asp Gly Ala Pro Cys Ile Phe Gly
65                  70                  75                  80
Gly Thr Val Tyr Arg Ser Gly Glu Ser Phe Gln Ser Ser Cys Lys Tyr
                85                  90                  95
Gln Cys Thr Cys Leu Asp Gly Ala Val Gly Cys Met Pro Leu Cys Ser
            100                 105                 110
Met Asp Val Arg Leu Pro Ser Pro Asp Cys Pro Phe Pro Arg Arg Val
        115                 120                 125
Lys Leu Pro Gly Lys Cys Cys Glu Glu Trp Val Cys Asp Glu Pro Lys
    130                 135                 140
Asp Gln Thr Val Val Gly Pro Ala Leu Ala Ala Tyr Arg Leu Glu Asp
145                 150                 155                 160
Thr Phe Gly Pro Asp Pro Thr Met Ile Arg Ala Asn Cys Leu Val Gln
                165                 170                 175
Thr Thr Glu Trp Ser Ala Cys Ser Lys Thr Cys Gly Met Gly Ile Ser
            180                 185                 190
Thr Arg Val Thr Asn Asp Asn Ala Ser Cys Arg Leu Glu Lys Gln Ser
        195                 200                 205
Arg Leu Cys Met Val Arg Pro Cys Glu Ala Asp Leu Glu Glu Asn Ile
    210                 215                 220
Lys Lys Gly Lys Lys Cys Ile Arg Thr Pro Lys Ile Ser Lys Pro Ile
225                 230                 235                 240
Lys Phe Glu Leu Ser Gly Cys Thr Ser Met Lys Thr Tyr Arg Ala Lys
                245                 250                 255
Phe Cys Gly Val Cys Thr Asp Gly Arg Cys Cys Thr Pro His Arg Thr
            260                 265                 270
Thr Thr Leu Pro Val Glu Phe Lys Cys Pro Asp Gly Glu Val Met Lys
        275                 280                 285
Lys Asn Met Met Phe Ile Lys Thr Cys Ala Cys His Tyr Asn Cys Pro

```
        290                    295                    300
Gly Asp Asn Asp Ile Phe Glu Ser Leu Tyr Tyr Arg Lys Met Tyr Gly
305                    310                    315                    320
Asp Met Ala


<210>  2
<211>  348
<212>  PRT
<213>  Artificial Sequence

<220>
<223>  CTGF-his


<400>  2
Met Glu Phe Gly Leu Ser Trp Leu Phe Leu Val Ala Ile Leu Lys Gly
1               5                   10                  15
Val Gln Cys Gln Asn Cys Ser Gly Pro Cys Arg Cys Pro Asp Glu Pro
            20                  25                  30
Ala Pro Arg Cys Pro Ala Gly Val Ser Leu Val Leu Asp Gly Cys Gly
            35                  40                  45
Cys Cys Arg Val Cys Ala Lys Gln Leu Gly Glu Leu Cys Thr Glu Arg
        50                  55                  60
Asp Pro Cys Asp Pro His Lys Gly Leu Phe Cys Asp Phe Gly Ser Pro
65                  70                  75                  80
Ala Asn Arg Lys Ile Gly Val Cys Thr Ala Lys Asp Gly Ala Pro Cys
                85                  90                  95
Ile Phe Gly Gly Thr Val Tyr Arg Ser Gly Glu Ser Phe Gln Ser Ser
            100                 105                 110
Cys Lys Tyr Gln Cys Thr Cys Leu Asp Gly Ala Val Gly Cys Met Pro
            115                 120                 125
Leu Cys Ser Met Asp Val Arg Leu Pro Ser Pro Asp Cys Pro Phe Pro
            130                 135                 140
Arg Arg Val Lys Leu Pro Gly Lys Cys Cys Glu Glu Trp Val Cys Asp
145                 150                 155                 160
Glu Pro Lys Asp Gln Thr Val Val Gly Pro Ala Leu Ala Ala Tyr Arg
                165                 170                 175
Leu Glu Asp Thr Phe Gly Pro Asp Pro Thr Met Ile Arg Ala Asn Cys
            180                 185                 190
Leu Val Gln Thr Thr Glu Trp Ser Ala Cys Ser Lys Thr Cys Gly Met
            195                 200                 205
Gly Ile Ser Thr Arg Val Thr Asn Asp Asn Ala Ser Cys Arg Leu Glu
            210                 215                 220
Lys Gln Ser Arg Leu Cys Met Val Arg Pro Cys Glu Ala Asp Leu Glu
225                 230                 235                 240
Glu Asn Ile Lys Lys Gly Lys Lys Cys Ile Arg Thr Pro Lys Ile Ser
                245                 250                 255
Lys Pro Ile Lys Phe Glu Leu Ser Gly Cys Thr Ser Met Lys Thr Tyr
            260                 265                 270
Arg Ala Lys Phe Cys Gly Val Cys Thr Asp Gly Arg Cys Cys Thr Pro
            275                 280                 285
His Arg Thr Thr Thr Leu Pro Val Glu Phe Lys Cys Pro Asp Gly Glu
    290                 295                 300
Val Met Lys Lys Asn Met Met Phe Ile Lys Thr Cys Ala Cys His Tyr
305                 310                 315                 320
Asn Cys Pro Gly Asp Asn Asp Ile Phe Glu Ser Leu Tyr Tyr Arg Lys
                325                 330                 335
Met Tyr Gly Asp Met Ala His His His His His
            340                 345

<210>  3
<211>  575
```

<212> PRT
<213> Artificial Sequence

<220>
<223> mCTGF-Fc

<400> 3
Met Glu Phe Gly Leu Ser Trp Leu Phe Leu Val Ala Ile Leu Lys Gly
1               5                   10                  15
Val Gln Cys Gln Asp Cys Ser Ala Gln Cys Gln Cys Ala Ala Glu Ala
            20                  25                  30
Ala Pro His Cys Pro Ala Gly Val Ser Leu Val Leu Asp Gly Cys Gly
        35                  40                  45
Cys Cys Arg Val Cys Ala Lys Gln Leu Gly Glu Leu Cys Thr Glu Arg
    50                  55                  60
Asp Pro Cys Asp Pro His Lys Gly Leu Phe Cys Asp Phe Gly Ser Pro
65                  70                  75                  80
Ala Asn Arg Lys Ile Gly Val Cys Thr Ala Lys Asp Gly Ala Pro Cys
            85                  90                  95
Val Phe Gly Gly Ser Val Tyr Arg Ser Gly Glu Ser Phe Gln Ser Ser
            100                 105                 110
Cys Lys Tyr Gln Cys Thr Cys Leu Asp Gly Ala Val Gly Cys Val Pro
        115                 120                 125
Leu Cys Ser Met Asp Val Arg Leu Pro Ser Pro Asp Cys Pro Phe Pro
    130                 135                 140
Arg Arg Val Lys Leu Pro Gly Lys Cys Cys Glu Glu Trp Val Cys Asp
145                 150                 155                 160
Glu Pro Lys Asp Arg Thr Ala Val Gly Pro Ala Leu Ala Ala Tyr Arg
            165                 170                 175
Leu Glu Asp Thr Phe Gly Pro Asp Pro Thr Met Met Arg Ala Asn Cys
        180                 185                 190
Leu Val Gln Thr Thr Glu Trp Ser Ala Cys Ser Lys Thr Cys Gly Met
    195                 200                 205
Gly Ile Ser Thr Arg Val Thr Asn Asp Asn Thr Phe Cys Arg Leu Glu
    210                 215                 220
Lys Gln Ser Arg Leu Cys Met Val Arg Pro Cys Glu Ala Asp Leu Glu
225                 230                 235                 240
Glu Asn Ile Lys Lys Gly Lys Lys Cys Ile Arg Thr Pro Lys Ile Ala
            245                 250                 255
Lys Pro Val Lys Phe Glu Leu Ser Gly Cys Thr Ser Val Lys Thr Tyr
        260                 265                 270
Arg Ala Lys Phe Cys Gly Val Cys Thr Asp Gly Arg Cys Cys Thr Pro
    275                 280                 285
His Arg Thr Thr Thr Leu Pro Val Glu Phe Lys Cys Pro Asp Gly Glu
    290                 295                 300
Ile Met Lys Lys Asn Met Met Phe Ile Lys Thr Cys Ala Cys His Tyr
305                 310                 315                 320
Asn Cys Pro Gly Asp Asn Asp Ile Phe Glu Ser Leu Tyr Tyr Arg Lys
            325                 330                 335
Met Tyr Gly Asp Met Ala Glu Pro Arg Gly Pro Thr Ile Lys Pro Cys
        340                 345                 350
Pro Pro Cys Lys Cys Pro Ala Pro Asn Leu Leu Gly Gly Pro Ser Val
        355                 360                 365
Phe Ile Phe Pro Pro Lys Ile Lys Asp Val Leu Met Ile Ser Leu Ser
        370                 375                 380
Pro Ile Val Thr Cys Val Val Val Asp Val Ser Glu Asp Asp Pro Asp
385                 390                 395                 400
Val Gln Ile Ser Trp Phe Val Asn Asn Val Glu Val His Thr Ala Gln
            405                 410                 415
Thr Gln Thr His Arg Glu Asp Tyr Asn Ser Thr Leu Arg Val Val Ser
        420                 425                 430
Ala Leu Pro Ile Gln His Gln Asp Trp Met Ser Gly Lys Glu Phe Lys

50

```
            435                   440                   445
      Cys Lys Val Asn Asn Lys Asp Leu Pro Ala Pro Ile Glu Arg Thr Ile
          450                   455                   460
      Ser Lys Pro Lys Gly Ser Val Arg Ala Pro Gln Val Tyr Val Leu Pro
      465                   470                   475                   480
      Pro Pro Glu Glu Glu Met Thr Lys Lys Gln Val Thr Leu Thr Cys Met
                          485                   490                   495
      Val Thr Asp Phe Met Pro Glu Asp Ile Tyr Val Glu Trp Thr Asn Asn
                      500                   505                   510
      Gly Lys Thr Glu Leu Asn Tyr Lys Asn Thr Glu Pro Val Leu Asp Ser
                      515                   520                   525
      Asp Gly Ser Tyr Phe Met Tyr Ser Lys Leu Arg Val Glu Lys Lys Asn
                      530                   535                   540
      Trp Val Glu Arg Asn Ser Tyr Ser Cys Ser Val Val His Glu Gly Leu
      545                   550                   555                   560
      His Asn His His Thr Thr Lys Ser Phe Ser Arg Thr Pro Gly Lys
                          565                   570                   575


      <210>   4
      <211>   348
      <212>   PRT
      <213>   Artificial Sequence

      <220>
      <223>   mouse CTGF protein-His tag


      <400>   4
      Met Glu Phe Gly Leu Ser Trp Leu Phe Leu Val Ala Ile Leu Lys Gly
      1               5                   10                  15
      Val Gln Cys Gln Asp Cys Ser Ala Gln Cys Gln Cys Ala Ala Glu Ala
                      20                  25                  30
      Ala Pro His Cys Pro Ala Gly Val Ser Leu Val Leu Asp Gly Cys Gly
                  35                  40                  45
      Cys Cys Arg Val Cys Ala Lys Gln Leu Gly Glu Leu Cys Thr Glu Arg
          50                  55                  60
      Asp Pro Cys Asp Pro His Lys Gly Leu Phe Cys Asp Phe Gly Ser Pro
      65                  70                  75                  80
      Ala Asn Arg Lys Ile Gly Val Cys Thr Ala Lys Asp Gly Ala Pro Cys
                      85                  90                  95
      Val Phe Gly Gly Ser Val Tyr Arg Ser Gly Glu Ser Phe Gln Ser Ser
                  100                 105                 110
      Cys Lys Tyr Gln Cys Thr Cys Leu Asp Gly Ala Val Gly Cys Val Pro
          115                 120                 125
      Leu Cys Ser Met Asp Val Arg Leu Pro Ser Pro Asp Cys Pro Phe Pro
          130                 135                 140
      Arg Arg Val Lys Leu Pro Gly Lys Cys Cys Glu Glu Trp Val Cys Asp
      145                 150                 155                 160
      Glu Pro Lys Asp Arg Thr Ala Val Gly Pro Ala Leu Ala Ala Tyr Arg
                      165                 170                 175
      Leu Glu Asp Thr Phe Gly Pro Asp Pro Thr Met Met Arg Ala Asn Cys
                  180                 185                 190
      Leu Val Gln Thr Thr Glu Trp Ser Ala Cys Ser Lys Thr Cys Gly Met
              195                 200                 205
      Gly Ile Ser Thr Arg Val Thr Asn Asp Asn Thr Phe Cys Arg Leu Glu
          210                 215                 220
      Lys Gln Ser Arg Leu Cys Met Val Arg Pro Cys Glu Ala Asp Leu Glu
      225                 230                 235                 240
      Glu Asn Ile Lys Lys Gly Lys Lys Cys Ile Arg Thr Pro Lys Ile Ala
                      245                 250                 255
      Lys Pro Val Lys Phe Glu Leu Ser Gly Cys Thr Ser Val Lys Thr Tyr
                  260                 265                 270
      Arg Ala Lys Phe Cys Gly Val Cys Thr Asp Gly Arg Cys Cys Thr Pro
```

```
                 275                      280                         285
         His Arg Thr Thr Thr Leu Pro Val Glu Phe Lys Cys Pro Asp Gly Glu
             290                 295                 300
         Ile Met Lys Lys Asn Met Met Phe Ile Lys Thr Cys Ala Cys His Tyr
         305                 310                 315                 320
         Asn Cys Pro Gly Asp Asn Asp Ile Phe Glu Ser Leu Tyr Tyr Arg Lys
                         325                 330                 335
         Met Tyr Gly Asp Met Ala His His His His His His
                         340                 345


         <210>  5
         <211>  574
         <212>  PRT
         <213>  Artificial Sequence

         <220>
         <223>  cynomolgus CTGF-Fc


         <400>  5
         Met Glu Phe Gly Leu Ser Trp Leu Phe Leu Val Ala Ile Leu Lys Gly
         1               5                   10                  15
         Val Gln Cys Gln Asn Cys Ser Gly Pro Cys Arg Cys Pro Ala Glu Gln
                     20                  25                  30
         Ala Pro Arg Cys Pro Ala Gly Val Ser Leu Val Leu Asp Gly Cys Gly
                     35                  40                  45
         Cys Cys Arg Val Cys Ala Lys Gln Leu Gly Glu Leu Cys Thr Glu Arg
                 50                  55                  60
         Asp Pro Cys Asp Pro His Lys Gly Leu Phe Cys Asp Phe Gly Ser Pro
         65                  70                  75                  80
         Ala Asn Arg Lys Ile Gly Val Cys Thr Ala Lys Asp Gly Ala Pro Cys
                         85                  90                  95
         Ile Phe Gly Gly Thr Val Tyr Arg Ser Gly Glu Ser Phe Gln Ser Ser
                     100                 105                 110
         Cys Lys Tyr Gln Cys Thr Cys Leu Asp Gly Ala Val Gly Cys Met Pro
                     115                 120                 125
         Leu Cys Ser Met Asp Val Arg Leu Pro Ser Pro Asp Cys Pro Phe Pro
                 130                 135                 140
         Arg Arg Val Lys Leu Pro Gly Lys Cys Cys Glu Glu Trp Val Cys Asp
         145                 150                 155                 160
         Glu Pro Lys Asp Gln Thr Val Val Gly Pro Ala Leu Ala Ala Tyr Arg
                         165                 170                 175
         Leu Glu Asp Thr Phe Gly Pro Asp Pro Thr Met Ile Arg Ala Asn Cys
                     180                 185                 190
         Leu Val Gln Thr Thr Glu Trp Ser Ala Cys Ser Lys Thr Cys Gly Met
                     195                 200                 205
         Gly Ile Ser Thr Arg Val Thr Asn Asp Asn Ala Ser Cys Arg Leu Glu
                 210                 215                 220
         Lys Gln Ser Arg Leu Cys Met Val Arg Pro Cys Glu Ala Asp Leu Glu
         225                 230                 235                 240
         Glu Asn Ile Lys Lys Gly Lys Lys Cys Ile Arg Thr Pro Lys Ile Ser
                         245                 250                 255
         Lys Pro Ile Lys Phe Glu Leu Ser Gly Cys Thr Ser Val Lys Thr Tyr
                     260                 265                 270
         Arg Ala Lys Phe Cys Gly Val Cys Thr Asp Gly Arg Cys Cys Thr Pro
                     275                 280                 285
         His Arg Thr Thr Thr Leu Pro Val Glu Phe Lys Cys Pro Asp Gly Glu
                     290                 295                 300
         Val Met Lys Lys Asn Met Met Phe Ile Lys Thr Cys Ala Cys His Tyr
         305                 310                 315                 320
         Asn Cys Pro Gly Asp Asn Asp Ile Phe Glu Ser Leu Tyr Tyr Arg Lys
                         325                 330                 335
         Met Tyr Gly Asp Met Ala Glu Pro Lys Ser Ser Asp Lys Thr His Thr
```

52

```
                  340                         345                         350
Cys Pro Pro Cys Pro Ala Pro Glu Leu Leu Gly Gly Pro Ser Val Phe
         355                         360                         365
Leu Phe Pro Pro Lys Pro Lys Asp Thr Leu Met Ile Ser Arg Thr Pro
         370                         375                         380
Glu Val Thr Cys Val Val Asp Val Ser His Glu Asp Pro Glu Val
385                         390                         395                         400
Lys Phe Asn Trp Tyr Val Asp Gly Val Glu Val His Asn Ala Lys Thr
                  405                         410                         415
Lys Pro Arg Glu Glu Gln Tyr Asn Ser Thr Tyr Arg Val Val Ser Val
         420                         425                         430
Leu Thr Val Leu His Gln Asp Trp Leu Asn Gly Lys Glu Tyr Lys Cys
         435                         440                         445
Lys Val Ser Asn Lys Ala Leu Pro Ala Pro Ile Glu Lys Thr Ile Ser
450                         455                         460
Lys Ala Lys Gly Gln Pro Arg Glu Pro Gln Val Tyr Thr Leu Pro Pro
465                         470                         475                         480
Ser Arg Asp Glu Leu Thr Lys Asn Gln Val Ser Leu Thr Cys Leu Val
                  485                         490                         495
Lys Gly Phe Tyr Pro Ser Asp Ile Ala Val Glu Trp Glu Ser Asn Gly
         500                         505                         510
Gln Pro Glu Asn Asn Tyr Lys Thr Thr Pro Pro Val Leu Asp Ser Asp
         515                         520                         525
Gly Ser Phe Phe Leu Tyr Ser Lys Leu Thr Val Asp Lys Ser Arg Trp
         530                         535                         540
Gln Gln Gly Asn Val Phe Ser Cys Ser Val Met His Glu Ala Leu His
545                         550                         555                         560
Asn His Tyr Thr Gln Lys Ser Leu Ser Leu Ser Pro Gly Lys
                  565                         570
```

<210> 6
<211> 116
<212> PRT
<213> Mus musculus

<400> 6
```
Glu Val Gln Leu Val Glu Ser Gly Gly Gly Leu Val Gln Pro Glu Gly
1                   5                           10                          15
Ser Leu Lys Leu Ser Cys Ala Ala Ser Gly Phe Ser Phe Asn Thr Tyr
         20                          25                          30
Ala Met Asn Trp Val Arg Gln Ala Pro Gly Lys Gly Leu Glu Trp Val
         35                          40                          45
Ala Arg Ile Arg Thr Lys Ser Asn Asn Tyr Ala Thr Tyr Tyr Ala Asp
         50                          55                          60
Ser Val Lys Asp Arg Phe Thr Ile Ser Arg Asp Asp Ser Glu Ser Met
65                          70                          75                          80
Leu Tyr Leu Gln Met Asn Asn Leu Lys Thr Glu Asp Thr Ala Met Tyr
                  85                          90                          95
Tyr Cys Val Glu Thr Gly Phe Ala Tyr Trp Asp Gln Gly Thr Leu Val
                  100                         105                         110
Thr Val Ser Ala
         115
```

<210> 7
<211> 106
<212> PRT
<213> Mus musculus

<400> 7
```
Gln Ile Val Leu Thr Gln Ser Pro Ala Ile Met Ser Ala Ser Pro Gly
1                   5                           10                          15
```

53

Glu Lys Val Thr Ile Thr Cys Ser Ala Ser Ser Ser Val Ser Tyr Met
                20                      25                  30
His Trp Phe Gln Gln Lys Pro Gly Thr Ser Pro Lys Leu Trp Ile Tyr
         35                      40                  45
Ser Thr Ser Asn Leu Ala Ser Gly Val Pro Ala Arg Phe Ser Gly Ser
     50                      55                  60
Gly Ser Gly Thr Ser Tyr Ser Leu Thr Ile Ser Arg Met Glu Ala Glu
65                   70                  75                      80
Asp Ala Ala Thr Tyr Tyr Cys Gln Gln Arg Ser Ser Tyr Pro Leu Thr
                85                      90                  95
Phe Gly Ala Gly Thr Lys Leu Glu Leu Lys
             100                 105

<210> 8
<211> 114
<212> PRT
<213> Mus musculus

<400> 8
Gln Val Gln Leu Lys Gln Ser Gly Pro Gly Leu Val Gln Pro Ser Gln
1                   5                   10                  15
Ser Leu Ser Ile Thr Cys Thr Val Ser Gly Phe Ser Leu Thr Thr Phe
             20                      25                  30
Gly Val His Trp Ile Arg Gln Ser Pro Gly Lys Gly Leu Glu Trp Leu
         35                      40                  45
Gly Val Ile Trp Arg Arg Gly Gly Thr Asp Tyr Asn Ala Ala Phe Met
         50                      55                  60
Ser Arg Leu Ser Ile Thr Lys Asp Asn Ser Arg Ser His Val Phe Phe
65                   70                  75                      80
Lys Met Thr Ser Leu Gln Thr Asp Asp Ser Ala Ile Tyr Tyr Cys Ala
                85                      90                  95
Arg Asp Gly Gly Phe Asp Tyr Trp Gly Gln Gly Thr Thr Leu Thr Val
             100                 105                 110
Ser Ser

<210> 9
<211> 109
<212> PRT
<213> Mus musculus

<400> 9
Gln Ala Val Val Thr Gln Glu Ser Ala Leu Thr Thr Ser Pro Gly Gly
1                   5                   10                  15
Thr Val Ile Leu Thr Cys Arg Ser Ser Ile Gly Ala Val Thr Thr Ser
             20                      25                  30
Asn Tyr Ala Asn Trp Val Gln Glu Lys Pro Asp His Leu Phe Thr Gly
         35                      40                  45
Leu Ile Gly Gly Thr Ser Asn Arg Ala Pro Gly Val Pro Val Arg Phe
     50                      55                  60
Ser Gly Ser Leu Ile Gly Asp Lys Ala Ala Leu Thr Ile Thr Gly Ala
65                   70                  75                      80
Gln Ala Glu Asp Asp Ala Met Tyr Phe Cys Ala Leu Trp Tyr Ser Thr
                85                      90                  95
His Tyr Val Phe Gly Gly Gly Thr Lys Val Thr Val Leu
             100                 105

<210> 10
<211> 5
<212> PRT
<213> Mus musculus

<400> 10
Thr Tyr Ala Met Asn
1               5


<210> 11
<211> 19
<212> PRT
<213> Mus musculus


<400> 11
Arg Ile Arg Thr Lys Ser Asn Asn Tyr Ala Thr Tyr Tyr Ala Asp Ser
1               5                   10                  15
Val Lys Asp


<210> 12
<211> 5
<212> PRT
<213> Mus musculus


<400> 12
Thr Gly Phe Ala Tyr
1               5

<210> 13
<211> 10
<212> PRT
<213> Mus musculus


<400> 13
Ser Ala Ser Ser Ser Val Ser Tyr Met His
1               5                   10

<210> 14
<211> 7
<212> PRT
<213> Mus musculus


<400> 14
Ser Thr Ser Asn Leu Ala Ser
1               5

<210> 15
<211> 9
<212> PRT
<213> Mus musculus


<400> 15
Gln Gln Arg Ser Ser Tyr Pro Leu Thr
1               5

<210> 16
<211> 5
<212> PRT
<213> Mus musculus

```
<400>  16
Thr Phe Gly Val His
1               5


<210>  17
<211>  16
<212>  PRT
<213>  Mus musculus


<400>  17
Val Ile Trp Arg Arg Gly Gly Thr Asp Tyr Asn Ala Ala Phe Met Ser
1               5                   10                  15


<210>  18
<211>  6
<212>  PRT
<213>  Mus musculus


<400>  18
Asp Gly Gly Phe Asp Tyr
1               5


<210>  19
<211>  14
<212>  PRT
<213>  Mus musculus


<400>  19
Arg Ser Ser Ile Gly Ala Val Thr Thr Ser Asn Tyr Ala Asn
1               5                   10


<210>  20
<211>  7
<212>  PRT
<213>  Mus musculus


<400>  20
Gly Thr Ser Asn Arg Ala Pro
1               5


<210>  21
<211>  9
<212>  PRT
<213>  Mus musculus


<400>  21
Ala Leu Trp Tyr Ser Thr His Tyr Val
1               5


<210>  22
<211>  106
<212>  PRT
<213>  Artificial Sequence


<220>
<223>  Hu147-VL1
```

<400> 22
Glu Ile Val Leu Thr Gln Ser Pro Ala Thr Leu Ser Leu Ser Pro Gly
1               5               10              15
Glu Arg Ala Thr Leu Ser Cys Ser Ala Ser Ser Ser Val Ser Tyr Met
            20              25              30
His Trp Phe Gln Gln Lys Pro Gly Gln Ser Pro Arg Leu Leu Ile Tyr
        35              40              45
Ser Thr Ser Asn Leu Ala Ser Gly Ile Pro Ala Arg Phe Ser Gly Ser
    50              55              60
Gly Ser Gly Thr Asp Tyr Thr Leu Thr Ile Ser Ser Leu Glu Pro Glu
65              70              75              80
Asp Phe Ala Val Tyr Tyr Cys Gln Gln Arg Ser Ser Tyr Pro Leu Thr
            85              90              95
Phe Gly Gln Gly Thr Lys Leu Glu Ile Lys
            100             105


<210>   23
<211>   106
<212>   PRT
<213>   Artificial Sequence

<220>
<223>   Hu147-VL2


<400>   23
Glu Ile Val Leu Thr Gln Ser Pro Ala Thr Leu Ser Leu Ser Pro Gly
1               5               10              15
Glu Arg Ala Thr Leu Ser Cys Ser Ala Ser Ser Ser Val Ser Tyr Met
            20              25              30
His Trp Phe Gln Gln Lys Pro Gly Gln Ser Pro Lys Leu Leu Ile Tyr
        35              40              45
Ser Thr Ser Asn Leu Ala Ser Gly Ile Pro Ala Arg Phe Ser Gly Ser
    50              55              60
Gly Ser Gly Thr Asp Tyr Thr Leu Thr Ile Ser Ser Leu Glu Pro Glu
65              70              75              80
Asp Phe Ala Val Tyr Tyr Cys Gln Gln Arg Ser Ser Tyr Pro Leu Thr
            85              90              95
Phe Gly Gln Gly Thr Lys Leu Glu Ile Lys
            100             105


<210>   24
<211>   106
<212>   PRT
<213>   Artificial Sequence

<220>
<223>   Hu147-VL3


<400>   24
Glu Ile Val Leu Thr Gln Ser Pro Ala Thr Leu Ser Leu Ser Pro Gly
1               5               10              15
Glu Arg Ala Thr Leu Ser Cys Ser Ala Ser Ser Ser Val Ser Tyr Met
            20              25              30
His Trp Phe Gln Gln Lys Pro Gly Gln Ser Pro Lys Leu Trp Ile Tyr
        35              40              45
Ser Thr Ser Asn Leu Ala Ser Gly Val Pro Ala Arg Phe Ser Gly Ser
    50              55              60
Gly Ser Gly Thr Asp Tyr Thr Leu Thr Ile Ser Ser Leu Glu Pro Glu
65              70              75              80
Asp Phe Ala Val Tyr Tyr Cys Gln Gln Arg Ser Ser Tyr Pro Leu Thr
            85              90              95

```
Phe Gly Gln Gly Thr Lys Leu Glu Ile Lys
            100                 105


<210>   25
<211>   106
<212>   PRT
<213>   Artificial Sequence


<220>
<223>   Hu147-VL4


<400>   25
Asp Ile Gln Met Thr Gln Ser Pro Ser Ser Leu Ser Ala Ser Val Gly
1                 5                 10                  15
Asp Arg Val Thr Ile Thr Cys Ser Ala Ser Ser Ser Val Ser Tyr Met
            20                  25                  30
His Trp Phe Gln Gln Lys Pro Gly Lys Ser Pro Lys Leu Leu Ile Tyr
        35                  40                  45
Ser Thr Ser Asn Leu Ala Ser Gly Val Pro Ser Arg Phe Ser Gly Ser
    50                  55                  60
Gly Ser Gly Thr Asp Tyr Thr Leu Thr Ile Ser Ser Leu Gln Pro Glu
65                  70                  75                  80
Asp Phe Ala Thr Tyr Tyr Cys Gln Gln Arg Ser Ser Tyr Pro Leu Thr
                85                  90                  95
Phe Gly Gln Gly Thr Lys Leu Glu Ile Lys
            100                 105


<210>   26
<211>   106
<212>   PRT
<213>   Artificial Sequence


<220>
<223>   Hu147-VL5


<400>   26
Asp Ile Gln Leu Thr Gln Ser Pro Ser Ser Leu Ser Ala Ser Val Gly
1                 5                 10                  15
Asp Arg Val Thr Ile Thr Cys Ser Ala Ser Ser Ser Val Ser Tyr Met
            20                  25                  30
His Trp Phe Gln Gln Lys Pro Gly Lys Ser Pro Lys Leu Trp Ile Tyr
        35                  40                  45
Ser Thr Ser Asn Leu Ala Ser Gly Val Pro Ser Arg Phe Ser Gly Ser
    50                  55                  60
Gly Ser Gly Thr Asp Tyr Thr Leu Thr Ile Ser Ser Leu Gln Pro Glu
65                  70                  75                  80
Asp Phe Ala Thr Tyr Tyr Cys Gln Gln Arg Ser Ser Tyr Pro Leu Thr
                85                  90                  95
Phe Gly Gln Gly Thr Lys Leu Glu Ile Lys
            100                 105


<210>   27
<211>   116
<212>   PRT
<213>   Artificial Sequence


<220>
<223>   Hu147-VH1


<400>   27
```

```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Thr Tyr
            20                  25                  30
Ala Met Asn Trp Val Arg Gln Ala Pro Gly Lys Gly Leu Glu Trp Val
        35                  40                  45
Gly Arg Ile Arg Thr Lys Ser Asn Asn Tyr Ala Thr Tyr Tyr Ala Asp
    50                  55                  60
Ser Val Lys Asp Arg Phe Thr Ile Ser Arg Asp Asp Ser Lys Asn Ser
65                  70                  75                  80
Leu Tyr Leu Gln Met Asn Ser Leu Lys Thr Glu Asp Thr Ala Val Tyr
                85                  90                  95
Tyr Cys Val Glu Thr Gly Phe Ala Tyr Trp Asp Gln Gly Thr Leu Val
            100                 105                 110
Thr Val Ser Ser
            115
```

```
<210>   28
<211>   116
<212>   PRT
<213>   Artificial Sequence

<220>
<223>   Hu147-VH2
```

```
<400>   28
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Asn Thr Tyr
            20                  25                  30
Ala Met Asn Trp Val Arg Gln Ala Pro Gly Lys Gly Leu Glu Trp Val
        35                  40                  45
Ala Arg Ile Arg Thr Lys Ser Asn Asn Tyr Ala Thr Tyr Tyr Ala Asp
    50                  55                  60
Ser Val Lys Asp Arg Phe Thr Ile Ser Arg Asp Asp Ser Lys Asn Ser
65                  70                  75                  80
Leu Tyr Leu Gln Met Asn Ser Leu Lys Thr Glu Asp Thr Ala Val Tyr
                85                  90                  95
Tyr Cys Val Glu Thr Gly Phe Ala Tyr Trp Asp Gln Gly Thr Leu Val
            100                 105                 110
Thr Val Ser Ser
            115
```

```
<210>   29
<211>   116
<212>   PRT
<213>   Artificial Sequence

<220>
<223>   Hu147-VH3
```

```
<400>   29
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Ser Phe Asn Thr Tyr
            20                  25                  30
Ala Met Asn Trp Val Arg Gln Ala Pro Gly Lys Gly Leu Glu Trp Val
        35                  40                  45
Ala Arg Ile Arg Thr Lys Ser Asn Asn Tyr Ala Thr Tyr Tyr Ala Asp
    50                  55                  60
Ser Val Lys Asp Arg Phe Thr Ile Ser Arg Asp Asp Ser Glu Ser Ser
```

```
        65                    70                    75                    80
        Leu Tyr Leu Gln Met Asn Ser Leu Lys Thr Glu Asp Thr Ala Val Tyr
                        85                    90                    95
        Tyr Cys Val Glu Thr Gly Phe Ala Tyr Trp Asp Gln Gly Thr Leu Val
                        100                   105                   110
        Thr Val Ser Ser
                        115
```

```
<210>   30
<211>   109
<212>   PRT
<213>   Artificial Sequence

<220>
<223>   Hu164-VL7
```

```
<400>   30
Glu Thr Val Val Thr Gln Glu Pro Ser Leu Thr Val Ser Pro Gly Gly
1                   5                   10                    15
Thr Val Thr Leu Thr Cys Arg Ser Ser Ile Gly Ala Val Thr Thr Ser
                20                    25                    30
Asn Tyr Ala Asn Trp Val Gln Gln Lys Pro Gly Gln Ala Phe Arg Gly
            35                    40                    45
Leu Ile Gly Gly Thr Ser Asn Arg Ala Pro Trp Thr Pro Ala Arg Phe
        50                    55                    60
Ser Gly Ser Leu Leu Gly Gly Lys Ala Ala Leu Thr Leu Ser Gly Val
65                    70                    75                    80
Gln Pro Glu Asp Glu Ala Glu Tyr Tyr Cys Ala Leu Trp Tyr Ser Thr
                85                    90                    95
His Tyr Val Phe Gly Gly Gly Thr Lys Leu Thr Val Leu
                100                   105
```

```
<210>   31
<211>   109
<212>   PRT
<213>   Artificial Sequence

<220>
<223>   Hu164-VL8
```

```
<400>   31
Glu Thr Val Val Thr Gln Glu Pro Ser Phe Ser Val Ser Pro Gly Gly
1                   5                   10                    15
Thr Val Thr Leu Thr Cys Arg Ser Ser Ile Gly Ala Val Thr Thr Ser
                20                    25                    30
Asn Tyr Ala Asn Trp Val Gln Gln Thr Pro Gly Gln Ala Phe Arg Gly
            35                    40                    45
Leu Ile Gly Gly Thr Ser Asn Arg Ala Pro Gly Val Pro Asp Arg Phe
        50                    55                    60
Ser Gly Ser Ile Leu Gly Asn Lys Ala Ala Leu Thr Ile Thr Gly Ala
65                    70                    75                    80
Gln Ala Asp Asp Glu Ser Asp Tyr Tyr Cys Ala Leu Trp Tyr Ser Thr
                85                    90                    95
His Tyr Val Phe Gly Gly Gly Thr Lys Leu Thr Val Leu
                100                   105
```

```
<210>   32
<211>   109
<212>   PRT
<213>   Artificial Sequence
```

<220>
<223> Hu164-VL9

<400> 32
Glu Ser Val Val Thr Gln Pro Pro Ser Val Ser Gly Ala Pro Gly Gln
1               5                   10                  15
Arg Val Thr Ile Ser Cys Arg Ser Ser Ile Gly Ala Val Thr Thr Ser
            20                  25                  30
Asn Tyr Ala Asn Trp Val Gln Gln Leu Pro Gly Thr Ala Phe Lys Gly
        35                  40                  45
Leu Ile Gly Gly Thr Ser Asn Arg Ala Pro Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Ala Leu Trp Tyr Ser Thr
                85                  90                  95
His Tyr Val Phe Gly Gly Gly Thr Lys Leu Thr Val Leu
            100                 105

<210> 33
<211> 114
<212> PRT
<213> Artificial Sequence

<220>
<223> Hu164-VH5

<400> 33
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Thr Phe
            20                  25                  30
Gly Val His Trp Ile Arg Gln Pro Pro Gly Lys Ala Leu Glu Trp Leu
        35                  40                  45
Ala Val Ile Trp Arg Arg Gly Gly Thr Asp Tyr Asn Ala Ala Phe Met
    50                  55                  60
Ser Arg Leu Thr Ile Ser Lys Asp Thr Ser Lys Ser Gln Val Val Leu
65                  70                  75                  80
Thr Met Thr Asn Met Asp Pro Val Asp Thr Ala Thr Tyr Tyr Cys Ala
                85                  90                  95
Arg Asp Gly Gly Phe Asp Tyr Trp Gly Gln Gly Thr Thr Val Thr Val
            100                 105                 110
Ser Ser

<210> 34
<211> 114
<212> PRT
<213> Artificial Sequence

<220>
<223> Hu164-VH6

<400> 34
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Thr Phe
            20                  25                  30
Gly Val His Trp Ile Arg Gln Pro Pro Gly Lys Gly Leu Glu Trp Leu
        35                  40                  45

```
Gly Val Ile Trp Arg Arg Gly Gly Thr Asp Tyr Asn Ala Ala Phe Met
    50                  55                  60
Ser Arg Leu Thr Ile Ser Lys Asp Thr Ser Lys Ser Gln Val Val Phe
65                  70                  75                  80
Thr Met Thr Asn Met Asp Pro Val Asp Thr Ala Thr Tyr Tyr Cys Ala
                85                  90                  95
Arg Asp Gly Gly Phe Asp Tyr Trp Gly Gln Gly Thr Thr Val Thr Val
            100                 105                 110
Ser Ser


<210>  35
<211>  114
<212>  PRT
<213>  Artificial Sequence

<220>
<223>  Hu164-VH7


<400>  35
Glu Val Gln Leu Gln Glu Ser Gly Pro Gly Leu Val Lys Pro Ser Gln
1                   5                   10                  15
Thr Leu Ser Leu Thr Cys Thr Val Ser Gly Phe Ser Ile Ser Thr Phe
                20                  25                  30
Gly Val His Trp Ile Arg Gln His Pro Gly Lys Gly Leu Glu Trp Ile
            35                  40                  45
Gly Val Ile Trp Arg Arg Gly Gly Thr Asp Tyr Asn Ala Ala Phe Met
    50                  55                  60
Ser Arg Val Thr Ile Ser Lys Asp Thr Ser Lys Asn Gln Val Ser Leu
65                  70                  75                  80
Lys Leu Ser Ser Val Thr Ala Ala Asp Thr Ala Val Tyr Tyr Cys Ala
                85                  90                  95
Arg Asp Gly Gly Phe Asp Tyr Trp Gly Gln Gly Thr Thr Val Thr Val
            100                 105                 110
Ser Ser


<210>  36
<211>  114
<212>  PRT
<213>  Artificial Sequence

<220>
<223>  Hu164-VH8


<400>  36
Glu Val Gln Leu Gln Glu Ser Gly Pro Gly Leu Val Lys Pro Ser Gln
1                   5                   10                  15
Thr Leu Ser Leu Thr Cys Thr Val Ser Gly Phe Ser Ile Ser Thr Phe
                20                  25                  30
Gly Val His Trp Ile Arg Gln His Pro Gly Lys Gly Leu Glu Trp Leu
            35                  40                  45
Gly Val Ile Trp Arg Arg Gly Gly Thr Asp Tyr Asn Ala Ala Phe Met
    50                  55                  60
Ser Arg Leu Thr Ile Ser Lys Asp Thr Ser Lys Asn Gln Val Ser Phe
65                  70                  75                  80
Lys Leu Ser Ser Val Thr Ala Ala Asp Thr Ala Val Tyr Tyr Cys Ala
                85                  90                  95
Arg Asp Gly Gly Phe Asp Tyr Trp Gly Gln Gly Thr Thr Val Thr Val
            100                 105                 110
Ser Ser
```

<210>    37
<211>    330
<212>    PRT
<213>    Artificial Sequence

<220>
<223>    heavy chain variable region w

<400>    37
Ala Ser Thr Lys Gly Pro Ser Val Phe Pro Leu Ala Pro Ser Ser Lys
1                   5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
            35                  40                  45
Gly Val His Thr Phe Pro Ala Val Leu Gln Ser Ser Gly Leu Tyr Ser
            50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
            115                 120                 125
Lys Pro Lys Asp Thr Leu Met Ile Ser Arg Thr Pro Glu Val Thr Cys
            130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
            195                 200                 205
Lys Ala Leu Pro Ala Pro Ile Glu Lys Thr Ile Ser Lys Ala Lys Gly
            210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
            245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
            275                 280                 285
Leu Tyr Ser Lys Leu Thr Val Asp Lys Ser Arg Trp Gln Gln Gly Asn
            290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
            325                 330

<210>    38
<211>    330
<212>    PRT
<213>    Artificial Sequence

<220>
<223>    heavy chain variable region y

```
<400>  38
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
            85                  90                  95
Lys Val Glu Pro Lys Ser Cys Asp Lys Thr His Thr Cys Pro Pro Cys
        100                 105                 110
Pro Ala Pro Glu Leu Leu Gly Gly Pro Ser Val Phe Leu Phe Pro Pro
        115                 120                 125
Lys Pro Lys Asp Thr Leu Tyr Ile Thr Arg Glu Pro Glu Val Thr Cys
    130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
            195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
            245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
    275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
            325                 330


<210>  39
<211>  107
<212>  PRT
<213>  Artificial Sequence

<220>
<223>  humanized antibody light chain kappa constant region


<400>  39
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
```

Thr Tyr Ser Leu Ser Ser Thr Leu Thr Leu Ser Lys Ala Asp Tyr Glu
65              70              75                      80
Lys His Lys Val Tyr Ala Cys Glu Val Thr His Gln Gly Leu Ser Ser
                85              90                      95
Pro Val Thr Lys Ser Phe Asn Arg Gly Glu Cys
            100              105


<210>   40
<211>   105
<212>   PRT
<213>   Artificial Sequence

<220>
<223>   humanized antibody light chain lambda constant region


<400>   40
Gly Gln Pro Lys Ala Asn Pro Thr Val Thr Leu Phe Pro Pro Ser Ser
1               5               10                      15
Glu Glu Leu Gln Ala Asn Lys Ala Thr Leu Val Cys Leu Ile Ser Asp
            20              25                      30
Phe Tyr Pro Gly Ala Val Thr Val Ala Trp Lys Ala Asp Gly Ser Pro
        35              40                      45
Val Lys Ala Gly Val Glu Thr Thr Lys Pro Ser Lys Gln Ser Asn Asn
    50              55                      60
Lys Tyr Ala Ala Ser Ser Tyr Leu Ser Leu Thr Pro Glu Gln Trp Lys
65              70              75                      80
Ser His Arg Ser Tyr Ser Cys Gln Val Thr His Glu Gly Ser Thr Val
                85              90                      95
Glu Lys Thr Val Ala Pro Thr Glu Cys
            100              105


<210>   41
<211>   446
<212>   PRT
<213>   Artificial Sequence

<220>
<223>   Ch147 heavy chain


<400>   41
Glu Val Gln Leu Val Glu Ser Gly Gly Gly Leu Val Gln Pro Glu Gly
1               5               10                      15
Ser Leu Lys Leu Ser Cys Ala Ala Ser Gly Phe Ser Phe Asn Thr Tyr
            20              25                      30
Ala Met Asn Trp Val Arg Gln Ala Pro Gly Lys Gly Leu Glu Trp Val
        35              40                      45
Ala Arg Ile Arg Thr Lys Ser Asn Asn Tyr Ala Thr Tyr Tyr Ala Asp
    50              55                      60
Ser Val Lys Asp Arg Phe Thr Ile Ser Arg Asp Asp Ser Glu Ser Met
65              70              75                      80
Leu Tyr Leu Gln Met Asn Asn Leu Lys Thr Glu Asp Thr Ala Met Tyr
                85              90                      95
Tyr Cys Val Glu Thr Gly Phe Ala Tyr Trp Asp Gln Gly Thr Leu Val
                100             105                     110
Thr Val Ser Ala Ala Ser Thr Lys Gly Pro Ser Val Phe Pro Leu Ala
        115             120                     125
Pro Ser Ser Lys Ser Thr Ser Gly Gly Thr Ala Ala Leu Gly Cys Leu
        130             135                     140
Val Lys Asp Tyr Phe Pro Glu Pro Val Thr Val Ser Trp Asn Ser Gly
145             150                     155                     160
Ala Leu Thr Ser Gly Val His Thr Phe Pro Ala Val Leu Gln Ser Ser
                165             170                     175


65

```
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
            195                 200                 205
Lys Val Asp Lys Lys Val Glu Pro Lys Ser Cys Asp Lys Thr His Thr
            210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                    245                 250                 255
Glu Val Thr Cys Val Val Val Asp Val Ser His Glu Asp Pro Glu Val
            260                 265                 270
Lys Phe Asn Trp Tyr Val Asp Gly Val Glu Val His Asn Ala Lys Thr
            275                 280                 285
Lys Pro Arg Glu Glu Gln Tyr Asn Ser Thr Tyr Arg Val Val Ser Val
            290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                    325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
            355                 360                 365
Lys Gly Phe Tyr Pro Ser Asp Ile Ala Val Glu Trp Glu Ser Asn Gly
            370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                    405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
            435                 440                 445
```

```
<210>    42
<211>    213
<212>    PRT
<213>    Artificial Sequence

<220>
<223>    Ch147 light chain

<400>    42
Gln Ile Val Leu Thr Gln Ser Pro Ala Ile Met Ser Ala Ser Pro Gly
1                5                   10                  15
Glu Lys Val Thr Ile Thr Cys Ser Ala Ser Ser Ser Val Ser Tyr Met
                    20                  25                  30
His Trp Phe Gln Gln Lys Pro Gly Thr Ser Pro Lys Leu Trp Ile Tyr
            35                  40                  45
Ser Thr Ser Asn Leu Ala Ser Gly Val Pro Ala Arg Phe Ser Gly Ser
            50                  55                  60
Gly Ser Gly Thr Ser Tyr Ser Leu Thr Ile Ser Arg Met Glu Ala Glu
65                  70                  75                  80
Asp Ala Ala Thr Tyr Cys Gln Gln Arg Ser Ser Tyr Pro Leu Thr
                    85                  90                  95
Phe Gly Ala Gly Thr Lys Leu Glu Leu Lys Arg Thr Val Ala Ala Pro
            100                 105                 110
Ser Val Phe Ile Phe Pro Pro Ser Asp Glu Gln Leu Lys Ser Gly Thr
            115                 120                 125
Ala Ser Val Val Cys Leu Leu Asn Asn Phe Tyr Pro Arg Glu Ala Lys
            130                 135                 140
Val Gln Trp Lys Val Asp Asn Ala Leu Gln Ser Gly Asn Ser Gln Glu
```

```
                    145                          150                          155                          160
                    Ser Val Thr Glu Gln Asp Ser Lys Asp Ser Thr Tyr Ser Leu Ser Ser
                                        165                          170                          175
                    Thr Leu Thr Leu Ser Lys Ala Asp Tyr Glu Lys His Lys Val Tyr Ala
                                        180                          185                          190
                    Cys Glu Val Thr His Gln Gly Leu Ser Ser Pro Val Thr Lys Ser Phe
                                        195                          200                          205
                    Asn Arg Gly Glu Cys
                        210


<210>   43
<211>   446
<212>   PRT
<213>   Artificial Sequence

<220>
<223>   Hu147-11wk heavy chain


<400>   43
Glu Val Gln Leu Val Glu Ser Gly Gly Gly Leu Val Gln Pro Gly Gly
1                   5                   10                  15
Ser Leu Arg Leu Ser Cys Ala Ala Ser Gly Phe Thr Phe Ser Thr Tyr
                20                  25                  30
Ala Met Asn Trp Val Arg Gln Ala Pro Gly Lys Gly Leu Glu Trp Val
            35                  40                  45
Gly Arg Ile Arg Thr Lys Ser Asn Asn Tyr Ala Thr Tyr Tyr Ala Asp
        50                  55                  60
Ser Val Lys Asp Arg Phe Thr Ile Ser Arg Asp Asp Ser Lys Asn Ser
65                  70                  75                  80
Leu Tyr Leu Gln Met Asn Ser Leu Lys Thr Glu Asp Thr Ala Val Tyr
                85                  90                  95
Tyr Cys Val Glu Thr Gly Phe Ala Tyr Trp Asp Gln Gly Thr Leu Val
                100                 105                 110
Thr Val Ser Ser Ala Ser Thr Lys Gly Pro Ser Val Phe Pro Leu Ala
            115                 120                 125
Pro Ser Ser Lys Ser Thr Ser Gly Gly Thr Ala Ala Leu Gly Cys Leu
        130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
                180                 185                 190
Gly Thr Gln Thr Tyr Ile Cys Asn Val Asn His Lys Pro Ser Asn Thr
                195                 200                 205
Lys Val Asp Lys Lys Val Glu Pro Lys Ser Cys Asp Lys Thr His Thr
        210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Asp Val Ser His Glu Asp Pro Glu Val
                260                 265                 270
Lys Phe Asn Trp Tyr Val Asp Gly Val Glu Val His Asn Ala Lys Thr
                275                 280                 285
Lys Pro Arg Glu Glu Gln Tyr Asn Ser Thr Tyr Arg Val Val Ser Val
        290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
```

```
                    340                      345                      350
         Ser Arg Asp Glu Leu Thr Lys Asn Gln Val Ser Leu Thr Cys Leu Val
                 355                      360                      365
         Lys Gly Phe Tyr Pro Ser Asp Ile Ala Val Glu Trp Glu Ser Asn Gly
             370                      375                      380
         Gln Pro Glu Asn Asn Tyr Lys Thr Thr Pro Pro Val Leu Asp Ser Asp
         385                      390                      395                      400
         Gly Ser Phe Phe Leu Tyr Ser Lys Leu Thr Val Asp Lys Ser Arg Trp
                     405                      410                      415
         Gln Gln Gly Asn Val Phe Ser Cys Ser Val Met His Glu Ala Leu His
                     420                      425                      430
         Asn His Tyr Thr Gln Lys Ser Leu Ser Leu Ser Pro Gly Lys
                 435                      440                      445


         <210>   44
         <211>   446
         <212>   PRT
         <213>   Artificial Sequence

         <220>
         <223>   Hu147-21wk heavy chain


         <400>   44
         Glu Val Gln Leu Val Glu Ser Gly Gly Gly Leu Val Gln Pro Gly Gly
         1               5                      10                      15
         Ser Leu Arg Leu Ser Cys Ala Ala Ser Gly Phe Thr Phe Asn Thr Tyr
                     20                      25                      30
         Ala Met Asn Trp Val Arg Gln Ala Pro Gly Lys Gly Leu Glu Trp Val
                 35                      40                      45
         Ala Arg Ile Arg Thr Lys Ser Asn Asn Tyr Ala Thr Tyr Tyr Ala Asp
             50                      55                      60
         Ser Val Lys Asp Arg Phe Thr Ile Ser Arg Asp Asp Ser Lys Asn Ser
         65                      70                      75                      80
         Leu Tyr Leu Gln Met Asn Ser Leu Lys Thr Glu Asp Thr Ala Val Tyr
                         85                      90                      95
         Tyr Cys Val Glu Thr Gly Phe Ala Tyr Trp Asp Gln Gly Thr Leu Val
                     100                      105                      110
         Thr Val Ser Ser Ala Ser Thr Lys Gly Pro Ser Val Phe Pro Leu Ala
                 115                      120                      125
         Pro Ser Ser Lys Ser Thr Ser Gly Gly Thr Ala Ala Leu Gly Cys Leu
             130                      135                      140
         Val Lys Asp Tyr Phe Pro Glu Pro Val Thr Val Ser Trp Asn Ser Gly
         145                      150                      155                      160
         Ala Leu Thr Ser Gly Val His Thr Phe Pro Ala Val Leu Gln Ser Ser
                     165                      170                      175
         Gly Leu Tyr Ser Leu Ser Ser Val Val Thr Val Pro Ser Ser Ser Leu
                     180                      185                      190
         Gly Thr Gln Thr Tyr Ile Cys Asn Val Asn His Lys Pro Ser Asn Thr
                 195                      200                      205
         Lys Val Asp Lys Lys Val Glu Pro Lys Ser Cys Asp Lys Thr His Thr
             210                      215                      220
         Cys Pro Pro Cys Pro Ala Pro Glu Leu Leu Gly Gly Pro Ser Val Phe
         225                      230                      235                      240
         Leu Phe Pro Pro Lys Pro Lys Asp Thr Leu Met Ile Ser Arg Thr Pro
                     245                      250                      255
         Glu Val Thr Cys Val Val Val Asp Val Ser His Glu Asp Pro Glu Val
                     260                      265                      270
         Lys Phe Asn Trp Tyr Val Asp Gly Val Glu Val His Asn Ala Lys Thr
                     275                      280                      285
         Lys Pro Arg Glu Glu Gln Tyr Asn Ser Thr Tyr Arg Val Val Ser Val
                 290                      295                      300
         Leu Thr Val Leu His Gln Asp Trp Leu Asn Gly Lys Glu Tyr Lys Cys
```

```
                      305                          310                          315                          320
Lys Val Ser Asn Lys Ala Leu Pro Ala Pro Ile Glu Lys Thr Ile Ser
                325                          330                          335
Lys Ala Lys Gly Gln Pro Arg Glu Pro Gln Val Tyr Thr Leu Pro Pro
            340                          345                          350
Ser Arg Asp Glu Leu Thr Lys Asn Gln Val Ser Leu Thr Cys Leu Val
        355                          360                          365
Lys Gly Phe Tyr Pro Ser Asp Ile Ala Val Glu Trp Glu Ser Asn Gly
    370                          375                          380
Gln Pro Glu Asn Asn Tyr Lys Thr Thr Pro Pro Val Leu Asp Ser Asp
385                          390                          395                          400
Gly Ser Phe Phe Leu Tyr Ser Lys Leu Thr Val Asp Lys Ser Arg Trp
                405                          410                          415
Gln Gln Gly Asn Val Phe Ser Cys Ser Val Met His Glu Ala Leu His
                420                          425                          430
Asn His Tyr Thr Gln Lys Ser Leu Ser Leu Ser Pro Gly Lys
                435                          440                          445
```

<210> 45
<211> 446
<212> PRT
<213> Artificial Sequence

<220>
<223> Hu147-31wk heavy chain


<400> 45
```
Glu Val Gln Leu Val Glu Ser Gly Gly Gly Leu Val Gln Pro Gly Gly
1                   5                           10                          15
Ser Leu Arg Leu Ser Cys Ala Ala Ser Gly Phe Ser Phe Asn Thr Tyr
            20                          25                          30
Ala Met Asn Trp Val Arg Gln Ala Pro Gly Lys Gly Leu Glu Trp Val
        35                          40                          45
Ala Arg Ile Arg Thr Lys Ser Asn Asn Tyr Ala Thr Tyr Tyr Ala Asp
    50                          55                          60
Ser Val Lys Asp Arg Phe Thr Ile Ser Arg Asp Asp Ser Glu Ser Ser
65                          70                          75                          80
Leu Tyr Leu Gln Met Asn Ser Leu Lys Thr Glu Asp Thr Ala Val Tyr
                85                          90                          95
Tyr Cys Val Glu Thr Gly Phe Ala Tyr Trp Asp Gln Gly Thr Leu Val
            100                          105                          110
Thr Val Ser Ser Ala Ser Thr Lys Gly Pro Ser Val Phe Pro Leu Ala
            115                          120                          125
Pro Ser Ser Lys Ser Thr Ser Gly Gly Thr Ala Ala Leu Gly Cys Leu
            130                          135                          140
Val Lys Asp Tyr Phe Pro Glu Pro Val Thr Val Ser Trp Asn Ser Gly
145                          150                          155                          160
Ala Leu Thr Ser Gly Val His Thr Phe Pro Ala Val Leu Gln Ser Ser
                165                          170                          175
Gly Leu Tyr Ser Leu Ser Ser Val Val Thr Val Pro Ser Ser Ser Leu
            180                          185                          190
Gly Thr Gln Thr Tyr Ile Cys Asn Val Asn His Lys Pro Ser Asn Thr
            195                          200                          205
Lys Val Asp Lys Lys Val Glu Pro Lys Ser Cys Asp Lys Thr His Thr
            210                          215                          220
Cys Pro Pro Cys Pro Ala Pro Glu Leu Leu Gly Gly Pro Ser Val Phe
225                          230                          235                          240
Leu Phe Pro Pro Lys Pro Lys Asp Thr Leu Met Ile Ser Arg Thr Pro
                245                          250                          255
Glu Val Thr Cys Val Val Val Asp Val Ser His Glu Asp Pro Glu Val
            260                          265                          270
Lys Phe Asn Trp Tyr Val Asp Gly Val Glu Val His Asn Ala Lys Thr
```

```
                275                 280                 285
        Lys Pro Arg Glu Glu Gln Tyr Asn Ser Thr Tyr Arg Val Val Ser Val
            290                 295                 300
        Leu Thr Val Leu His Gln Asp Trp Leu Asn Gly Lys Glu Tyr Lys Cys
            305                 310                 315                 320
        Lys Val Ser Asn Lys Ala Leu Pro Ala Pro Ile Glu Lys Thr Ile Ser
                        325                 330                 335
        Lys Ala Lys Gly Gln Pro Arg Glu Pro Gln Val Tyr Thr Leu Pro Pro
                    340                 345                 350
        Ser Arg Asp Glu Leu Thr Lys Asn Gln Val Ser Leu Thr Cys Leu Val
                    355                 360                 365
        Lys Gly Phe Tyr Pro Ser Asp Ile Ala Val Glu Trp Glu Ser Asn Gly
                370                 375                 380
        Gln Pro Glu Asn Asn Tyr Lys Thr Thr Pro Pro Val Leu Asp Ser Asp
            385                 390                 395                 400
        Gly Ser Phe Phe Leu Tyr Ser Lys Leu Thr Val Asp Lys Ser Arg Trp
                        405                 410                 415
        Gln Gln Gly Asn Val Phe Ser Cys Ser Val Met His Glu Ala Leu His
                    420                 425                 430
        Asn His Tyr Thr Gln Lys Ser Leu Ser Leu Ser Pro Gly Lys
                435                 440                 445


        <210>   46
        <211>   446
        <212>   PRT
        <213>   Artificial Sequence


        <220>
        <223>   Hu147-11yk heavy chain



        <400>   46
        Glu Val Gln Leu Val Glu Ser Gly Gly Gly Leu Val Gln Pro Gly Gly
        1               5                   10                  15
        Ser Leu Arg Leu Ser Cys Ala Ala Ser Gly Phe Thr Phe Ser Thr Tyr
                    20                  25                  30
        Ala Met Asn Trp Val Arg Gln Ala Pro Gly Lys Gly Leu Glu Trp Val
                    35                  40                  45
        Gly Arg Ile Arg Thr Lys Ser Asn Asn Tyr Ala Thr Tyr Tyr Ala Asp
                50                  55                  60
        Ser Val Lys Asp Arg Phe Thr Ile Ser Arg Asp Asp Ser Lys Asn Ser
        65                  70                  75                  80
        Leu Tyr Leu Gln Met Asn Ser Leu Lys Thr Glu Asp Thr Ala Val Tyr
                        85                  90                  95
        Tyr Cys Val Glu Thr Gly Phe Ala Tyr Trp Asp Gln Gly Thr Leu Val
                    100                 105                 110
        Thr Val Ser Ser Ala Ser Thr Lys Gly Pro Ser Val Phe Pro Leu Ala
                    115                 120                 125
        Pro Ser Ser Lys Ser Thr Ser Gly Gly Thr Ala Ala Leu Gly Cys Leu
                    130                 135                 140
        Val Lys Asp Tyr Phe Pro Glu Pro Val Thr Val Ser Trp Asn Ser Gly
        145                 150                 155                 160
        Ala Leu Thr Ser Gly Val His Thr Phe Pro Ala Val Leu Gln Ser Ser
                        165                 170                 175
        Gly Leu Tyr Ser Leu Ser Ser Val Val Thr Val Pro Ser Ser Ser Leu
                    180                 185                 190
        Gly Thr Gln Thr Tyr Ile Cys Asn Val Asn His Lys Pro Ser Asn Thr
                    195                 200                 205
        Lys Val Asp Lys Lys Val Glu Pro Lys Ser Cys Asp Lys Thr His Thr
                    210                 215                 220
        Cys Pro Pro Cys Pro Ala Pro Glu Leu Leu Gly Gly Pro Ser Val Phe
        225                 230                 235                 240
        Leu Phe Pro Pro Lys Pro Lys Asp Thr Leu Tyr Ile Thr Arg Glu Pro
```

```
                       245                    250                    255
        Glu Val Thr Cys Val Val Val Asp Val Ser His Glu Asp Pro Glu Val
                    260                    265                    270
        Lys Phe Asn Trp Tyr Val Asp Gly Val Glu Val His Asn Ala Lys Thr
                    275                    280                    285
        Lys Pro Arg Glu Glu Gln Tyr Asn Ser Thr Tyr Arg Val Val Ser Val
            290                    295                    300
        Leu Thr Val Leu His Gln Asp Trp Leu Asn Gly Lys Glu Tyr Lys Cys
        305                    310                    315                    320
        Lys Val Ser Asn Lys Ala Leu Pro Ala Pro Ile Glu Lys Thr Ile Ser
                    325                    330                    335
        Lys Ala Lys Gly Gln Pro Arg Glu Pro Gln Val Tyr Thr Leu Pro Pro
                340                    345                    350
        Ser Arg Asp Glu Leu Thr Lys Asn Gln Val Ser Leu Thr Cys Leu Val
                355                    360                    365
        Lys Gly Phe Tyr Pro Ser Asp Ile Ala Val Glu Trp Glu Ser Asn Gly
                370                    375                    380
        Gln Pro Glu Asn Asn Tyr Lys Thr Thr Pro Pro Val Leu Asp Ser Asp
        385                    390                    395                    400
        Gly Ser Phe Phe Leu Tyr Ser Lys Leu Thr Val Asp Lys Ser Arg Trp
                    405                    410                    415
        Gln Gln Gly Asn Val Phe Ser Cys Ser Val Met His Glu Ala Leu His
                    420                    425                    430
        Asn His Tyr Thr Gln Lys Ser Leu Ser Leu Ser Pro Gly Lys
                    435                    440                    445
```

```
<210>   47
<211>   446
<212>   PRT
<213>   Artificial Sequence

<220>
<223>   Hu147-21yk heavy chain


<400>   47
Glu Val Gln Leu Val Glu Ser Gly Gly Gly Leu Val Gln Pro Gly Gly
1                   5                   10                  15
Ser Leu Arg Leu Ser Cys Ala Ala Ser Gly Phe Thr Phe Asn Thr Tyr
            20                  25                  30
Ala Met Asn Trp Val Arg Gln Ala Pro Gly Lys Gly Leu Glu Trp Val
            35                  40                  45
Ala Arg Ile Arg Thr Lys Ser Asn Asn Tyr Ala Thr Tyr Tyr Ala Asp
            50                  55                  60
Ser Val Lys Asp Arg Phe Thr Ile Ser Arg Asp Asp Ser Lys Asn Ser
65                  70                  75                  80
Leu Tyr Leu Gln Met Asn Ser Leu Lys Thr Glu Asp Thr Ala Val Tyr
                85                  90                  95
Tyr Cys Val Glu Thr Gly Phe Ala Tyr Trp Asp Gln Gly Thr Leu Val
            100                 105                 110
Thr Val Ser Ser Ala Ser Thr Lys Gly Pro Ser Val Phe Pro Leu Ala
            115                 120                 125
Pro Ser Ser Lys Ser Thr Ser Gly Gly Thr Ala Ala Leu Gly Cys Leu
            130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
            195                 200                 205
Lys Val Asp Lys Lys Val Glu Pro Lys Ser Cys Asp Lys Thr His Thr
```

```
              210                         215                         220
Cys Pro Pro Cys Pro Ala Pro Glu Leu Leu Gly Gly Pro Ser Val Phe
225                         230                         235                         240
Leu Phe Pro Pro Lys Pro Lys Asp Thr Leu Tyr Ile Thr Arg Glu Pro
                245                         250                         255
Glu Val Thr Cys Val Val Val Asp Val Ser His Glu Asp Pro Glu Val
                260                         265                         270
Lys Phe Asn Trp Tyr Val Asp Gly Val Glu Val His Asn Ala Lys Thr
                275                         280                         285
Lys Pro Arg Glu Glu Gln Tyr Asn Ser Thr Tyr Arg Val Val Ser Val
                290                         295                         300
Leu Thr Val Leu His Gln Asp Trp Leu Asn Gly Lys Glu Tyr Lys Cys
305                         310                         315                         320
Lys Val Ser Asn Lys Ala Leu Pro Ala Pro Ile Glu Lys Thr Ile Ser
                325                         330                         335
Lys Ala Lys Gly Gln Pro Arg Glu Pro Gln Val Tyr Thr Leu Pro Pro
                340                         345                         350
Ser Arg Asp Glu Leu Thr Lys Asn Gln Val Ser Leu Thr Cys Leu Val
                355                         360                         365
Lys Gly Phe Tyr Pro Ser Asp Ile Ala Val Glu Trp Glu Ser Asn Gly
                370                         375                         380
Gln Pro Glu Asn Asn Tyr Lys Thr Thr Pro Pro Val Leu Asp Ser Asp
385                         390                         395                         400
Gly Ser Phe Phe Leu Tyr Ser Lys Leu Thr Val Asp Lys Ser Arg Trp
                405                         410                         415
Gln Gln Gly Asn Val Phe Ser Cys Ser Val Met His Glu Ala Leu His
                420                         425                         430
Asn His Tyr Thr Gln Lys Ser Leu Ser Leu Ser Pro Gly Lys
                435                         440                         445
```

<210> 48
<211> 446
<212> PRT
<213> Artificial Sequence

<220>
<223> Hu147-31yk heavy chain


<400> 48
```
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Ser Phe Asn Thr Tyr
            20                  25                  30
Ala Met Asn Trp Val Arg Gln Ala Pro Gly Lys Gly Leu Glu Trp Val
            35                  40                  45
Ala Arg Ile Arg Thr Lys Ser Asn Asn Tyr Ala Thr Tyr Tyr Ala Asp
            50                  55                  60
Ser Val Lys Asp Arg Phe Thr Ile Ser Arg Asp Asp Ser Glu Ser Ser
65                  70                  75                  80
Leu Tyr Leu Gln Met Asn Ser Leu Lys Thr Glu Asp Thr Ala Val Tyr
                85                  90                  95
Tyr Cys Val Glu Thr Gly Phe Ala Tyr Trp Asp Gln Gly Thr Leu Val
            100                 105                 110
Thr Val Ser Ser Ala Ser Thr Lys Gly Pro Ser Val Phe Pro Leu Ala
            115                 120                 125
Pro Ser Ser Lys Ser Thr Ser Gly Gly Thr Ala Ala Leu Gly Cys Leu
            130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
```

```
                    180                   185                   190
Gly Thr Gln Thr Tyr Ile Cys Asn Val Asn His Lys Pro Ser Asn Thr
        195                   200                   205
Lys Val Asp Lys Lys Val Glu Pro Lys Ser Cys Asp Lys Thr His Thr
        210                   215                   220
Cys Pro Pro Cys Pro Ala Pro Glu Leu Leu Gly Gly Pro Ser Val Phe
225                   230                   235                   240
Leu Phe Pro Pro Lys Pro Lys Asp Thr Leu Tyr Ile Thr Arg Glu Pro
                245                   250                   255
Glu Val Thr Cys Val Val Val Asp Val Ser His Glu Asp Pro Glu Val
            260                   265                   270
Lys Phe Asn Trp Tyr Val Asp Gly Val Glu Val His Asn Ala Lys Thr
        275                   280                   285
Lys Pro Arg Glu Glu Gln Tyr Asn Ser Thr Tyr Arg Val Val Ser Val
        290                   295                   300
Leu Thr Val Leu His Gln Asp Trp Leu Asn Gly Lys Glu Tyr Lys Cys
305                   310                   315                   320
Lys Val Ser Asn Lys Ala Leu Pro Ala Pro Ile Glu Lys Thr Ile Ser
                325                   330                   335
Lys Ala Lys Gly Gln Pro Arg Glu Pro Gln Val Tyr Thr Leu Pro Pro
            340                   345                   350
Ser Arg Asp Glu Leu Thr Lys Asn Gln Val Ser Leu Thr Cys Leu Val
        355                   360                   365
Lys Gly Phe Tyr Pro Ser Asp Ile Ala Val Glu Trp Glu Ser Asn Gly
        370                   375                   380
Gln Pro Glu Asn Asn Tyr Lys Thr Thr Pro Pro Val Leu Asp Ser Asp
385                   390                   395                   400
Gly Ser Phe Phe Leu Tyr Ser Lys Leu Thr Val Asp Lys Ser Arg Trp
                405                   410                   415
Gln Gln Gly Asn Val Phe Ser Cys Ser Val Met His Glu Ala Leu His
            420                   425                   430
Asn His Tyr Thr Gln Lys Ser Leu Ser Leu Ser Pro Gly Lys
            435                   440                   445
```

<210> 49
<211> 213
<212> PRT
<213> Artificial Sequence

<220>
<223> Hu147-11wk light chain


<400> 49
```
Glu Ile Val Leu Thr Gln Ser Pro Ala Thr Leu Ser Leu Ser Pro Gly
1               5                   10                  15
Glu Arg Ala Thr Leu Ser Cys Ser Ala Ser Ser Ser Val Ser Tyr Met
            20                  25                  30
His Trp Phe Gln Gln Lys Pro Gly Gln Ser Pro Arg Leu Leu Ile Tyr
        35                  40                  45
Ser Thr Ser Asn Leu Ala Ser Gly Ile Pro Ala Arg Phe Ser Gly Ser
        50                  55                  60
Gly Ser Gly Thr Asp Tyr Thr Leu Thr Ile Ser Ser Leu Glu Pro Glu
65                  70                  75                  80
Asp Phe Ala Val Tyr Tyr Cys Gln Gln Arg Ser Ser Tyr Pro Leu Thr
                85                  90                  95
Phe Gly Gln Gly Thr Lys Leu Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
        130                 135                 140
Val Gln Trp Lys Val Asp Asn Ala Leu Gln Ser Gly Asn Ser Gln Glu
```

```
145                     150                     155                     160
Ser Val Thr Glu Gln Asp Ser Lys Asp Ser Thr Tyr Ser Leu Ser Ser
                165                     170                     175
Thr Leu Thr Leu Ser Lys Ala Asp Tyr Glu Lys His Lys Val Tyr Ala
                180                     185                     190
Cys Glu Val Thr His Gln Gly Leu Ser Ser Pro Val Thr Lys Ser Phe
                195                     200                     205
Asn Arg Gly Glu Cys
        210


<210>   50
<211>   213
<212>   PRT
<213>   Artificial Sequence

<220>
<223>   Hu147-12wk light chain


<400>   50
Glu Ile Val Leu Thr Gln Ser Pro Ala Thr Leu Ser Leu Ser Pro Gly
1                   5                   10                      15
Glu Arg Ala Thr Leu Ser Cys Ser Ala Ser Ser Ser Val Ser Tyr Met
                20                      25                      30
His Trp Phe Gln Gln Lys Pro Gly Gln Ser Pro Lys Leu Leu Ile Tyr
                35                      40                      45
Ser Thr Ser Asn Leu Ala Ser Gly Ile Pro Ala Arg Phe Ser Gly Ser
        50                      55                      60
Gly Ser Gly Thr Asp Tyr Thr Leu Thr Ile Ser Ser Leu Glu Pro Glu
65                      70                      75                      80
Asp Phe Ala Val Tyr Tyr Cys Gln Gln Arg Ser Ser Tyr Pro Leu Thr
                85                      90                      95
Phe Gly Gln Gly Thr Lys Leu Glu Ile Lys Arg Thr Val Ala Ala Pro
                100                     105                     110
Ser Val Phe Ile Phe Pro Pro Ser Asp Glu Gln Leu Lys Ser Gly Thr
                115                     120                     125
Ala Ser Val Val Cys Leu Leu Asn Asn Phe Tyr Pro Arg Glu Ala Lys
        130                     135                     140
Val Gln Trp Lys Val Asp Asn Ala Leu Gln Ser Gly Asn Ser Gln Glu
145                     150                     155                     160
Ser Val Thr Glu Gln Asp Ser Lys Asp Ser Thr Tyr Ser Leu Ser Ser
                165                     170                     175
Thr Leu Thr Leu Ser Lys Ala Asp Tyr Glu Lys His Lys Val Tyr Ala
                180                     185                     190
Cys Glu Val Thr His Gln Gly Leu Ser Ser Pro Val Thr Lys Ser Phe
                195                     200                     205
Asn Arg Gly Glu Cys
        210


<210>   51
<211>   213
<212>   PRT
<213>   Artificial Sequence

<220>
<223>   Hu147-13wk light chain


<400>   51
Glu Ile Val Leu Thr Gln Ser Pro Ala Thr Leu Ser Leu Ser Pro Gly
1                   5                   10                      15
Glu Arg Ala Thr Leu Ser Cys Ser Ala Ser Ser Ser Val Ser Tyr Met
                20                      25                      30
```

```
His Trp Phe Gln Gln Lys Pro Gly Gln Ser Pro Lys Leu Trp Ile Tyr
        35                  40                  45
Ser Thr Ser Asn Leu Ala Ser Gly Val Pro Ala Arg Phe Ser Gly Ser
    50                  55                  60
Gly Ser Gly Thr Asp Tyr Thr Leu Thr Ile Ser Ser Leu Glu Pro Glu
65                  70                  75                  80
Asp Phe Ala Val Tyr Tyr Cys Gln Gln Arg Ser Ser Tyr Pro Leu Thr
                85                  90                  95
Phe Gly Gln Gly Thr Lys Leu Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
        210
```

```
<210>   52
<211>   213
<212>   PRT
<213>   Artificial Sequence

<220>
<223>   Hu147-14wk light chain


<400>   52
Asp Ile Gln Met Thr Gln Ser Pro Ser Ser Leu Ser Ala Ser Val Gly
1               5                   10                  15
Asp Arg Val Thr Ile Thr Cys Ser Ala Ser Ser Ser Val Ser Tyr Met
            20                  25                  30
His Trp Phe Gln Gln Lys Pro Gly Lys Ser Pro Lys Leu Leu Ile Tyr
        35                  40                  45
Ser Thr Ser Asn Leu Ala Ser Gly Val Pro Ser Arg Phe Ser Gly Ser
    50                  55                  60
Gly Ser Gly Thr Asp Tyr Thr Leu Thr Ile Ser Ser Leu Gln Pro Glu
65                  70                  75                  80
Asp Phe Ala Thr Tyr Tyr Cys Gln Gln Arg Ser Ser Tyr Pro Leu Thr
                85                  90                  95
Phe Gly Gln Gly Thr Lys Leu Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
        210
```

<210> 53
<211> 213
<212> PRT
<213> Artificial Sequence

<220>
<223> Hu147-15wk light chain

<400> 53
Asp Ile Gln Leu Thr Gln Ser Pro Ser Ser Leu Ser Ala Ser Val Gly
1               5                   10                  15
Asp Arg Val Thr Ile Thr Cys Ser Ala Ser Ser Ser Val Ser Tyr Met
            20                  25                  30
His Trp Phe Gln Gln Lys Pro Gly Lys Ser Pro Lys Leu Trp Ile Tyr
        35                  40                  45
Ser Thr Ser Asn Leu Ala Ser Gly Val Pro Ser Arg Phe Ser Gly Ser
    50                  55                  60
Gly Ser Gly Thr Asp Tyr Thr Leu Thr Ile Ser Ser Leu Gln Pro Glu
65                  70                  75                  80
Asp Phe Ala Thr Tyr Tyr Cys Gln Gln Arg Ser Ser Tyr Pro Leu Thr
                85                  90                  95
Phe Gly Gln Gly Thr Lys Leu Glu Ile Lys Arg Thr Val Ala Ala Pro
            100                 105                 110
Ser Val Phe Ile Phe Pro Pro Ser Asp Glu Gln Leu Lys Ser Gly Thr
            115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
            195                 200                 205
Asn Arg Gly Glu Cys
            210

<210> 54
<211> 444
<212> PRT
<213> Artificial Sequence

<220>
<223> Ch164 heavy chain

<400> 54
Gln Val Gln Leu Lys Gln Ser Gly Pro Gly Leu Val Gln Pro Ser Gln
1               5                   10                  15
Ser Leu Ser Ile Thr Cys Thr Val Ser Gly Phe Ser Leu Thr Thr Phe
            20                  25                  30
Gly Val His Trp Ile Arg Gln Ser Pro Gly Lys Gly Leu Glu Trp Leu
        35                  40                  45
Gly Val Ile Trp Arg Arg Gly Gly Thr Asp Tyr Asn Ala Ala Phe Met
    50                  55                  60
Ser Arg Leu Ser Ile Thr Lys Asp Asn Ser Arg Ser His Val Phe Phe
65                  70                  75                  80
Lys Met Thr Ser Leu Gln Thr Asp Asp Ser Ala Ile Tyr Tyr Cys Ala
                85                  90                  95
Arg Asp Gly Gly Phe Asp Tyr Trp Gly Gln Gly Thr Thr Leu Thr Val

```
                  100                        105                        110
         Ser Ser Ala Ser Thr Lys Gly Pro Ser Val Phe Pro Leu Ala Pro Ser
                      115                        120                        125
         Ser Lys Ser Thr Ser Gly Gly Thr Ala Ala Leu Gly Cys Leu Val Lys
             130                        135                        140
         Asp Tyr Phe Pro Glu Pro Val Thr Val Ser Trp Asn Ser Gly Ala Leu
         145                        150                        155                        160
         Thr Ser Gly Val His Thr Phe Pro Ala Val Leu Gln Ser Ser Gly Leu
                          165                        170                        175
         Tyr Ser Leu Ser Ser Val Val Thr Val Pro Ser Ser Ser Leu Gly Thr
                      180                        185                        190
         Gln Thr Tyr Ile Cys Asn Val Asn His Lys Pro Ser Asn Thr Lys Val
                  195                        200                        205
         Asp Lys Lys Val Glu Pro Lys Ser Cys Asp Lys Thr His Thr Cys Pro
             210                        215                        220
         Pro Cys Pro Ala Pro Glu Leu Leu Gly Gly Pro Ser Val Phe Leu Phe
         225                        230                        235                        240
         Pro Pro Lys Pro Lys Asp Thr Leu Met Ile Ser Arg Thr Pro Glu Val
                          245                        250                        255
         Thr Cys Val Val Val Asp Val Ser His Glu Asp Pro Glu Val Lys Phe
                      260                        265                        270
         Asn Trp Tyr Val Asp Gly Val Glu Val His Asn Ala Lys Thr Lys Pro
                  275                        280                        285
         Arg Glu Glu Gln Tyr Asn Ser Thr Tyr Arg Val Val Ser Val Leu Thr
             290                        295                        300
         Val Leu His Gln Asp Trp Leu Asn Gly Lys Glu Tyr Lys Cys Lys Val
         305                        310                        315                        320
         Ser Asn Lys Ala Leu Pro Ala Pro Ile Glu Lys Thr Ile Ser Lys Ala
                          325                        330                        335
         Lys Gly Gln Pro Arg Glu Pro Gln Val Tyr Thr Leu Pro Pro Ser Arg
                      340                        345                        350
         Asp Glu Leu Thr Lys Asn Gln Val Ser Leu Thr Cys Leu Val Lys Gly
                  355                        360                        365
         Phe Tyr Pro Ser Asp Ile Ala Val Glu Trp Glu Ser Asn Gly Gln Pro
             370                        375                        380
         Glu Asn Asn Tyr Lys Thr Thr Pro Pro Val Leu Asp Ser Asp Gly Ser
         385                        390                        395                        400
         Phe Phe Leu Tyr Ser Lys Leu Thr Val Asp Lys Ser Arg Trp Gln Gln
                          405                        410                        415
         Gly Asn Val Phe Ser Cys Ser Val Met His Glu Ala Leu His Asn His
                      420                        425                        430
         Tyr Thr Gln Lys Ser Leu Ser Leu Ser Pro Gly Lys
             435                        440
```

```
<210>    55
<211>    214
<212>    PRT
<213>    Artificial Sequence

<220>
<223>    Ch164 light chain


<400>    55
Gln Ala Val Val Thr Gln Glu Ser Ala Leu Thr Thr Ser Pro Gly Gly
1                   5                       10                      15
Thr Val Ile Leu Thr Cys Arg Ser Ser Ile Gly Ala Val Thr Thr Ser
                20                      25                      30
Asn Tyr Ala Asn Trp Val Gln Glu Lys Pro Asp His Leu Phe Thr Gly
            35                      40                      45
Leu Ile Gly Gly Thr Ser Asn Arg Ala Pro Gly Val Pro Val Arg Phe
        50                      55                      60
Ser Gly Ser Leu Ile Gly Asp Lys Ala Ala Leu Thr Ile Thr Gly Ala
```

```
                              65                            70                            75                            80
Gln Ala Glu Asp Asp Ala Met Tyr Phe Cys Ala Leu Trp Tyr Ser Thr
                    85                            90                            95
His Tyr Val Phe Gly Gly Gly Thr Lys Val Thr Val Leu Gly Gln Pro
            100                           105                           110
Lys Ala Asn Pro Thr Val Thr Leu Phe Pro Pro Ser Ser Glu Glu Leu
        115                           120                           125
Gln Ala Asn Lys Ala Thr Leu Val Cys Leu Ile Ser Asp Phe Tyr Pro
    130                           135                           140
Gly Ala Val Thr Val Ala Trp Lys Ala Asp Gly Ser Pro Val Lys Ala
145                           150                           155                           160
Gly Val Glu Thr Thr Lys Pro Ser Lys Gln Ser Asn Asn Lys Tyr Ala
                165                           170                           175
Ala Ser Ser Tyr Leu Ser Leu Thr Pro Glu Gln Trp Lys Ser His Arg
            180                           185                           190
Ser Tyr Ser Cys Gln Val Thr His Glu Gly Ser Thr Val Glu Lys Thr
        195                           200                           205
Val Ala Pro Thr Glu Cys
    210


<210>  56
<211>  444
<212>  PRT
<213>  Artificial Sequence

<220>
<223>  Hu164-57wl heavy chain


<400>  56
Glu Val Thr Leu Lys Glu Ser Gly Pro Val Leu Val Lys Pro Thr Glu
1               5                             10                            15
Thr Leu Thr Leu Thr Cys Thr Val Ser Gly Phe Ser Leu Ser Thr Phe
            20                            25                            30
Gly Val His Trp Ile Arg Gln Pro Pro Gly Lys Ala Leu Glu Trp Leu
            35                            40                            45
Ala Val Ile Trp Arg Arg Gly Gly Thr Asp Tyr Asn Ala Ala Phe Met
        50                            55                            60
Ser Arg Leu Thr Ile Ser Lys Asp Thr Ser Lys Ser Gln Val Val Leu
65                            70                            75                            80
Thr Met Thr Asn Met Asp Pro Val Asp Thr Ala Thr Tyr Tyr Cys Ala
                85                            90                            95
Arg Asp Gly Gly Phe Asp Tyr Trp Gly Gln Gly Thr Thr Val Thr Val
            100                           105                           110
Ser Ser Ala Ser Thr Lys Gly Pro Ser Val Phe Pro Leu Ala Pro Ser
        115                           120                           125
Ser Lys Ser Thr Ser Gly Gly Thr Ala Ala Leu Gly Cys Leu Val Lys
    130                           135                           140
Asp Tyr Phe Pro Glu Pro Val Thr Val Ser Trp Asn Ser Gly Ala Leu
145                           150                           155                           160
Thr Ser Gly Val His Thr Phe Pro Ala Val Leu Gln Ser Ser Gly Leu
                165                           170                           175
Tyr Ser Leu Ser Ser Val Val Thr Val Pro Ser Ser Ser Leu Gly Thr
            180                           185                           190
Gln Thr Tyr Ile Cys Asn Val Asn His Lys Pro Ser Asn Thr Lys Val
        195                           200                           205
Asp Lys Lys Val Glu Pro Lys Ser Cys Asp Lys Thr His Thr Cys Pro
    210                           215                           220
Pro Cys Pro Ala Pro Glu Leu Leu Gly Gly Pro Ser Val Phe Leu Phe
225                           230                           235                           240
Pro Pro Lys Pro Lys Asp Thr Leu Met Ile Ser Arg Thr Pro Glu Val
                245                           250                           255
Thr Cys Val Val Val Asp Val Ser His Glu Asp Pro Glu Val Lys Phe
```

```
                  260                        265                        270
Asn Trp Tyr Val Asp Gly Val Glu Val His Asn Ala Lys Thr Lys Pro
        275                        280                        285
Arg Glu Gln Tyr Asn Ser Thr Tyr Arg Val Val Ser Val Leu Thr
    290                        295                        300
Val Leu His Gln Asp Trp Leu Asn Gly Lys Glu Tyr Lys Cys Lys Val
305                        310                        315                        320
Ser Asn Lys Ala Leu Pro Ala Pro Ile Glu Lys Thr Ile Ser Lys Ala
                325                        330                        335
Lys Gly Gln Pro Arg Glu Pro Gln Val Tyr Thr Leu Pro Pro Ser Arg
                340                        345                        350
Asp Glu Leu Thr Lys Asn Gln Val Ser Leu Thr Cys Leu Val Lys Gly
        355                        360                        365
Phe Tyr Pro Ser Asp Ile Ala Val Glu Trp Glu Ser Asn Gly Gln Pro
    370                        375                        380
Glu Asn Asn Tyr Lys Thr Thr Pro Pro Val Leu Asp Ser Asp Gly Ser
385                        390                        395                        400
Phe Phe Leu Tyr Ser Lys Leu Thr Val Asp Lys Ser Arg Trp Gln Gln
                405                        410                        415
Gly Asn Val Phe Ser Cys Ser Val Met His Glu Ala Leu His Asn His
                420                        425                        430
Tyr Thr Gln Lys Ser Leu Ser Leu Ser Pro Gly Lys
        435                        440
```

<210> 57
<211> 444
<212> PRT
<213> Artificial Sequence

<220>
<223> Hu164-67wl heavy chain

<400> 57
```
Glu Val Thr Leu Lys Glu Ser Gly Pro Val Leu Val Lys Pro Thr Glu
1                   5                   10                  15
Thr Leu Thr Leu Thr Cys Thr Val Ser Gly Phe Ser Leu Ser Thr Phe
            20                  25                  30
Gly Val His Trp Ile Arg Gln Pro Pro Gly Lys Gly Leu Glu Trp Leu
        35                  40                  45
Gly Val Ile Trp Arg Arg Gly Gly Thr Asp Tyr Asn Ala Ala Phe Met
    50                  55                  60
Ser Arg Leu Thr Ile Ser Lys Asp Thr Ser Lys Ser Gln Val Val Phe
65                  70                  75                  80
Thr Met Thr Asn Met Asp Pro Val Asp Thr Ala Thr Tyr Tyr Cys Ala
            85                  90                  95
Arg Asp Gly Gly Phe Asp Tyr Trp Gly Gln Gly Thr Thr Val Thr Val
        100                 105                 110
Ser Ser Ala Ser Thr Lys Gly Pro Ser Val Phe Pro Leu Ala Pro Ser
        115                 120                 125
Ser Lys Ser Thr Ser Gly Gly Thr Ala Ala Leu Gly Cys Leu Val Lys
        130                 135                 140
Asp Tyr Phe Pro Glu Pro Val Thr Val Ser Trp Asn Ser Gly Ala Leu
145                 150                 155                 160
Thr Ser Gly Val His Thr Phe Pro Ala Val Leu Gln Ser Ser Gly Leu
                165                 170                 175
Tyr Ser Leu Ser Ser Val Val Thr Val Pro Ser Ser Ser Leu Gly Thr
            180                 185                 190
Gln Thr Tyr Ile Cys Asn Val Asn His Lys Pro Ser Asn Thr Lys Val
        195                 200                 205
Asp Lys Lys Val Glu Pro Lys Ser Cys Asp Lys Thr His Thr Cys Pro
    210                 215                 220
Pro Cys Pro Ala Pro Glu Leu Leu Gly Gly Pro Ser Val Phe Leu Phe
```

```
                    225                         230                         235                         240
Pro Pro Lys Pro Lys Asp Thr Leu Met Ile Ser Arg Thr Pro Glu Val
                        245                         250                         255
Thr Cys Val Val Asp Val Ser His Glu Asp Pro Glu Val Lys Phe
                260                         265                         270
Asn Trp Tyr Val Asp Gly Val Glu Val His Asn Ala Lys Thr Lys Pro
            275                         280                         285
Arg Glu Glu Gln Tyr Asn Ser Thr Tyr Arg Val Val Ser Val Leu Thr
        290                         295                         300
Val Leu His Gln Asp Trp Leu Asn Gly Lys Glu Tyr Lys Cys Lys Val
    305                         310                         315                         320
Ser Asn Lys Ala Leu Pro Ala Pro Ile Glu Lys Thr Ile Ser Lys Ala
                325                         330                         335
Lys Gly Gln Pro Arg Glu Pro Gln Val Tyr Thr Leu Pro Pro Ser Arg
                340                         345                         350
Asp Glu Leu Thr Lys Asn Gln Val Ser Leu Thr Cys Leu Val Lys Gly
            355                         360                         365
Phe Tyr Pro Ser Asp Ile Ala Val Glu Trp Glu Ser Asn Gly Gln Pro
        370                         375                         380
Glu Asn Asn Tyr Lys Thr Thr Pro Pro Val Leu Asp Ser Asp Gly Ser
    385                         390                         395                         400
Phe Phe Leu Tyr Ser Lys Leu Thr Val Asp Lys Ser Arg Trp Gln Gln
                405                         410                         415
Gly Asn Val Phe Ser Cys Ser Val Met His Glu Ala Leu His Asn His
                420                         425                         430
Tyr Thr Gln Lys Ser Leu Ser Leu Ser Pro Gly Lys
            435                         440


<210>   58
<211>   444
<212>   PRT
<213>   Artificial Sequence

<220>
<223>   Hu164-77wl heavy chain


<400>   58
Glu Val Gln Leu Gln Glu Ser Gly Pro Gly Leu Val Lys Pro Ser Gln
1                   5                           10                          15
Thr Leu Ser Leu Thr Cys Thr Val Ser Gly Phe Ser Ile Ser Thr Phe
                20                          25                          30
Gly Val His Trp Ile Arg Gln His Pro Gly Lys Gly Leu Glu Trp Ile
            35                          40                          45
Gly Val Ile Trp Arg Arg Gly Gly Thr Asp Tyr Asn Ala Ala Phe Met
        50                          55                          60
Ser Arg Val Thr Ile Ser Lys Asp Thr Ser Lys Asn Gln Val Ser Leu
65                          70                          75                          80
Lys Leu Ser Ser Val Thr Ala Ala Asp Thr Ala Val Tyr Tyr Cys Ala
                85                          90                          95
Arg Asp Gly Gly Phe Asp Tyr Trp Gly Gln Gly Thr Thr Val Thr Val
                100                         105                         110
Ser Ser Ala Ser Thr Lys Gly Pro Ser Val Phe Pro Leu Ala Pro Ser
            115                         120                         125
Ser Lys Ser Thr Ser Gly Gly Thr Ala Ala Leu Gly Cys Leu Val Lys
            130                         135                         140
Asp Tyr Phe Pro Glu Pro Val Thr Val Ser Trp Asn Ser Gly Ala Leu
145                         150                         155                         160
Thr Ser Gly Val His Thr Phe Pro Ala Val Leu Gln Ser Ser Gly Leu
            165                         170                         175
Tyr Ser Leu Ser Ser Val Val Thr Val Pro Ser Ser Ser Leu Gly Thr
            180                         185                         190
Gln Thr Tyr Ile Cys Asn Val Asn His Lys Pro Ser Asn Thr Lys Val
```

80

```
                195                      200                      205
        Asp Lys Lys Val Glu Pro Lys Ser Cys Asp Lys Thr His Thr Cys Pro
            210                 215                 220
        Pro Cys Pro Ala Pro Glu Leu Leu Gly Gly Pro Ser Val Phe Leu Phe
        225                 230                 235                 240
        Pro Pro Lys Pro Lys Asp Thr Leu Met Ile Ser Arg Thr Pro Glu Val
                        245                 250                 255
        Thr Cys Val Val Val Asp Val Ser His Glu Asp Pro Glu Val Lys Phe
                    260                 265                 270
        Asn Trp Tyr Val Asp Gly Val Glu Val His Asn Ala Lys Thr Lys Pro
                275                 280                 285
        Arg Glu Glu Gln Tyr Asn Ser Thr Tyr Arg Val Val Ser Val Leu Thr
            290                 295                 300
        Val Leu His Gln Asp Trp Leu Asn Gly Lys Glu Tyr Lys Cys Lys Val
        305                 310                 315                 320
        Ser Asn Lys Ala Leu Pro Ala Pro Ile Glu Lys Thr Ile Ser Lys Ala
                        325                 330                 335
        Lys Gly Gln Pro Arg Glu Pro Gln Val Tyr Thr Leu Pro Pro Ser Arg
                    340                 345                 350
        Asp Glu Leu Thr Lys Asn Gln Val Ser Leu Thr Cys Leu Val Lys Gly
                355                 360                 365
        Phe Tyr Pro Ser Asp Ile Ala Val Glu Trp Glu Ser Asn Gly Gln Pro
            370                 375                 380
        Glu Asn Asn Tyr Lys Thr Thr Pro Pro Val Leu Asp Ser Asp Gly Ser
        385                 390                 395                 400
        Phe Phe Leu Tyr Ser Lys Leu Thr Val Asp Lys Ser Arg Trp Gln Gln
                        405                 410                 415
        Gly Asn Val Phe Ser Cys Ser Val Met His Glu Ala Leu His Asn His
                    420                 425                 430
        Tyr Thr Gln Lys Ser Leu Ser Leu Ser Pro Gly Lys
                435                 440


        <210>   59
        <211>   444
        <212>   PRT
        <213>   Artificial Sequence

        <220>
        <223>   Hu164-87wl heavy chain


        <400>   59
        Glu Val Gln Leu Gln Glu Ser Gly Pro Gly Leu Val Lys Pro Ser Gln
        1               5                   10                  15
        Thr Leu Ser Leu Thr Cys Thr Val Ser Gly Phe Ser Ile Ser Thr Phe
                    20                  25                  30
        Gly Val His Trp Ile Arg Gln His Pro Gly Lys Gly Leu Glu Trp Leu
                35                  40                  45
        Gly Val Ile Trp Arg Arg Gly Gly Thr Asp Tyr Asn Ala Ala Phe Met
                50                  55                  60
        Ser Arg Leu Thr Ile Ser Lys Asp Thr Ser Lys Asn Gln Val Ser Phe
        65                  70                  75                  80
        Lys Leu Ser Ser Val Thr Ala Ala Asp Thr Ala Val Tyr Tyr Cys Ala
                        85                  90                  95
        Arg Asp Gly Gly Phe Asp Tyr Trp Gly Gln Gly Thr Thr Val Thr Val
                    100                 105                 110
        Ser Ser Ala Ser Thr Lys Gly Pro Ser Val Phe Pro Leu Ala Pro Ser
                115                 120                 125
        Ser Lys Ser Thr Ser Gly Gly Thr Ala Ala Leu Gly Cys Leu Val Lys
                130                 135                 140
        Asp Tyr Phe Pro Glu Pro Val Thr Val Ser Trp Asn Ser Gly Ala Leu
        145                 150                 155                 160
        Thr Ser Gly Val His Thr Phe Pro Ala Val Leu Gln Ser Ser Gly Leu
```

```
                           165                     170                     175
      Tyr Ser Leu Ser Ser Val Val Thr Val Pro Ser Ser Ser Leu Gly Thr
                  180                     185                     190
      Gln Thr Tyr Ile Cys Asn Val Asn His Lys Pro Ser Asn Thr Lys Val
                  195                     200                     205
      Asp Lys Lys Val Glu Pro Lys Ser Cys Asp Lys Thr His Thr Cys Pro
          210                     215                     220
      Pro Cys Pro Ala Pro Glu Leu Leu Gly Gly Pro Ser Val Phe Leu Phe
      225                     230                     235                     240
      Pro Pro Lys Pro Lys Asp Thr Leu Met Ile Ser Arg Thr Pro Glu Val
                      245                     250                     255
      Thr Cys Val Val Val Asp Val Ser His Glu Asp Pro Glu Val Lys Phe
                  260                     265                     270
      Asn Trp Tyr Val Asp Gly Val Glu Val His Asn Ala Lys Thr Lys Pro
                  275                     280                     285
      Arg Glu Glu Gln Tyr Asn Ser Thr Tyr Arg Val Val Ser Val Leu Thr
          290                     295                     300
      Val Leu His Gln Asp Trp Leu Asn Gly Lys Glu Tyr Lys Cys Lys Val
      305                     310                     315                     320
      Ser Asn Lys Ala Leu Pro Ala Pro Ile Glu Lys Thr Ile Ser Lys Ala
                  325                     330                     335
      Lys Gly Gln Pro Arg Glu Pro Gln Val Tyr Thr Leu Pro Pro Ser Arg
                  340                     345                     350
      Asp Glu Leu Thr Lys Asn Gln Val Ser Leu Thr Cys Leu Val Lys Gly
              355                     360                     365
      Phe Tyr Pro Ser Asp Ile Ala Val Glu Trp Glu Ser Asn Gly Gln Pro
          370                     375                     380
      Glu Asn Asn Tyr Lys Thr Thr Pro Pro Val Leu Asp Ser Asp Gly Ser
      385                     390                     395                     400
      Phe Phe Leu Tyr Ser Lys Leu Thr Val Asp Lys Ser Arg Trp Gln Gln
                  405                     410                     415
      Gly Asn Val Phe Ser Cys Ser Val Met His Glu Ala Leu His Asn His
                  420                     425                     430
      Tyr Thr Gln Lys Ser Leu Ser Leu Ser Pro Gly Lys
              435                     440
```

```
<210>   60
<211>   444
<212>   PRT
<213>   Artificial Sequence

<220>
<223>   Hu164-57yl heavy chain


<400>   60
Glu Val Thr Leu Lys Glu Ser Gly Pro Val Leu Val Lys Pro Thr Glu
1                   5                   10                  15
Thr Leu Thr Leu Thr Cys Thr Val Ser Gly Phe Ser Leu Ser Thr Phe
                20                  25                  30
Gly Val His Trp Ile Arg Gln Pro Pro Gly Lys Ala Leu Glu Trp Leu
            35                  40                  45
Ala Val Ile Trp Arg Arg Gly Gly Thr Asp Tyr Asn Ala Ala Phe Met
        50                  55                  60
Ser Arg Leu Thr Ile Ser Lys Asp Thr Ser Lys Ser Gln Val Val Leu
65                  70                  75                  80
Thr Met Thr Asn Met Asp Pro Val Asp Thr Ala Thr Tyr Tyr Cys Ala
                85                  90                  95
Arg Asp Gly Gly Phe Asp Tyr Trp Gly Gln Gly Thr Thr Val Thr Val
            100                 105                 110
Ser Ser Ala Ser Thr Lys Gly Pro Ser Val Phe Pro Leu Ala Pro Ser
        115                 120                 125
Ser Lys Ser Thr Ser Gly Gly Thr Ala Ala Leu Gly Cys Leu Val Lys
```

```
          130                           135                           140
      Asp Tyr Phe Pro Glu Pro Val Thr Val Ser Trp Asn Ser Gly Ala Leu
      145                   150                   155                   160
      Thr Ser Gly Val His Thr Phe Pro Ala Val Leu Gln Ser Ser Gly Leu
                        165                   170                   175
      Tyr Ser Leu Ser Ser Val Val Thr Val Pro Ser Ser Ser Leu Gly Thr
                    180                   185                   190
      Gln Thr Tyr Ile Cys Asn Val Asn His Lys Pro Ser Asn Thr Lys Val
                195                   200                   205
      Asp Lys Lys Val Glu Pro Lys Ser Cys Asp Lys Thr His Thr Cys Pro
          210                   215                   220
      Pro Cys Pro Ala Pro Glu Leu Leu Gly Gly Pro Ser Val Phe Leu Phe
      225                   230                   235                   240
      Pro Pro Lys Pro Lys Asp Thr Leu Tyr Ile Thr Arg Glu Pro Glu Val
                        245                   250                   255
      Thr Cys Val Val Val Asp Val Ser His Glu Asp Pro Glu Val Lys Phe
                    260                   265                   270
      Asn Trp Tyr Val Asp Gly Val Glu Val His Asn Ala Lys Thr Lys Pro
                275                   280                   285
      Arg Glu Glu Gln Tyr Asn Ser Thr Tyr Arg Val Val Ser Val Leu Thr
          290                   295                   300
      Val Leu His Gln Asp Trp Leu Asn Gly Lys Glu Tyr Lys Cys Lys Val
      305                   310                   315                   320
      Ser Asn Lys Ala Leu Pro Ala Pro Ile Glu Lys Thr Ile Ser Lys Ala
                        325                   330                   335
      Lys Gly Gln Pro Arg Glu Pro Gln Val Tyr Thr Leu Pro Pro Ser Arg
                    340                   345                   350
      Asp Glu Leu Thr Lys Asn Gln Val Ser Leu Thr Cys Leu Val Lys Gly
                355                   360                   365
      Phe Tyr Pro Ser Asp Ile Ala Val Glu Trp Glu Ser Asn Gly Gln Pro
          370                   375                   380
      Glu Asn Asn Tyr Lys Thr Thr Pro Pro Val Leu Asp Ser Asp Gly Ser
      385                   390                   395                   400
      Phe Phe Leu Tyr Ser Lys Leu Thr Val Asp Lys Ser Arg Trp Gln Gln
                        405                   410                   415
      Gly Asn Val Phe Ser Cys Ser Val Met His Glu Ala Leu His Asn His
                    420                   425                   430
      Tyr Thr Gln Lys Ser Leu Ser Leu Ser Pro Gly Lys
                435                   440

      <210>  61
      <211>  444
      <212>  PRT
      <213>  Artificial Sequence

      <220>
      <223>  Hu164-67yl heavy chain


      <400>  61
      Glu Val Thr Leu Lys Glu Ser Gly Pro Val Leu Val Lys Pro Thr Glu
      1                   5                   10                  15
      Thr Leu Thr Leu Thr Cys Thr Val Ser Gly Phe Ser Leu Ser Thr Phe
                        20                  25                  30
      Gly Val His Trp Ile Arg Gln Pro Pro Gly Lys Gly Leu Glu Trp Leu
                35                  40                  45
      Gly Val Ile Trp Arg Arg Gly Gly Thr Asp Tyr Asn Ala Ala Phe Met
            50                  55                  60
      Ser Arg Leu Thr Ile Ser Lys Asp Thr Ser Lys Ser Gln Val Val Phe
      65                  70                  75                  80
      Thr Met Thr Asn Met Asp Pro Val Asp Thr Ala Thr Tyr Tyr Cys Ala
                        85                  90                  95
      Arg Asp Gly Gly Phe Asp Tyr Trp Gly Gln Gly Thr Thr Val Thr Val
```

```
                    100                       105                      110
        Ser Ser Ala Ser Thr Lys Gly Pro Ser Val Phe Pro Leu Ala Pro Ser
                    115                       120                      125
        Ser Lys Ser Thr Ser Gly Gly Thr Ala Ala Leu Gly Cys Leu Val Lys
            130                       135                      140
        Asp Tyr Phe Pro Glu Pro Val Thr Val Ser Trp Asn Ser Gly Ala Leu
        145                       150                      155                  160
        Thr Ser Gly Val His Thr Phe Pro Ala Val Leu Gln Ser Ser Gly Leu
                        165                       170                      175
        Tyr Ser Leu Ser Ser Val Val Thr Val Pro Ser Ser Ser Leu Gly Thr
                    180                       185                      190
        Gln Thr Tyr Ile Cys Asn Val Asn His Lys Pro Ser Asn Thr Lys Val
                    195                       200                      205
        Asp Lys Lys Val Glu Pro Lys Ser Cys Asp Lys Thr His Thr Cys Pro
            210                       215                      220
        Pro Cys Pro Ala Pro Glu Leu Leu Gly Gly Pro Ser Val Phe Leu Phe
        225                       230                      235                  240
        Pro Pro Lys Pro Lys Asp Thr Leu Tyr Ile Thr Arg Glu Pro Glu Val
                        245                       250                      255
        Thr Cys Val Val Val Asp Val Ser His Glu Asp Pro Glu Val Lys Phe
                        260                       265                      270
        Asn Trp Tyr Val Asp Gly Val Glu Val His Asn Ala Lys Thr Lys Pro
                    275                       280                      285
        Arg Glu Glu Gln Tyr Asn Ser Thr Tyr Arg Val Val Ser Val Leu Thr
            290                       295                      300
        Val Leu His Gln Asp Trp Leu Asn Gly Lys Glu Tyr Lys Cys Lys Val
        305                       310                      315                  320
        Ser Asn Lys Ala Leu Pro Ala Pro Ile Glu Lys Thr Ile Ser Lys Ala
                        325                       330                      335
        Lys Gly Gln Pro Arg Glu Pro Gln Val Tyr Thr Leu Pro Pro Ser Arg
                    340                       345                      350
        Asp Glu Leu Thr Lys Asn Gln Val Ser Leu Thr Cys Leu Val Lys Gly
            355                       360                      365
        Phe Tyr Pro Ser Asp Ile Ala Val Glu Trp Glu Ser Asn Gly Gln Pro
            370                       375                      380
        Glu Asn Asn Tyr Lys Thr Thr Pro Pro Val Leu Asp Ser Asp Gly Ser
        385                       390                      395                  400
        Phe Phe Leu Tyr Ser Lys Leu Thr Val Asp Lys Ser Arg Trp Gln Gln
                        405                       410                      415
        Gly Asn Val Phe Ser Cys Ser Val Met His Glu Ala Leu His Asn His
                    420                       425                      430
        Tyr Thr Gln Lys Ser Leu Ser Leu Ser Pro Gly Lys
                    435                       440
```

```
<210>   62
<211>   444
<212>   PRT
<213>   Artificial Sequence

<220>
<223>   Hu164-77yl heavy chain


<400>   62
Glu Val Gln Leu Gln Glu Ser Gly Pro Gly Leu Val Lys Pro Ser Gln
1                   5                   10                  15
Thr Leu Ser Leu Thr Cys Thr Val Ser Gly Phe Ser Ile Ser Thr Phe
                20                  25                  30
Gly Val His Trp Ile Arg Gln His Pro Gly Lys Gly Leu Glu Trp Ile
        35                  40                  45
Gly Val Ile Trp Arg Arg Gly Gly Thr Asp Tyr Asn Ala Ala Phe Met
    50                  55                  60
Ser Arg Val Thr Ile Ser Lys Asp Thr Ser Lys Asn Gln Val Ser Leu
```

84

Lys Leu Ser Ser Val Thr Ala Ala Asp Thr Ala Val Tyr Tyr Cys Ala
65                  70                  75                  80

Arg Asp Gly Gly Phe Asp Tyr Trp Gly Gln Gly Thr Thr Val Thr Val
                85                  90                  95

Ser Ser Ala Ser Thr Lys Gly Pro Ser Val Phe Pro Leu Ala Pro Ser
            100                 105                 110

Ser Lys Ser Thr Ser Gly Gly Thr Ala Ala Leu Gly Cys Leu Val Lys
    115                 120                 125

Asp Tyr Phe Pro Glu Pro Val Thr Val Ser Trp Asn Ser Gly Ala Leu
130                 150                 155                 160

Thr Ser Gly Val His Thr Phe Pro Ala Val Leu Gln Ser Ser Gly Leu
            165                 170                 175

Tyr Ser Leu Ser Ser Val Val Thr Val Pro Ser Ser Ser Leu Gly Thr
        180                 185                 190

Gln Thr Tyr Ile Cys Asn Val Asn His Lys Pro Ser Asn Thr Lys Val
    195                 200                 205

Asp Lys Lys Val Glu Pro Lys Ser Cys Asp Lys Thr His Thr Cys Pro
    210                 215                 220

Pro Cys Pro Ala Pro Glu Leu Leu Gly Gly Pro Ser Val Phe Leu Phe
225                 230                 235                 240

Pro Pro Lys Pro Lys Asp Thr Leu Tyr Ile Thr Arg Glu Pro Glu Val
            245                 250                 255

Thr Cys Val Val Val Asp Val Ser His Glu Asp Pro Glu Val Lys Phe
            260                 265                 270

Asn Trp Tyr Val Asp Gly Val Glu Val His Asn Ala Lys Thr Lys Pro
        275                 280                 285

Arg Glu Glu Gln Tyr Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Ala Leu Pro Ala Pro Ile Glu Lys Thr Ile Ser Lys Ala
            325                 330                 335

Lys Gly Gln Pro Arg Glu Pro Gln Val Tyr Thr Leu Pro Pro Ser Arg
            340                 345                 350

Asp Glu Leu Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Lys Leu Thr Val Asp Lys Ser Arg Trp Gln Gln
            405                 410                 415

Gly Asn Val Phe Ser Cys Ser Val Met His Glu Ala Leu His Asn His
            420                 425                 430

Tyr Thr Gln Lys Ser Leu Ser Leu Ser Pro Gly Lys
    435                 440

<210> 63
<211> 444
<212> PRT
<213> Artificial Sequence

<220>
<223> Hu164-87yl heavy chain

<400> 63
Glu Val Gln Leu Gln Glu Ser Gly Pro Gly Leu Val Lys Pro Ser Gln
1               5               10              15

Thr Leu Ser Leu Thr Cys Thr Val Ser Gly Phe Ser Ile Ser Thr Phe
            20              25              30

Gly Val His Trp Ile Arg Gln His Pro Gly Lys Gly Leu Glu Trp Leu

```
              35                      40                      45
     Gly Val Ile Trp Arg Arg Gly Gly Thr Asp Tyr Asn Ala Ala Phe Met
         50                      55                      60
     Ser Arg Leu Thr Ile Ser Lys Asp Thr Ser Lys Asn Gln Val Ser Phe
     65                      70                      75                  80
     Lys Leu Ser Ser Val Thr Ala Ala Asp Thr Ala Val Tyr Tyr Cys Ala
                 85                      90                      95
     Arg Asp Gly Gly Phe Asp Tyr Trp Gly Gln Gly Thr Thr Val Thr Val
                100                     105                     110
     Ser Ser Ala Ser Thr Lys Gly Pro Ser Val Phe Pro Leu Ala Pro Ser
             115                     120                     125
     Ser Lys Ser Thr Ser Gly Gly Thr Ala Ala Leu Gly Cys Leu Val Lys
         130                     135                     140
     Asp Tyr Phe Pro Glu Pro Val Thr Val Ser Trp Asn Ser Gly Ala Leu
     145                     150                     155                 160
     Thr Ser Gly Val His Thr Phe Pro Ala Val Leu Gln Ser Ser Gly Leu
                 165                     170                     175
     Tyr Ser Leu Ser Ser Val Val Thr Val Pro Ser Ser Ser Leu Gly Thr
                 180                     185                     190
     Gln Thr Tyr Ile Cys Asn Val Asn His Lys Pro Ser Asn Thr Lys Val
             195                     200                     205
     Asp Lys Lys Val Glu Pro Lys Ser Cys Asp Lys Thr His Thr Cys Pro
         210                     215                     220
     Pro Cys Pro Ala Pro Glu Leu Leu Gly Gly Pro Ser Val Phe Leu Phe
     225                     230                     235                 240
     Pro Pro Lys Pro Lys Asp Thr Leu Tyr Ile Thr Arg Glu Pro Glu Val
                 245                     250                     255
     Thr Cys Val Val Val Asp Val Ser His Glu Asp Pro Glu Val Lys Phe
                 260                     265                     270
     Asn Trp Tyr Val Asp Gly Val Glu Val His Asn Ala Lys Thr Lys Pro
                 275                     280                     285
     Arg Glu Glu Gln Tyr Asn Ser Thr Tyr Arg Val Val Ser Val Leu Thr
         290                     295                     300
     Val Leu His Gln Asp Trp Leu Asn Gly Lys Glu Tyr Lys Cys Lys Val
     305                     310                     315                 320
     Ser Asn Lys Ala Leu Pro Ala Pro Ile Glu Lys Thr Ile Ser Lys Ala
                 325                     330                     335
     Lys Gly Gln Pro Arg Glu Pro Gln Val Tyr Thr Leu Pro Pro Ser Arg
                 340                     345                     350
     Asp Glu Leu Thr Lys Asn Gln Val Ser Leu Thr Cys Leu Val Lys Gly
             355                     360                     365
     Phe Tyr Pro Ser Asp Ile Ala Val Glu Trp Glu Ser Asn Gly Gln Pro
         370                     375                     380
     Glu Asn Asn Tyr Lys Thr Thr Pro Pro Val Leu Asp Ser Asp Gly Ser
     385                     390                     395                 400
     Phe Phe Leu Tyr Ser Lys Leu Thr Val Asp Lys Ser Arg Trp Gln Gln
                 405                     410                     415
     Gly Asn Val Phe Ser Cys Ser Val Met His Glu Ala Leu His Asn His
                 420                     425                     430
     Tyr Thr Gln Lys Ser Leu Ser Leu Ser Pro Gly Lys
             435                     440
```

```
<210>  64
<211>  214
<212>  PRT
<213>  Artificial Sequence

<220>
<223>  Hu164-57wl light chain


<400>  64
Glu Thr Val Val Thr Gln Glu Pro Ser Leu Thr Val Ser Pro Gly Gly
```

```
1                   5                           10                          15
Thr Val Thr Leu Thr Cys Arg Ser Ser Ile Gly Ala Val Thr Thr Ser
            20                          25                          30
Asn Tyr Ala Asn Trp Val Gln Gln Lys Pro Gly Gln Ala Phe Arg Gly
            35                          40                          45
Leu Ile Gly Gly Thr Ser Asn Arg Ala Pro Trp Thr Pro Ala Arg Phe
            50                          55                          60
Ser Gly Ser Leu Leu Gly Gly Lys Ala Ala Leu Thr Leu Ser Gly Val
65                          70                          75                          80
Gln Pro Glu Asp Glu Ala Glu Tyr Tyr Cys Ala Leu Trp Tyr Ser Thr
                        85                          90                          95
His Tyr Val Phe Gly Gly Gly Thr Lys Leu Thr Val Leu Gly Gln Pro
                100                         105                         110
Lys Ala Asn Pro Thr Val Thr Leu Phe Pro Pro Ser Ser Glu Glu Leu
                115                         120                         125
Gln Ala Asn Lys Ala Thr Leu Val Cys Leu Ile Ser Asp Phe Tyr Pro
                130                         135                         140
Gly Ala Val Thr Val Ala Trp Lys Ala Asp Gly Ser Pro Val Lys Ala
145                         150                         155                         160
Gly Val Glu Thr Thr Lys Pro Ser Lys Gln Ser Asn Asn Lys Tyr Ala
                        165                         170                         175
Ala Ser Ser Tyr Leu Ser Leu Thr Pro Glu Gln Trp Lys Ser His Arg
                180                         185                         190
Ser Tyr Ser Cys Gln Val Thr His Glu Gly Ser Thr Val Glu Lys Thr
                195                         200                         205
Val Ala Pro Thr Glu Cys
                210


<210>   65
<211>   214
<212>   PRT
<213>   Artificial Sequence

<220>
<223>   Hu164-58wl light chain


<400>   65
Glu Thr Val Val Thr Gln Glu Pro Ser Phe Ser Val Ser Pro Gly Gly
1                   5                           10                          15
Thr Val Thr Leu Thr Cys Arg Ser Ser Ile Gly Ala Val Thr Thr Ser
            20                          25                          30
Asn Tyr Ala Asn Trp Val Gln Gln Thr Pro Gly Gln Ala Phe Arg Gly
            35                          40                          45
Leu Ile Gly Gly Thr Ser Asn Arg Ala Pro Gly Val Pro Asp Arg Phe
            50                          55                          60
Ser Gly Ser Ile Leu Gly Asn Lys Ala Ala Leu Thr Ile Thr Gly Ala
65                          70                          75                          80
Gln Ala Asp Asp Glu Ser Asp Tyr Tyr Cys Ala Leu Trp Tyr Ser Thr
                        85                          90                          95
His Tyr Val Phe Gly Gly Gly Thr Lys Leu Thr Val Leu Gly Gln Pro
                100                         105                         110
Lys Ala Asn Pro Thr Val Thr Leu Phe Pro Pro Ser Ser Glu Glu Leu
                115                         120                         125
Gln Ala Asn Lys Ala Thr Leu Val Cys Leu Ile Ser Asp Phe Tyr Pro
                130                         135                         140
Gly Ala Val Thr Val Ala Trp Lys Ala Asp Gly Ser Pro Val Lys Ala
145                         150                         155                         160
Gly Val Glu Thr Thr Lys Pro Ser Lys Gln Ser Asn Asn Lys Tyr Ala
                        165                         170                         175
Ala Ser Ser Tyr Leu Ser Leu Thr Pro Glu Gln Trp Lys Ser His Arg
                180                         185                         190
Ser Tyr Ser Cys Gln Val Thr His Glu Gly Ser Thr Val Glu Lys Thr
```

```
                195                      200                      205
       Val Ala Pro Thr Glu Cys
           210


       <210>  66
       <211>  214
       <212>  PRT
       <213>  Artificial Sequence

       <220>
       <223>  Hu164-59wl light chain


       <400>  66
       Glu Ser Val Val Thr Gln Pro Pro Ser Val Ser Gly Ala Pro Gly Gln
       1                   5                   10                  15
       Arg Val Thr Ile Ser Cys Arg Ser Ser Ile Gly Ala Val Thr Thr Ser
                   20                  25                  30
       Asn Tyr Ala Asn Trp Val Gln Gln Leu Pro Gly Thr Ala Phe Lys Gly
               35                  40                  45
       Leu Ile Gly Gly Thr Ser Asn Arg Ala Pro Gly Val Pro Asp Arg Phe
           50                  55                  60
       Ser Gly Ser Lys Ser Gly Thr Ser Ala Ser Leu Ala Ile Thr Gly Leu
       65                  70                  75                  80
       Gln Ala Glu Asp Glu Ala Asp Tyr Tyr Cys Ala Leu Trp Tyr Ser Thr
                       85                  90                  95
       His Tyr Val Phe Gly Gly Gly Thr Lys Leu Thr Val Leu Gly Gln Pro
               100                 105                 110
       Lys Ala Asn Pro Thr Val Thr Leu Phe Pro Pro Ser Ser Glu Glu Leu
               115                 120                 125
       Gln Ala Asn Lys Ala Thr Leu Val Cys Leu Ile Ser Asp Phe Tyr Pro
           130                 135                 140
       Gly Ala Val Thr Val Ala Trp Lys Ala Asp Gly Ser Pro Val Lys Ala
       145                 150                 155                 160
       Gly Val Glu Thr Thr Lys Pro Ser Lys Gln Ser Asn Asn Lys Tyr Ala
                   165                 170                 175
       Ala Ser Ser Tyr Leu Ser Leu Thr Pro Glu Gln Trp Lys Ser His Arg
                   180                 185                 190
       Ser Tyr Ser Cys Gln Val Thr His Glu Gly Ser Thr Val Glu Lys Thr
                   195                 200                 205
       Val Ala Pro Thr Glu Cys
           210


       <210>  67
       <211>  450
       <212>  PRT
       <213>  Artificial Sequence

       <220>
       <223>  mAb1 heavy chain


       <400>  67
       Glu Gly Gln Leu Val Gln Ser Gly Gly Gly Leu Val His Pro Gly Gly
       1                   5                   10                  15
       Ser Leu Arg Leu Ser Cys Ala Gly Ser Gly Phe Thr Phe Ser Ser Tyr
                   20                  25                  30
       Gly Met His Trp Val Arg Gln Ala Pro Gly Lys Gly Leu Glu Trp Val
               35                  40                  45
       Ser Gly Ile Gly Thr Gly Gly Gly Thr Tyr Ser Thr Asp Ser Val Lys
           50                  55                  60
       Gly Arg Phe Thr Ile Ser Arg Asp Asn Ala Lys Asn Ser Leu Tyr Leu
       65                  70                  75                  80
```

```
Gln Met Asn Ser Leu Arg Ala Glu Asp Met Ala Val Tyr Tyr Cys Ala
            85                  90                  95
Arg Gly Asp Tyr Tyr Gly Ser Gly Ser Phe Phe Asp Cys Trp Gly Gln
            100             105                 110
Gly Thr Leu Val Thr Val Ser Ser Ala Ser Thr Lys Gly Pro Ser Val
            115             120                 125
Phe Pro Leu Ala Pro Ser Ser Lys Ser Thr Ser Gly Gly Thr Ala Ala
    130             135                 140
Leu Gly Cys Leu Val Lys Asp Tyr Phe Pro Glu Pro Val Thr Val Ser
145             150                 155                     160
Trp Asn Ser Gly Ala Leu Thr Ser Gly Val His Thr Phe Pro Ala Val
            165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
            195                 200                 205
Pro Ser Asn Thr Lys Val Asp Lys Arg Val Glu Pro Lys Ser Cys Asp
    210                 215                 220
Lys Thr His Thr Cys Pro Pro Cys Pro Ala Pro Glu Leu Leu Gly Gly
225                 230                 235                     240
Pro Ser Val Phe Leu Phe Pro Pro Lys Pro Lys Asp Thr Leu Met Ile
            245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
            275                 280                 285
Asn Ala Lys Thr Lys Pro Arg Glu Glu Gln Tyr Asn Ser Thr Tyr Arg
            290                 295                 300
Val Val Ser Val Leu Thr Val Leu His Gln Asp Trp Leu Asn Gly Lys
305                 310                 315                     320
Glu Tyr Lys Cys Lys Val Ser Asn Lys Ala Leu Pro Ala Pro Ile Glu
            325                 330                 335
Lys Thr Ile Ser Lys Ala Lys Gly Gln Pro Arg Glu Pro Gln Val Tyr
            340                 345                 350
Thr Leu Pro Pro Ser Arg Glu Glu Met Thr Lys Asn Gln Val Ser Leu
            355                 360                 365
Thr Cys Leu Val Lys Gly Phe Tyr Pro Ser Asp Ile Ala Val Glu Trp
            370                 375                 380
Glu Ser Asn Gly Gln Pro Glu Asn Asn Tyr Lys Thr Thr Pro Pro Val
385                 390                 395                     400
Leu Asp Ser Asp Gly Ser Phe Phe Leu Tyr Ser Lys Leu Thr Val Asp
            405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
            435                 440                 445
Gly Lys
    450
```

<210> 68
<211> 214
<212> PRT
<213> Artificial Sequence

<220>
<223> mAb1 light chain


<400> 68
```
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Ser Ser Trp
            20                  25                  30
```

```
Leu Ala Trp Tyr Gln Gln Lys Pro Glu Lys Ala Pro Lys Ser Leu Ile
        35              40              45
Tyr Ala Ala Ser Ser Leu Gln Ser Gly Val Pro Ser Arg Phe Ser Gly
    50              55              60
Ser Gly Ser Gly Thr Asp Phe Thr Leu Thr Ile Ser Ser Leu Gln Pro
65              70              75              80
Glu Asp Phe Ala Thr Tyr Tyr Cys Gln Gln Tyr Asn Ser Tyr Pro Pro
                85              90              95
Thr Phe Gly Gln Gly Thr Lys Leu Glu Ile Lys Arg Thr Val Ala Ala
            100             105             110
Pro Ser Val Phe Ile Phe Pro Pro Ser Asp Glu Gln Leu Lys Ser Gly
        115             120             125
Thr Ala Ser Val Val Cys Leu Leu Asn Asn Phe Tyr Pro Arg Glu Ala
    130             135             140
Lys Val Gln Trp Lys Val Asp Asn Ala Leu Gln Ser Gly Asn Ser Gln
145             150             155             160
Glu Ser Val Thr Glu Gln Asp Ser Lys Asp Ser Thr Tyr Ser Leu Ser
                165             170             175
Ser Thr Leu Thr Leu Ser Lys Ala Asp Tyr Glu Lys His Lys Val Tyr
            180             185             190
Ala Cys Glu Val Thr His Gln Gly Leu Ser Ser Pro Val Thr Lys Ser
            195             200             205
Phe Asn Arg Gly Glu Cys
            210


<210>   69
<211>   122
<212>   PRT
<213>   Artificial Sequence

<220>
<223>   mab95 VH


<400>   69
Glu Val Leu Leu Gln Gln Ser Gly Pro Val Leu Val Lys Pro Gly Pro
1               5               10              15
Ser Val Lys Ile Ser Cys Lys Ala Ser Gly Phe Thr Phe Thr Asn Tyr
            20              25              30
Asp Ile His Trp Val Lys Gln Ser His Gly Lys Ser Leu Glu Trp Ile
        35              40              45
Gly Leu Val Tyr Pro Tyr Asn Gly Gly Thr Ala Tyr Asn Gln Lys Phe
    50              55              60
Lys Asp Lys Ala Thr Leu Thr Phe Asp Thr Ser Ser Ser Thr Ala Tyr
65              70              75              80
Met Glu Leu Asn Ser Leu Thr Ser Glu Asp Ser Ala Val Tyr Tyr Cys
                85              90              95
Ala Arg Trp Gly Leu Ile Pro Gly Thr Ser Tyr Phe Asp Val Trp
            100             105             110
Gly Thr Gly Thr Thr Val Thr Val Ser Ser
            115             120


<210>   70
<211>   106
<212>   PRT
<213>   Artificial Sequence

<220>
<223>   mab95 VL


<400>   70
Gln Ile Val Leu Ser Gln Ser Pro Pro Ile Leu Ser Ala Ser Pro Gly
```

90

```
1                   5                   10                  15
Glu Lys Val Thr Met Thr Cys Arg Ala Ser Ser Ser Val Ser Tyr Ile
            20                  25                  30
His Trp Tyr Gln Gln Lys Pro Arg Ser Ser Pro Lys Pro Trp Ile Tyr
        35                  40                  45
Ala Thr Ser Asn Leu Ala Ser Gly Val Pro Ala Arg Phe Ser Gly Ser
        50                  55                  60
Gly Ser Gly Thr Ser Tyr Ser Leu Thr Ile Ser Arg Val Glu Ala Glu
65                  70                  75                  80
Asp Ala Ala Thr Tyr Tyr Cys Gln Gln Trp Asn Ser Asn Pro Trp Thr
                85                  90                  95
Phe Gly Gly Gly Thr Arg Leu Glu Ile Lys
            100                 105
```

<210> 71
<211> 5
<212> PRT
<213> Artificial Sequence

<220>
<223> mab95 HCDR1


<400> 71
Asn Tyr Asp Ile His
1               5

<210> 72
<211> 17
<212> PRT
<213> Artificial Sequence

<220>
<223> mab95 HCDR2


<400> 72
Leu Val Tyr Pro Tyr Asn Gly Gly Thr Ala Tyr Asn Gln Lys Phe Lys
1               5                   10                  15
Asp


<210> 73
<211> 13
<212> PRT
<213> Artificial Sequence

<220>
<223> mab95 HCDR3


<400> 73
Trp Gly Leu Ile Pro Gly Thr Thr Ser Tyr Phe Asp Val
1               5                   10

<210> 74
<211> 10
<212> PRT
<213> Artificial Sequence

<220>
<223> mab95 LCDR1

```
<400>  74
Arg Ala Ser Ser Ser Val Ser Tyr Ile His
1               5                   10


<210>  75
<211>  7
<212>  PRT
<213>  Artificial Sequence


<220>
<223>  mab95 LCDR2


<400>  75
Ala Thr Ser Asn Leu Ala Ser
1               5


<210>  76
<211>  9
<212>  PRT
<213>  Artificial Sequence


<220>
<223>  mab95 LCDR3


<400>  76
Gln Gln Trp Asn Ser Asn Pro Trp Thr
1               5


<210>  77
<211>  106
<212>  PRT
<213>  Artificial Sequence


<220>
<223>  Hu95-VL1


<400>  77
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Ser Ser Val Ser Tyr Ile
            20                  25                  30
His Trp Tyr Gln Gln Lys Pro Gly Gln Ala Pro Arg Pro Trp Ile Tyr
            35                  40                  45
Ala Thr Ser Asn Leu Ala Ser Gly Ile Pro Ala Arg Phe Ser Gly Ser
        50                  55                  60
Gly Ser Gly Thr Asp Tyr Thr Leu Thr Ile Ser Arg Leu Glu Pro Glu
65                  70                  75                  80
Asp Phe Ala Val Tyr Tyr Cys Gln Gln Trp Asn Ser Asn Pro Trp Thr
                85                  90                  95
Phe Gly Gly Gly Thr Lys Val Glu Ile Lys
            100                 105


<210>  78
<211>  106
<212>  PRT
<213>  Artificial Sequence


<220>
<223>  Hu95-VL2
```

```
<400>    78
Glu Ile Val Leu Thr Gln Ser Pro Ala Thr Leu Ser Leu Ser Pro Gly
1                   5                   10                  15
Glu Arg Ala Thr Leu Ser Cys Arg Ala Ser Ser Ser Val Ser Tyr Ile
            20                  25                  30
His Trp Tyr Gln Gln Lys Pro Gly Gln Ser Pro Arg Pro Trp Ile Tyr
        35                  40                  45
Ala Thr Ser Asn Leu Ala Ser Gly Val Pro Ala Arg Phe Ser Gly Ser
        50                  55                  60
Gly Ser Gly Thr Ser Tyr Thr Leu Thr Ile Ser Arg Leu Glu Pro Glu
65                  70                  75                  80
Asp Phe Ala Val Tyr Tyr Cys Gln Gln Trp Asn Ser Asn Pro Trp Thr
                85                  90                  95
Phe Gly Gly Gly Thr Lys Val Glu Ile Lys
            100                 105


<210>    79
<211>    105
<212>    PRT
<213>    Artificial Sequence

<220>
<223>    Hu95-VL3


<400>    79
Glu Ser Val Leu Thr Gln Pro Pro Ser Val Ser Gly Ala Pro Gly Gln
1                   5                   10                  15
Arg Val Thr Ile Ser Cys Arg Ala Ser Ser Ser Val Ser Tyr Ile His
            20                  25                  30
Trp Tyr Gln Gln Leu Pro Gly Thr Ala Pro Lys Pro Trp Ile Tyr Ala
        35                  40                  45
Thr Ser Asn Leu Ala Ser Gly Val Pro Asp Arg Phe Ser Gly Ser Lys
        50                  55                  60
Ser Gly Thr Ser Tyr Ser Leu Ala Ile Thr Gly Leu Gln Ala Glu Asp
65                  70                  75                  80
Glu Ala Asp Tyr Tyr Cys Gln Gln Trp Asn Ser Asn Pro Trp Thr Phe
                85                  90                  95
Gly Gly Gly Thr Lys Val Glu Ile Lys
            100                 105


<210>    80
<211>    105
<212>    PRT
<213>    Artificial Sequence

<220>
<223>    Hu95-VL4


<400>    80
Glu Ile Val Leu Thr Gln Pro Pro Ser Val Ser Gly Ala Pro Gly Gln
1                   5                   10                  15
Arg Val Thr Ile Ser Cys Arg Ala Ser Ser Ser Val Ser Tyr Ile His
            20                  25                  30
Trp Tyr Gln Gln Leu Pro Gly Thr Ser Pro Lys Pro Trp Ile Tyr Ala
        35                  40                  45
Thr Ser Asn Leu Ala Ser Gly Val Pro Asp Arg Phe Ser Gly Ser Lys
        50                  55                  60
Ser Gly Thr Ser Tyr Ser Leu Ala Ile Thr Gly Leu Gln Ala Glu Asp
65                  70                  75                  80
```

```
Glu Ala Asp Tyr Tyr Cys Gln Gln Trp Asn Ser Asn Pro Trp Thr Phe
                85                  90                  95
Gly Gly Gly Thr Lys Val Glu Ile Lys
            100                 105
```

```
<210>   81
<211>   122
<212>   PRT
<213>   Artificial Sequence

<220>
<223>   Hu95-VH1


<400>   81
Glu Val Gln Leu Val Gln Ser Gly Ala Glu Val Lys Lys Pro Gly Ala
1               5                   10                  15
Ser Val Lys Val Ser Cys Lys Ala Ser Gly Phe Thr Phe Thr Asn Tyr
            20                  25                  30
Asp Ile His Trp Val Arg Gln Ala Pro Gly Gln Arg Leu Glu Trp Met
        35                  40                  45
Gly Leu Val Tyr Pro Tyr Asn Gly Gly Thr Ala Tyr Asn Gln Lys Phe
        50                  55                  60
Lys Asp Arg Val Thr Leu Thr Phe Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Trp Gly Leu Ile Pro Gly Thr Ser Tyr Phe Asp Val Trp
            100                 105                 110
Gly Gln Gly Thr Thr Val Thr Val Ser Ser
            115                 120


<210>   82
<211>   122
<212>   PRT
<213>   Artificial Sequence

<220>
<223>   Hu95-VH2


<400>   82
Glu Val Gln Leu Val Gln Ser Gly Ala Glu Val Lys Lys Pro Gly Ala
1               5                   10                  15
Ser Val Lys Val Ser Cys Lys Ala Ser Gly Phe Thr Phe Thr Asn Tyr
            20                  25                  30
Asp Ile His Trp Val Arg Gln Ala Pro Gly Gln Arg Leu Glu Trp Ile
        35                  40                  45
Gly Leu Val Tyr Pro Tyr Asn Gly Gly Thr Ala Tyr Asn Gln Lys Phe
        50                  55                  60
Lys Asp Arg Ala Thr Leu Thr Phe Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Trp Gly Leu Ile Pro Gly Thr Ser Tyr Phe Asp Val Trp
            100                 105                 110
Gly Gln Gly Thr Thr Val Thr Val Ser Ser
            115                 120


<210>   83
<211>   122
<212>   PRT
<213>   Artificial Sequence
```

94

```
<220>
<223>   Hu95-VH3


<400>   83
Glu Val Gln Leu Val Gln Ser Gly Ala Glu Val Lys Lys Pro Gly Ala
1               5                   10                  15
Ser Val Lys Val Ser Cys Lys Ala Ser Gly Phe Thr Phe Thr Asn Tyr
            20                  25                  30
Asp Ile His Trp Val Lys Gln Ala Pro Gly Gln Arg Leu Glu Trp Met
        35                  40                  45
Gly Leu Val Tyr Pro Tyr Asn Gly Gly Thr Ala Tyr Asn Gln Lys Phe
    50                  55                  60
Lys Asp Lys Val Thr Leu Thr Phe Asp Thr Ser Ala Ser Thr Ala Tyr
65                  70                  75                  80
Met Glu Leu Ser Ser Leu Arg Ser Glu Asp Thr Ala Val Tyr Tyr Cys
            85                  90                  95
Ala Arg Trp Gly Leu Ile Pro Gly Thr Thr Ser Tyr Phe Asp Val Trp
            100                 105                 110
Gly Gln Gly Thr Thr Val Thr Val Ser Ser
            115                 120


<210>   84
<211>   122
<212>   PRT
<213>   Artificial Sequence


<220>
<223>   Hu95-VH4


<400>   84
Glu Val Gln Leu Val Gln Ser Gly Ala Glu Val Lys Lys Pro Gly Ala
1               5                   10                  15
Ser Val Lys Val Ser Cys Lys Ala Ser Gly Phe Thr Phe Thr Asn Tyr
            20                  25                  30
Asp Ile His Trp Val Lys Gln Ala Pro Gly Gln Arg Leu Glu Trp Ile
        35                  40                  45
Gly Leu Val Tyr Pro Tyr Asn Gly Gly Thr Ala Tyr Asn Gln Lys Phe
    50                  55                  60
Lys Asp Lys Ala Thr Leu Thr Phe Asp Thr Ser Ala Ser Thr Ala Tyr
65                  70                  75                  80
Met Glu Leu Ser Ser Leu Arg Ser Glu Asp Thr Ala Val Tyr Tyr Cys
            85                  90                  95
Ala Arg Trp Gly Leu Ile Pro Gly Thr Thr Ser Tyr Phe Asp Val Trp
            100                 105                 110
Gly Gln Gly Thr Thr Val Thr Val Ser Ser
            115                 120

<210>   85
<211>   122
<212>   PRT
<213>   Artificial Sequence


<220>
<223>   Hu95-VH5


<400>   85
Glu Val Gln Leu Val Gln Ser Gly Ala Glu Val Lys Lys Pro Gly Ala
1               5                   10                  15
Ser Val Lys Val Ser Cys Arg Ala Ser Gly Phe Thr Phe Thr Asn Tyr
```

```
                    20                      25                      30
        Ala Ile His Trp Val Lys Gln Ala Pro Gly Gln Arg Leu Glu Trp Met
                    35                      40                      45
        Gly Leu Val Tyr Pro Tyr Thr Gly Gly Thr Ala Tyr Asn Gln Lys Phe
                50                      55                      60
        Lys Asp Lys Val Thr Leu Thr Phe Asp Thr Ser Ala Ser Thr Ala Tyr
        65                      70                      75                      80
        Met Glu Leu Ser Ser Leu Arg Ser Glu Asp Thr Ala Val Tyr Tyr Cys
                        85                      90                      95
        Ala Arg Trp Gly Met Ile Pro Gly Thr Asn Ser Tyr Phe Asp Val Trp
                    100                     105                     110
        Gly Gln Gly Thr Thr Val Thr Val Ser Ser
                    115                     120


<210>   86
<211>   452
<212>   PRT
<213>   Artificial Sequence

<220>
<223>   Ch95 heavy chain


<400>   86
Glu Val Leu Leu Gln Gln Ser Gly Pro Val Leu Val Lys Pro Gly Pro
1                   5                   10                      15
Ser Val Lys Ile Ser Cys Lys Ala Ser Gly Phe Thr Phe Thr Asn Tyr
            20                      25                      30
Asp Ile His Trp Val Lys Gln Ser His Gly Lys Ser Leu Glu Trp Ile
        35                      40                      45
Gly Leu Val Tyr Pro Tyr Asn Gly Gly Thr Ala Tyr Asn Gln Lys Phe
        50                      55                      60
Lys Asp Lys Ala Thr Leu Thr Phe Asp Thr Ser Ser Ser Thr Ala Tyr
65                      70                      75                      80
Met Glu Leu Asn Ser Leu Thr Ser Glu Asp Ser Ala Val Tyr Tyr Cys
                85                      90                      95
Ala Arg Trp Gly Leu Ile Pro Gly Thr Thr Ser Tyr Phe Asp Val Trp
            100                     105                     110
Gly Thr Gly Thr Thr Val Thr Val Ser Ser Ala Ser Thr Lys Gly Pro
            115                     120                     125
Ser Val Phe Pro Leu Ala Pro Ser Ser Lys Ser Thr Ser Gly Gly Thr
            130                     135                     140
Ala Ala Leu Gly Cys Leu Val Lys Asp Tyr Phe Pro Glu Pro Val Thr
145                     150                     155                     160
Val Ser Trp Asn Ser Gly Ala Leu Thr Ser Gly Val His Thr Phe Pro
                165                     170                     175
Ala Val Leu Gln Ser Ser Gly Leu Tyr Ser Leu Ser Ser Val Val Thr
            180                     185                     190
Val Pro Ser Ser Ser Leu Gly Thr Gln Thr Tyr Ile Cys Asn Val Asn
            195                     200                     205
His Lys Pro Ser Asn Thr Lys Val Asp Lys Lys Val Glu Pro Lys Ser
            210                     215                     220
Cys Asp Lys Thr His Thr Cys Pro Pro Cys Pro Ala Pro Glu Leu Leu
225                     230                     235                     240
Gly Gly Pro Ser Val Phe Leu Phe Pro Pro Lys Pro Lys Asp Thr Leu
                245                     250                     255
Met Ile Ser Arg Thr Pro Glu Val Thr Cys Val Val Val Asp Val Ser
            260                     265                     270
His Glu Asp Pro Glu Val Lys Phe Asn Trp Tyr Val Asp Gly Val Glu
            275                     280                     285
Val His Asn Ala Lys Thr Lys Pro Arg Glu Glu Gln Tyr Asn Ser Thr
            290                     295                     300
Tyr Arg Val Val Ser Val Leu Thr Val Leu His Gln Asp Trp Leu Asn
```

```
                              305                            310                            315                            320
Gly Lys Glu Tyr Lys Cys Lys Val Ser Asn Lys Ala Leu Pro Ala Pro
                    325                            330                            335
Ile Glu Lys Thr Ile Ser Lys Ala Lys Gly Gln Pro Arg Glu Pro Gln
                    340                            345                            350
Val Tyr Thr Leu Pro Pro Ser Arg Asp Glu Leu Thr Lys Asn Gln Val
                    355                            360                            365
Ser Leu Thr Cys Leu Val Lys Gly Phe Tyr Pro Ser Asp Ile Ala Val
            370                            375                            380
Glu Trp Glu Ser Asn Gly Gln Pro Glu Asn Asn Tyr Lys Thr Thr Pro
385                            390                            395                            400
Pro Val Leu Asp Ser Asp Gly Ser Phe Phe Leu Tyr Ser Lys Leu Thr
                    405                            410                            415
Val Asp Lys Ser Arg Trp Gln Gln Gly Asn Val Phe Ser Cys Ser Val
                    420                            425                            430
Met His Glu Ala Leu His Asn His Tyr Thr Gln Lys Ser Leu Ser Leu
            435                            440                            445
Ser Pro Gly Lys
        450
```

```
<210>  87
<211>  213
<212>  PRT
<213>  Artificial Sequence

<220>
<223>  Ch95 light chain


<400>  87
Gln Ile Val Leu Ser Gln Ser Pro Pro Ile Leu Ser Ala Ser Pro Gly
1                   5                           10                          15
Glu Lys Val Thr Met Thr Cys Arg Ala Ser Ser Ser Val Ser Tyr Ile
                20                          25                          30
His Trp Tyr Gln Gln Lys Pro Arg Ser Ser Pro Lys Pro Trp Ile Tyr
            35                          40                          45
Ala Thr Ser Asn Leu Ala Ser Gly Val Pro Ala Arg Phe Ser Gly Ser
        50                          55                          60
Gly Ser Gly Thr Ser Tyr Ser Leu Thr Ile Ser Arg Val Glu Ala Glu
65                          70                          75                          80
Asp Ala Ala Thr Tyr Tyr Cys Gln Gln Trp Asn Ser Asn Pro Trp Thr
                    85                          90                          95
Phe Gly Gly Gly Thr Arg Leu Glu Ile Lys Arg Thr Val Ala Ala Pro
            100                         105                         110
Ser Val Phe Ile Phe Pro Pro Ser Asp Glu Gln Leu Lys Ser Gly Thr
            115                         120                         125
Ala Ser Val Val Cys Leu Leu Asn Asn Phe Tyr Pro Arg Glu Ala Lys
        130                         135                         140
Val Gln Trp Lys Val Asp Asn Ala Leu Gln Ser Gly Asn Ser Gln Glu
145                         150                         155                         160
Ser Val Thr Glu Gln Asp Ser Lys Asp Ser Thr Tyr Ser Leu Ser Ser
                    165                         170                         175
Thr Leu Thr Leu Ser Lys Ala Asp Tyr Glu Lys His Lys Val Tyr Ala
            180                         185                         190
Cys Glu Val Thr His Gln Gly Leu Ser Ser Pro Val Thr Lys Ser Phe
            195                         200                         205
Asn Arg Gly Glu Cys
        210

<210>  88
<211>  452
<212>  PRT
<213>  Artificial Sequence
```

<220>
<223> Hu95-11wk heavy chain


<400> 88
Glu Val Gln Leu Val Gln Ser Gly Ala Glu Val Lys Lys Pro Gly Ala
1               5                   10                  15
Ser Val Lys Val Ser Cys Lys Ala Ser Gly Phe Thr Phe Thr Asn Tyr
            20                  25                  30
Asp Ile His Trp Val Arg Gln Ala Pro Gly Gln Arg Leu Glu Trp Met
        35                  40                  45
Gly Leu Val Tyr Pro Tyr Asn Gly Gly Thr Ala Tyr Asn Gln Lys Phe
    50                  55                  60
Lys Asp Arg Val Thr Leu Thr Phe Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Trp Gly Leu Ile Pro Gly Thr Thr Ser Tyr Phe Asp Val Trp
            100                 105                 110
Gly Gln Gly Thr Thr Val Thr Val Ser Ser Ala Ser Thr Lys Gly Pro
        115                 120                 125
Ser Val Phe Pro Leu Ala Pro Ser Ser Lys Ser Thr Ser Gly Gly Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Lys Val Glu Pro Lys Ser
    210                 215                 220
Cys Asp Lys Thr His Thr Cys Pro Pro Cys Pro Ala Pro Glu Leu Leu
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
                260                 265                 270
His Glu Asp Pro Glu Val Lys Phe Asn Trp Tyr Val Asp Gly Val Glu
            275                 280                 285
Val His Asn Ala Lys Thr Lys Pro Arg Glu Glu Gln Tyr Asn Ser Thr
        290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
            325                 330                 335
Ile Glu Lys Thr Ile Ser Lys Ala Lys Gly Gln Pro Arg Glu Pro Gln
        340                 345                 350
Val Tyr Thr Leu Pro Pro Ser Arg Asp Glu Leu Thr Lys Asn Gln Val
    355                 360                 365
Ser Leu Thr Cys Leu Val Lys Gly Phe Tyr Pro Ser Asp Ile Ala Val
370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
                405                 410                 415
Val Asp Lys Ser Arg Trp Gln Gln Gly Asn Val Phe Ser Cys Ser Val
            420                 425                 430
Met His Glu Ala Leu His Asn His Tyr Thr Gln Lys Ser Leu Ser Leu
        435                 440                 445
Ser Pro Gly Lys

450

```
<210>  89
<211>  452
<212>  PRT
<213>  Artificial Sequence

<220>
<223>  Hu95-21wk  heavy chain


<400>  89
Glu Val Gln Leu Val Gln Ser Gly Ala Glu Val Lys Lys Pro Gly Ala
1               5                   10                  15
Ser Val Lys Val Ser Cys Lys Ala Ser Gly Phe Thr Phe Thr Asn Tyr
            20                  25                  30
Asp Ile His Trp Val Arg Gln Ala Pro Gly Gln Arg Leu Glu Trp Ile
        35                  40                  45
Gly Leu Val Tyr Pro Tyr Asn Gly Gly Thr Ala Tyr Asn Gln Lys Phe
    50                  55                  60
Lys Asp Arg Ala Thr Leu Thr Phe Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Trp Gly Leu Ile Pro Gly Thr Thr Ser Tyr Phe Asp Val Trp
            100                 105                 110
Gly Gln Gly Thr Thr Val Thr Val Ser Ser Ala Ser Thr Lys Gly Pro
        115                 120                 125
Ser Val Phe Pro Leu Ala Pro Ser Ser Lys Ser Thr Ser Gly Gly Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Lys Val Glu Pro Lys Ser
        210                 215                 220
Cys Asp Lys Thr His Thr Cys Pro Pro Cys Pro Ala Pro Glu Leu Leu
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
His Glu Asp Pro Glu Val Lys Phe Asn Trp Tyr Val Asp Gly Val Glu
            275                 280                 285
Val His Asn Ala Lys Thr Lys Pro Arg Glu Glu Gln Tyr Asn Ser Thr
        290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Arg Asp Glu Leu Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
```

```
                        405                    410                    415
        Val Asp Lys Ser Arg Trp Gln Gln Gly Asn Val Phe Ser Cys Ser Val
                        420                    425                    430
        Met His Glu Ala Leu His Asn His Tyr Thr Gln Lys Ser Leu Ser Leu
                        435                    440                    445
        Ser Pro Gly Lys
                        450


        <210>   90
        <211>   452
        <212>   PRT
        <213>   Artificial Sequence

        <220>
        <223>   Hu95-31wk   heavy chain


        <400>   90
        Glu Val Gln Leu Val Gln Ser Gly Ala Glu Val Lys Lys Pro Gly Ala
        1                   5                   10                  15
        Ser Val Lys Val Ser Cys Lys Ala Ser Gly Phe Thr Phe Thr Asn Tyr
                        20                  25                  30
        Asp Ile His Trp Val Lys Gln Ala Pro Gly Gln Arg Leu Glu Trp Met
                        35                  40                  45
        Gly Leu Val Tyr Pro Tyr Asn Gly Gly Thr Ala Tyr Asn Gln Lys Phe
                50                  55                  60
        Lys Asp Lys Val Thr Leu Thr Phe Asp Thr Ser Ala Ser Thr Ala Tyr
        65                  70                  75                  80
        Met Glu Leu Ser Ser Leu Arg Ser Glu Asp Thr Ala Val Tyr Tyr Cys
                        85                  90                  95
        Ala Arg Trp Gly Leu Ile Pro Gly Thr Thr Ser Tyr Phe Asp Val Trp
                        100                 105                 110
        Gly Gln Gly Thr Thr Val Thr Val Ser Ser Ala Ser Thr Lys Gly Pro
                        115                 120                 125
        Ser Val Phe Pro Leu Ala Pro Ser Ser Lys Ser Thr Ser Gly Gly Thr
                130                 135                 140
        Ala Ala Leu Gly Cys Leu Val Lys Asp Tyr Phe Pro Glu Pro Val Thr
        145                 150                 155                 160
        Val Ser Trp Asn Ser Gly Ala Leu Thr Ser Gly Val His Thr Phe Pro
                        165                 170                 175
        Ala Val Leu Gln Ser Ser Gly Leu Tyr Ser Leu Ser Ser Val Val Thr
                        180                 185                 190
        Val Pro Ser Ser Ser Leu Gly Thr Gln Thr Tyr Ile Cys Asn Val Asn
                        195                 200                 205
        His Lys Pro Ser Asn Thr Lys Val Asp Lys Lys Val Glu Pro Lys Ser
                210                 215                 220
        Cys Asp Lys Thr His Thr Cys Pro Pro Cys Pro Ala Pro Glu Leu Leu
        225                 230                 235                 240
        Gly Gly Pro Ser Val Phe Leu Phe Pro Pro Lys Pro Lys Asp Thr Leu
                        245                 250                 255
        Met Ile Ser Arg Thr Pro Glu Val Thr Cys Val Val Val Asp Val Ser
                        260                 265                 270
        His Glu Asp Pro Glu Val Lys Phe Asn Trp Tyr Val Asp Gly Val Glu
                275                 280                 285
        Val His Asn Ala Lys Thr Lys Pro Arg Glu Glu Gln Tyr Asn Ser Thr
                290                 295                 300
        Tyr Arg Val Val Ser Val Leu Thr Val Leu His Gln Asp Trp Leu Asn
        305                 310                 315                 320
        Gly Lys Glu Tyr Lys Cys Lys Val Ser Asn Lys Ala Leu Pro Ala Pro
                        325                 330                 335
        Ile Glu Lys Thr Ile Ser Lys Ala Lys Gly Gln Pro Arg Glu Pro Gln
                        340                 345                 350
        Val Tyr Thr Leu Pro Pro Ser Arg Asp Glu Leu Thr Lys Asn Gln Val
```

```
                355                   360                   365
      Ser Leu Thr Cys Leu Val Lys Gly Phe Tyr Pro Ser Asp Ile Ala Val
          370                   375                   380
      Glu Trp Glu Ser Asn Gly Gln Pro Glu Asn Asn Tyr Lys Thr Thr Pro
      385                   390                   395                   400
      Pro Val Leu Asp Ser Asp Gly Ser Phe Phe Leu Tyr Ser Lys Leu Thr
                405                   410                   415
      Val Asp Lys Ser Arg Trp Gln Gln Gly Asn Val Phe Ser Cys Ser Val
                420                   425                   430
      Met His Glu Ala Leu His Asn His Tyr Thr Gln Lys Ser Leu Ser Leu
                435                   440                   445
      Ser Pro Gly Lys
          450


<210>    91
<211>    452
<212>    PRT
<213>    Artificial Sequence

<220>
<223>    Hu95-41wk   heavy chain


<400>    91
      Glu Val Gln Leu Val Gln Ser Gly Ala Glu Val Lys Lys Pro Gly Ala
      1               5                   10                  15
      Ser Val Lys Val Ser Cys Lys Ala Ser Gly Phe Thr Phe Thr Asn Tyr
                20                  25                  30
      Asp Ile His Trp Val Lys Gln Ala Pro Gly Gln Arg Leu Glu Trp Ile
                35                  40                  45
      Gly Leu Val Tyr Pro Tyr Asn Gly Gly Thr Ala Tyr Asn Gln Lys Phe
          50                  55                  60
      Lys Asp Lys Ala Thr Leu Thr Phe Asp Thr Ser Ala Ser Thr Ala Tyr
      65                  70                  75                  80
      Met Glu Leu Ser Ser Leu Arg Ser Glu Asp Thr Ala Val Tyr Tyr Cys
                85                  90                  95
      Ala Arg Trp Gly Leu Ile Pro Gly Thr Ser Tyr Phe Asp Val Trp
                100                 105                 110
      Gly Gln Gly Thr Thr Val Thr Val Ser Ser Ala Ser Thr Lys Gly Pro
          115                 120                 125
      Ser Val Phe Pro Leu Ala Pro Ser Ser Lys Ser Thr Ser Gly Gly Thr
          130                 135                 140
      Ala Ala Leu Gly Cys Leu Val Lys Asp Tyr Phe Pro Glu Pro Val Thr
      145                 150                 155                 160
      Val Ser Trp Asn Ser Gly Ala Leu Thr Ser Gly Val His Thr Phe Pro
                165                 170                 175
      Ala Val Leu Gln Ser Ser Gly Leu Tyr Ser Leu Ser Ser Val Val Thr
                180                 185                 190
      Val Pro Ser Ser Ser Leu Gly Thr Gln Thr Tyr Ile Cys Asn Val Asn
                195                 200                 205
      His Lys Pro Ser Asn Thr Lys Val Asp Lys Lys Val Glu Pro Lys Ser
                210                 215                 220
      Cys Asp Lys Thr His Thr Cys Pro Pro Cys Pro Ala Pro Glu Leu Leu
      225                 230                 235                 240
      Gly Gly Pro Ser Val Phe Leu Phe Pro Pro Lys Pro Lys Asp Thr Leu
                245                 250                 255
      Met Ile Ser Arg Thr Pro Glu Val Thr Cys Val Val Val Asp Val Ser
                260                 265                 270
      His Glu Asp Pro Glu Val Lys Phe Asn Trp Tyr Val Asp Gly Val Glu
                275                 280                 285
      Val His Asn Ala Lys Thr Lys Pro Arg Glu Glu Gln Tyr Asn Ser Thr
                290                 295                 300
      Tyr Arg Val Val Ser Val Leu Thr Val Leu His Gln Asp Trp Leu Asn
```

```
          305                         310                         315                         320
          Gly Lys Glu Tyr Lys Cys Lys Val Ser Asn Lys Ala Leu Pro Ala Pro
                              325                         330                         335
          Ile Glu Lys Thr Ile Ser Lys Ala Lys Gly Gln Pro Arg Glu Pro Gln
                      340                         345                         350
          Val Tyr Thr Leu Pro Pro Ser Arg Asp Glu Leu Thr Lys Asn Gln Val
                  355                         360                         365
          Ser Leu Thr Cys Leu Val Lys Gly Phe Tyr Pro Ser Asp Ile Ala Val
              370                         375                         380
          Glu Trp Glu Ser Asn Gly Gln Pro Glu Asn Asn Tyr Lys Thr Thr Pro
          385                         390                         395                         400
          Pro Val Leu Asp Ser Asp Gly Ser Phe Phe Leu Tyr Ser Lys Leu Thr
                              405                         410                         415
          Val Asp Lys Ser Arg Trp Gln Gln Gly Asn Val Phe Ser Cys Ser Val
                      420                         425                         430
          Met His Glu Ala Leu His Asn His Tyr Thr Gln Lys Ser Leu Ser Leu
                  435                         440                         445
          Ser Pro Gly Lys
              450
```

```
<210>   92
<211>   452
<212>   PRT
<213>   Artificial Sequence

<220>
<223>   Hu95-51wk   heavy chain


<400>   92
Glu Val Gln Leu Val Gln Ser Gly Ala Glu Val Lys Lys Pro Gly Ala
1               5                   10                  15
Ser Val Lys Val Ser Cys Arg Ala Ser Gly Phe Thr Phe Thr Asn Tyr
            20                  25                  30
Ala Ile His Trp Val Lys Gln Ala Pro Gly Gln Arg Leu Glu Trp Met
        35                  40                  45
Gly Leu Val Tyr Pro Tyr Thr Gly Gly Thr Ala Tyr Asn Gln Lys Phe
    50                  55                  60
Lys Asp Lys Val Thr Leu Thr Phe Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Trp Gly Met Ile Pro Gly Thr Asn Ser Tyr Phe Asp Val Trp
            100                 105                 110
Gly Gln Gly Thr Thr Val Thr Val Ser Ser Ala Ser Thr Lys Gly Pro
        115                 120                 125
Ser Val Phe Pro Leu Ala Pro Ser Ser Lys Ser Thr Ser Gly Gly Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Lys Val Glu Pro Lys Ser
    210                 215                 220
Cys Asp Lys Thr His Thr Cys Pro Pro Cys Pro Ala Pro Glu Leu Leu
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
```

```
                    260                      265                      270
         His Glu Asp Pro Glu Val Lys Phe Asn Trp Tyr Val Asp Gly Val Glu
                 275                      280                      285
         Val His Asn Ala Lys Thr Lys Pro Arg Glu Glu Gln Tyr Asn Ser Thr
             290                      295                      300
         Tyr Arg Val Val Ser Val Leu Thr Val Leu His Gln Asp Trp Leu Asn
         305                      310                      315                      320
         Gly Lys Glu Tyr Lys Cys Lys Val Ser Asn Lys Ala Leu Pro Ala Pro
                         325                      330                      335
         Ile Glu Lys Thr Ile Ser Lys Ala Lys Gly Gln Pro Arg Glu Pro Gln
                 340                      345                      350
         Val Tyr Thr Leu Pro Pro Ser Arg Asp Glu Leu Thr Lys Asn Gln Val
                 355                      360                      365
         Ser Leu Thr Cys Leu Val Lys Gly Phe Tyr Pro Ser Asp Ile Ala Val
             370                      375                      380
         Glu Trp Glu Ser Asn Gly Gln Pro Glu Asn Asn Tyr Lys Thr Thr Pro
         385                      390                      395                      400
         Pro Val Leu Asp Ser Asp Gly Ser Phe Phe Leu Tyr Ser Lys Leu Thr
                         405                      410                      415
         Val Asp Lys Ser Arg Trp Gln Gln Gly Asn Val Phe Ser Cys Ser Val
                 420                      425                      430
         Met His Glu Ala Leu His Asn His Tyr Thr Gln Lys Ser Leu Ser Leu
                 435                      440                      445
         Ser Pro Gly Lys
             450


         <210>   93
         <211>   452
         <212>   PRT
         <213>   Artificial Sequence

         <220>
         <223>   Hu95-11yk   heavy chain


         <400>   93
         Glu Val Gln Leu Val Gln Ser Gly Ala Glu Val Lys Lys Pro Gly Ala
         1               5                   10                  15
         Ser Val Lys Val Ser Cys Lys Ala Ser Gly Phe Thr Phe Thr Asn Tyr
                 20                      25                      30
         Asp Ile His Trp Val Arg Gln Ala Pro Gly Gln Arg Leu Glu Trp Met
             35                      40                      45
         Gly Leu Val Tyr Pro Tyr Asn Gly Gly Thr Ala Tyr Asn Gln Lys Phe
             50                      55                      60
         Lys Asp Arg Val Thr Leu Thr Phe Asp Thr Ser Ala Ser Thr Ala Tyr
         65                      70                      75                      80
         Met Glu Leu Ser Ser Leu Arg Ser Glu Asp Thr Ala Val Tyr Tyr Cys
                         85                      90                      95
         Ala Arg Trp Gly Leu Ile Pro Gly Thr Thr Ser Tyr Phe Asp Val Trp
                 100                     105                     110
         Gly Gln Gly Thr Thr Val Thr Val Ser Ser Ala Ser Thr Lys Gly Pro
                 115                     120                     125
         Ser Val Phe Pro Leu Ala Pro Ser Ser Lys Ser Thr Ser Gly Gly Thr
             130                     135                     140
         Ala Ala Leu Gly Cys Leu Val Lys Asp Tyr Phe Pro Glu Pro Val Thr
         145                     150                     155                     160
         Val Ser Trp Asn Ser Gly Ala Leu Thr Ser Gly Val His Thr Phe Pro
                         165                     170                     175
         Ala Val Leu Gln Ser Ser Gly Leu Tyr Ser Leu Ser Ser Val Val Thr
                 180                     185                     190
         Val Pro Ser Ser Ser Leu Gly Thr Gln Thr Tyr Ile Cys Asn Val Asn
                 195                     200                     205
         His Lys Pro Ser Asn Thr Lys Val Asp Lys Lys Val Glu Pro Lys Ser
```

```
                210                     215                     220
      Cys Asp Lys Thr His Thr Cys Pro Pro Cys Pro Ala Pro Glu Leu Leu
      225                     230                     235                     240
      Gly Gly Pro Ser Val Phe Leu Phe Pro Pro Lys Pro Lys Asp Thr Leu
                            245                     250                     255
      Tyr Ile Thr Arg Glu Pro Glu Val Thr Cys Val Val Val Asp Val Ser
                        260                     265                     270
      His Glu Asp Pro Glu Val Lys Phe Asn Trp Tyr Val Asp Gly Val Glu
                    275                     280                     285
      Val His Asn Ala Lys Thr Lys Pro Arg Glu Glu Gln Tyr Asn Ser Thr
              290                     295                     300
      Tyr Arg Val Val Ser Val Leu Thr Val Leu His Gln Asp Trp Leu Asn
      305                     310                     315                     320
      Gly Lys Glu Tyr Lys Cys Lys Val Ser Asn Lys Ala Leu Pro Ala Pro
                            325                     330                     335
      Ile Glu Lys Thr Ile Ser Lys Ala Lys Gly Gln Pro Arg Glu Pro Gln
                        340                     345                     350
      Val Tyr Thr Leu Pro Pro Ser Arg Asp Glu Leu Thr Lys Asn Gln Val
                    355                     360                     365
      Ser Leu Thr Cys Leu Val Lys Gly Phe Tyr Pro Ser Asp Ile Ala Val
                370                     375                     380
      Glu Trp Glu Ser Asn Gly Gln Pro Glu Asn Asn Tyr Lys Thr Thr Pro
      385                     390                     395                     400
      Pro Val Leu Asp Ser Asp Gly Ser Phe Phe Leu Tyr Ser Lys Leu Thr
                            405                     410                     415
      Val Asp Lys Ser Arg Trp Gln Gln Gly Asn Val Phe Ser Cys Ser Val
                        420                     425                     430
      Met His Glu Ala Leu His Asn His Tyr Thr Gln Lys Ser Leu Ser Leu
                    435                     440                     445
      Ser Pro Gly Lys
          450


      <210>   94
      <211>   452
      <212>   PRT
      <213>   Artificial Sequence

      <220>
      <223>   Hu95-21yk  heavy chain


      <400>   94
      Glu Val Gln Leu Val Gln Ser Gly Ala Glu Val Lys Lys Pro Gly Ala
      1                   5                       10                      15
      Ser Val Lys Val Ser Cys Lys Ala Ser Gly Phe Thr Phe Thr Asn Tyr
                        20                      25                      30
      Asp Ile His Trp Val Arg Gln Ala Pro Gly Gln Arg Leu Glu Trp Ile
                    35                      40                      45
      Gly Leu Val Tyr Pro Tyr Asn Gly Gly Thr Ala Tyr Asn Gln Lys Phe
                50                      55                      60
      Lys Asp Arg Ala Thr Leu Thr Phe Asp Thr Ser Ala Ser Thr Ala Tyr
      65                      70                      75                      80
      Met Glu Leu Ser Ser Leu Arg Ser Glu Asp Thr Ala Val Tyr Tyr Cys
                            85                      90                      95
      Ala Arg Trp Gly Leu Ile Pro Gly Thr Thr Ser Tyr Phe Asp Val Trp
                        100                     105                     110
      Gly Gln Gly Thr Thr Val Thr Val Ser Ser Ala Ser Thr Lys Gly Pro
                    115                     120                     125
      Ser Val Phe Pro Leu Ala Pro Ser Ser Lys Ser Thr Ser Gly Gly Thr
                130                     135                     140
      Ala Ala Leu Gly Cys Leu Val Lys Asp Tyr Phe Pro Glu Pro Val Thr
      145                     150                     155                     160
      Val Ser Trp Asn Ser Gly Ala Leu Thr Ser Gly Val His Thr Phe Pro
```

```
                              165                   170                   175
         Ala Val Leu Gln Ser Ser Gly Leu Tyr Ser Leu Ser Ser Val Val Thr
                     180                   185                   190
         Val Pro Ser Ser Leu Gly Thr Gln Thr Tyr Ile Cys Asn Val Asn
                     195                   200                   205
         His Lys Pro Ser Asn Thr Lys Val Asp Lys Lys Val Glu Pro Lys Ser
                     210                   215                   220
         Cys Asp Lys Thr His Thr Cys Pro Pro Cys Pro Ala Pro Glu Leu Leu
         225                   230                   235                   240
         Gly Gly Pro Ser Val Phe Leu Phe Pro Pro Lys Pro Lys Asp Thr Leu
                     245                   250                   255
         Tyr Ile Thr Arg Glu Pro Glu Val Thr Cys Val Val Val Asp Val Ser
                     260                   265                   270
         His Glu Asp Pro Glu Val Lys Phe Asn Trp Tyr Val Asp Gly Val Glu
                     275                   280                   285
         Val His Asn Ala Lys Thr Lys Pro Arg Glu Glu Gln Tyr Asn Ser Thr
                     290                   295                   300
         Tyr Arg Val Val Ser Val Leu Thr Val Leu His Gln Asp Trp Leu Asn
         305                   310                   315                   320
         Gly Lys Glu Tyr Lys Cys Lys Val Ser Asn Lys Ala Leu Pro Ala Pro
                     325                   330                   335
         Ile Glu Lys Thr Ile Ser Lys Ala Lys Gly Gln Pro Arg Glu Pro Gln
                     340                   345                   350
         Val Tyr Thr Leu Pro Pro Ser Arg Asp Glu Leu Thr Lys Asn Gln Val
                     355                   360                   365
         Ser Leu Thr Cys Leu Val Lys Gly Phe Tyr Pro Ser Asp Ile Ala Val
                     370                   375                   380
         Glu Trp Glu Ser Asn Gly Gln Pro Glu Asn Asn Tyr Lys Thr Thr Pro
         385                   390                   395                   400
         Pro Val Leu Asp Ser Asp Gly Ser Phe Phe Leu Tyr Ser Lys Leu Thr
                     405                   410                   415
         Val Asp Lys Ser Arg Trp Gln Gln Gly Asn Val Phe Ser Cys Ser Val
                     420                   425                   430
         Met His Glu Ala Leu His Asn His Tyr Thr Gln Lys Ser Leu Ser Leu
         435                   440                   445
         Ser Pro Gly Lys
             450
```

<210> 95
<211> 452
<212> PRT
<213> Artificial Sequence

<220>
<223> Hu95-31yk

<400> 95

```
         Glu Val Gln Leu Val Gln Ser Gly Ala Glu Val Lys Lys Pro Gly Ala
         1                   5                   10                  15
         Ser Val Lys Val Ser Cys Lys Ala Ser Gly Phe Thr Phe Thr Asn Tyr
                     20                  25                  30
         Asp Ile His Trp Val Lys Gln Ala Pro Gly Gln Arg Leu Glu Trp Met
                     35                  40                  45
         Gly Leu Val Tyr Pro Tyr Asn Gly Gly Thr Ala Tyr Asn Gln Lys Phe
                     50                  55                  60
         Lys Asp Lys Val Thr Leu Thr Phe Asp Thr Ser Ala Ser Thr Ala Tyr
         65                  70                  75                  80
         Met Glu Leu Ser Ser Leu Arg Ser Glu Asp Thr Ala Val Tyr Tyr Cys
                     85                  90                  95
         Ala Arg Trp Gly Leu Ile Pro Gly Thr Ser Tyr Phe Asp Val Trp
                     100                 105                 110
         Gly Gln Gly Thr Thr Val Thr Val Ser Ser Ala Ser Thr Lys Gly Pro
```

```
                 115                      120                      125
        Ser Val Phe Pro Leu Ala Pro Ser Ser Lys Ser Thr Ser Gly Gly Thr
            130                      135                      140
        Ala Ala Leu Gly Cys Leu Val Lys Asp Tyr Phe Pro Glu Pro Val Thr
        145                      150                      155                      160
        Val Ser Trp Asn Ser Gly Ala Leu Thr Ser Gly Val His Thr Phe Pro
                         165                      170                      175
        Ala Val Leu Gln Ser Ser Gly Leu Tyr Ser Leu Ser Ser Val Val Thr
                     180                      185                      190
        Val Pro Ser Ser Ser Leu Gly Thr Gln Thr Tyr Ile Cys Asn Val Asn
                 195                      200                      205
        His Lys Pro Ser Asn Thr Lys Val Asp Lys Lys Val Glu Pro Lys Ser
            210                      215                      220
        Cys Asp Lys Thr His Thr Cys Pro Pro Cys Pro Ala Pro Glu Leu Leu
        225                      230                      235                      240
        Gly Gly Pro Ser Val Phe Leu Phe Pro Pro Lys Pro Lys Asp Thr Leu
                         245                      250                      255
        Tyr Ile Thr Arg Glu Pro Glu Val Thr Cys Val Val Val Asp Val Ser
                     260                      265                      270
        His Glu Asp Pro Glu Val Lys Phe Asn Trp Tyr Val Asp Gly Val Glu
                 275                      280                      285
        Val His Asn Ala Lys Thr Lys Pro Arg Glu Glu Gln Tyr Asn Ser Thr
            290                      295                      300
        Tyr Arg Val Val Ser Val Leu Thr Val Leu His Gln Asp Trp Leu Asn
        305                      310                      315                      320
        Gly Lys Glu Tyr Lys Cys Lys Val Ser Asn Lys Ala Leu Pro Ala Pro
                         325                      330                      335
        Ile Glu Lys Thr Ile Ser Lys Ala Lys Gly Gln Pro Arg Glu Pro Gln
                     340                      345                      350
        Val Tyr Thr Leu Pro Pro Ser Arg Asp Glu Leu Thr Lys Asn Gln Val
                 355                      360                      365
        Ser Leu Thr Cys Leu Val Lys Gly Phe Tyr Pro Ser Asp Ile Ala Val
            370                      375                      380
        Glu Trp Glu Ser Asn Gly Gln Pro Glu Asn Asn Tyr Lys Thr Thr Pro
        385                      390                      395                      400
        Pro Val Leu Asp Ser Asp Gly Ser Phe Phe Leu Tyr Ser Lys Leu Thr
                         405                      410                      415
        Val Asp Lys Ser Arg Trp Gln Gln Gly Asn Val Phe Ser Cys Ser Val
                     420                      425                      430
        Met His Glu Ala Leu His Asn His Tyr Thr Gln Lys Ser Leu Ser Leu
            435                      440                      445
        Ser Pro Gly Lys
            450


        <210>   96
        <211>   452
        <212>   PRT
        <213>   Artificial Sequence

        <220>
        <223>   Hu95-41yk   heavy chain


        <400>   96
        Glu Val Gln Leu Val Gln Ser Gly Ala Glu Val Lys Lys Pro Gly Ala
        1                 5                       10                      15
        Ser Val Lys Val Ser Cys Lys Ala Ser Gly Phe Thr Phe Thr Asn Tyr
                     20                      25                      30
        Asp Ile His Trp Val Lys Gln Ala Pro Gly Gln Arg Leu Glu Trp Ile
                 35                      40                      45
        Gly Leu Val Tyr Pro Tyr Asn Gly Gly Thr Ala Tyr Asn Gln Lys Phe
            50                      55                      60
        Lys Asp Lys Ala Thr Leu Thr Phe Asp Thr Ser Ala Ser Thr Ala Tyr
```

```
                65                            70                            75                            80
       Met Glu Leu Ser Ser Leu Arg Ser Glu Asp Thr Ala Val Tyr Tyr Cys
                         85                            90                            95
       Ala Arg Trp Gly Leu Ile Pro Gly Thr Thr Ser Tyr Phe Asp Val Trp
                    100                           105                           110
       Gly Gln Gly Thr Thr Val Thr Val Ser Ser Ala Ser Thr Lys Gly Pro
                115                           120                           125
       Ser Val Phe Pro Leu Ala Pro Ser Ser Lys Ser Thr Ser Gly Gly Thr
           130                           135                           140
       Ala Ala Leu Gly Cys Leu Val Lys Asp Tyr Phe Pro Glu Pro Val Thr
       145                           150                           155                           160
       Val Ser Trp Asn Ser Gly Ala Leu Thr Ser Gly Val His Thr Phe Pro
                         165                           170                           175
       Ala Val Leu Gln Ser Ser Gly Leu Tyr Ser Leu Ser Ser Val Val Thr
                    180                           185                           190
       Val Pro Ser Ser Ser Leu Gly Thr Gln Thr Tyr Ile Cys Asn Val Asn
                195                           200                           205
       His Lys Pro Ser Asn Thr Lys Val Asp Lys Lys Val Glu Pro Lys Ser
           210                           215                           220
       Cys Asp Lys Thr His Thr Cys Pro Pro Cys Pro Ala Pro Glu Leu Leu
       225                           230                           235                           240
       Gly Gly Pro Ser Val Phe Leu Phe Pro Pro Lys Pro Lys Asp Thr Leu
                         245                           250                           255
       Tyr Ile Thr Arg Glu Pro Glu Val Thr Cys Val Val Val Asp Val Ser
                    260                           265                           270
       His Glu Asp Pro Glu Val Lys Phe Asn Trp Tyr Val Asp Gly Val Glu
                275                           280                           285
       Val His Asn Ala Lys Thr Lys Pro Arg Glu Glu Gln Tyr Asn Ser Thr
           290                           295                           300
       Tyr Arg Val Val Ser Val Leu Thr Val Leu His Gln Asp Trp Leu Asn
       305                           310                           315                           320
       Gly Lys Glu Tyr Lys Cys Lys Val Ser Asn Lys Ala Leu Pro Ala Pro
                         325                           330                           335
       Ile Glu Lys Thr Ile Ser Lys Ala Lys Gly Gln Pro Arg Glu Pro Gln
                    340                           345                           350
       Val Tyr Thr Leu Pro Pro Ser Arg Asp Glu Leu Thr Lys Asn Gln Val
                355                           360                           365
       Ser Leu Thr Cys Leu Val Lys Gly Phe Tyr Pro Ser Asp Ile Ala Val
           370                           375                           380
       Glu Trp Glu Ser Asn Gly Gln Pro Glu Asn Asn Tyr Lys Thr Thr Pro
       385                           390                           395                           400
       Pro Val Leu Asp Ser Asp Gly Ser Phe Phe Leu Tyr Ser Lys Leu Thr
                         405                           410                           415
       Val Asp Lys Ser Arg Trp Gln Gln Gly Asn Val Phe Ser Cys Ser Val
                    420                           425                           430
       Met His Glu Ala Leu His Asn His Tyr Thr Gln Lys Ser Leu Ser Leu
                435                           440                           445
       Ser Pro Gly Lys
           450
```

```
<210>   97
<211>   452
<212>   PRT
<213>   Artificial Sequence

<220>
<223>   Hu95-51yk   heavy chain


<400>   97
Glu Val Gln Leu Val Gln Ser Gly Ala Glu Val Lys Lys Pro Gly Ala
1                   5                   10                  15
Ser Val Lys Val Ser Cys Arg Ala Ser Gly Phe Thr Phe Thr Asn Tyr
```

```
                    20                        25                        30
        Ala Ile His Trp Val Lys Gln Ala Pro Gly Gln Arg Leu Glu Trp Met
                35                        40                        45
        Gly Leu Val Tyr Pro Tyr Thr Gly Gly Thr Ala Tyr Asn Gln Lys Phe
                50                        55                        60
        Lys Asp Lys Val Thr Leu Thr Phe Asp Thr Ser Ala Ser Thr Ala Tyr
        65                        70                        75                        80
        Met Glu Leu Ser Ser Leu Arg Ser Glu Asp Thr Ala Val Tyr Tyr Cys
                        85                        90                        95
        Ala Arg Trp Gly Met Ile Pro Gly Thr Asn Ser Tyr Phe Asp Val Trp
                    100                       105                       110
        Gly Gln Gly Thr Thr Val Thr Val Ser Ser Ala Ser Thr Lys Gly Pro
                115                       120                       125
        Ser Val Phe Pro Leu Ala Pro Ser Ser Lys Ser Thr Ser Gly Gly Thr
                130                       135                       140
        Ala Ala Leu Gly Cys Leu Val Lys Asp Tyr Phe Pro Glu Pro Val Thr
        145                       150                       155                       160
        Val Ser Trp Asn Ser Gly Ala Leu Thr Ser Gly Val His Thr Phe Pro
                        165                       170                       175
        Ala Val Leu Gln Ser Ser Gly Leu Tyr Ser Leu Ser Ser Val Val Thr
                    180                       185                       190
        Val Pro Ser Ser Ser Leu Gly Thr Gln Thr Tyr Ile Cys Asn Val Asn
                195                       200                       205
        His Lys Pro Ser Asn Thr Lys Val Asp Lys Lys Val Glu Pro Lys Ser
                210                       215                       220
        Cys Asp Lys Thr His Thr Cys Pro Pro Cys Pro Ala Pro Glu Leu Leu
        225                       230                       235                       240
        Gly Gly Pro Ser Val Phe Leu Phe Pro Pro Lys Pro Lys Asp Thr Leu
                        245                       250                       255
        Tyr Ile Thr Arg Glu Pro Glu Val Thr Cys Val Val Val Asp Val Ser
                    260                       265                       270
        His Glu Asp Pro Glu Val Lys Phe Asn Trp Tyr Val Asp Gly Val Glu
                275                       280                       285
        Val His Asn Ala Lys Thr Lys Pro Arg Glu Glu Gln Tyr Asn Ser Thr
                290                       295                       300
        Tyr Arg Val Val Ser Val Leu Thr Val Leu His Gln Asp Trp Leu Asn
        305                       310                       315                       320
        Gly Lys Glu Tyr Lys Cys Lys Val Ser Asn Lys Ala Leu Pro Ala Pro
                        325                       330                       335
        Ile Glu Lys Thr Ile Ser Lys Ala Lys Gly Gln Pro Arg Glu Pro Gln
                    340                       345                       350
        Val Tyr Thr Leu Pro Pro Ser Arg Asp Glu Leu Thr Lys Asn Gln Val
                355                       360                       365
        Ser Leu Thr Cys Leu Val Lys Gly Phe Tyr Pro Ser Asp Ile Ala Val
                370                       375                       380
        Glu Trp Glu Ser Asn Gly Gln Pro Glu Asn Asn Tyr Lys Thr Thr Pro
        385                       390                       395                       400
        Pro Val Leu Asp Ser Asp Gly Ser Phe Phe Leu Tyr Ser Lys Leu Thr
                        405                       410                       415
        Val Asp Lys Ser Arg Trp Gln Gln Gly Asn Val Phe Ser Cys Ser Val
                    420                       425                       430
        Met His Glu Ala Leu His Asn His Tyr Thr Gln Lys Ser Leu Ser Leu
                435                       440                       445
        Ser Pro Gly Lys
                450
```

```
<210>   98
<211>   213
<212>   PRT
<213>   Artificial Sequence

<220>
<223>   Hu95-11wk light chain
```

<400> 98

```
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Ser Ser Val Ser Tyr Ile
            20                  25                  30
His Trp Tyr Gln Gln Lys Pro Gly Gln Ala Pro Arg Pro Trp Ile Tyr
        35                  40                  45
Ala Thr Ser Asn Leu Ala Ser Gly Ile Pro Ala Arg Phe Ser Gly Ser
        50                  55                  60
Gly Ser Gly Thr Asp Tyr Thr Leu Thr Ile Ser Arg Leu Glu Pro Glu
65                  70                  75                  80
Asp Phe Ala Val Tyr Tyr Cys Gln Gln Trp Asn Ser Asn Pro Trp Thr
                85                  90                  95
Phe Gly Gly Gly Thr Lys Val Glu Ile Lys Arg Thr Val Ala Ala Pro
            100                 105                 110
Ser Val Phe Ile Phe Pro Pro Ser Asp Glu Gln Leu Lys Ser Gly Thr
            115                 120                 125
Ala Ser Val Val Cys Leu Leu Asn Asn Phe Tyr Pro Arg Glu Ala Lys
        130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
            195                 200                 205
Asn Arg Gly Glu Cys
            210
```

<210> 99
<211> 213
<212> PRT
<213> Artificial Sequence

<220>
<223> Hu95-12wk light chain

<400> 99

```
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Ser Ser Val Ser Tyr Ile
            20                  25                  30
His Trp Tyr Gln Gln Lys Pro Gly Gln Ser Pro Arg Pro Trp Ile Tyr
        35                  40                  45
Ala Thr Ser Asn Leu Ala Ser Gly Val Pro Ala Arg Phe Ser Gly Ser
        50                  55                  60
Gly Ser Gly Thr Ser Tyr Thr Leu Thr Ile Ser Arg Leu Glu Pro Glu
65                  70                  75                  80
Asp Phe Ala Val Tyr Tyr Cys Gln Gln Trp Asn Ser Asn Pro Trp Thr
                85                  90                  95
Phe Gly Gly Gly Thr Lys Val Glu Ile Lys Arg Thr Val Ala Ala Pro
            100                 105                 110
Ser Val Phe Ile Phe Pro Pro Ser Asp Glu Gln Leu Lys Ser Gly Thr
            115                 120                 125
Ala Ser Val Val Cys Leu Leu Asn Asn Phe Tyr Pro Arg Glu Ala Lys
        130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
```

```
                       165                  170                  175
      Thr Leu Thr Leu Ser Lys Ala Asp Tyr Glu Lys His Lys Val Tyr Ala
                  180                  185                  190
      Cys Glu Val Thr His Gln Gly Leu Ser Ser Pro Val Thr Lys Ser Phe
                  195                  200                  205
      Asn Arg Gly Glu Cys
          210


<210>  100
<211>  212
<212>  PRT
<213>  Artificial Sequence

<220>
<223>  Hu95-13wk light chain


<400>  100
Glu Ser Val Leu Thr Gln Pro Pro Ser Val Ser Gly Ala Pro Gly Gln
1               5                   10                  15
Arg Val Thr Ile Ser Cys Arg Ala Ser Ser Ser Val Ser Tyr Ile His
              20                  25                  30
Trp Tyr Gln Gln Leu Pro Gly Thr Ala Pro Lys Pro Trp Ile Tyr Ala
              35                  40                  45
Thr Ser Asn Leu Ala Ser Gly Val Pro Asp Arg Phe Ser Gly Ser Lys
          50                  55                  60
Ser Gly Thr Ser Tyr Ser Leu Ala Ile Thr Gly Leu Gln Ala Glu Asp
65                  70                  75                  80
Glu Ala Asp Tyr Tyr Cys Gln Gln Trp Asn Ser Asn Pro Trp Thr Phe
                  85                  90                  95
Gly Gly Gly Thr Lys Val Glu Ile Lys Arg Thr Val Ala Ala Pro Ser
              100                 105                 110
Val Phe Ile Phe Pro Pro Ser Asp Glu Gln Leu Lys Ser Gly Thr Ala
          115                 120                 125
Ser Val Val Cys Leu Leu Asn Asn Phe Tyr Pro Arg Glu Ala Lys Val
      130                 135                 140
Gln Trp Lys Val Asp Asn Ala Leu Gln Ser Gly Asn Ser Gln Glu Ser
145                 150                 155                 160
Val Thr Glu Gln Asp Ser Lys Asp Ser Thr Tyr Ser Leu Ser Ser Thr
                  165                 170                 175
Leu Thr Leu Ser Lys Ala Asp Tyr Glu Lys His Lys Val Tyr Ala Cys
              180                 185                 190
Glu Val Thr His Gln Gly Leu Ser Ser Pro Val Thr Lys Ser Phe Asn
          195                 200                 205
Arg Gly Glu Cys
      210


<210>  101
<211>  212
<212>  PRT
<213>  Artificial Sequence

<220>
<223>  Hu95-14wk light chain


<400>  101
Glu Ile Val Leu Thr Gln Pro Pro Ser Val Ser Gly Ala Pro Gly Gln
1               5                   10                  15
Arg Val Thr Ile Ser Cys Arg Ala Ser Ser Ser Val Ser Tyr Ile His
              20                  25                  30
Trp Tyr Gln Gln Leu Pro Gly Thr Ser Pro Lys Pro Trp Ile Tyr Ala
              35                  40                  45
```

```
Thr Ser Asn Leu Ala Ser Gly Val Pro Asp Arg Phe Ser Gly Ser Lys
    50                  55              60
Ser Gly Thr Ser Tyr Ser Leu Ala Ile Thr Gly Leu Gln Ala Glu Asp
65              70              75                  80
Glu Ala Asp Tyr Tyr Cys Gln Gln Trp Asn Ser Asn Pro Trp Thr Phe
            85              90                  95
Gly Gly Gly Thr Lys Val Glu Ile Lys Arg Thr Val Ala Ala Pro Ser
        100             105                 110
Val Phe Ile Phe Pro Pro Ser Asp Glu Gln Leu Lys Ser Gly Thr Ala
    115             120                 125
Ser Val Val Cys Leu Leu Asn Asn Phe Tyr Pro Arg Glu Ala Lys Val
    130             135                 140
Gln Trp Lys Val Asp Asn Ala Leu Gln Ser Gly Asn Ser Gln Glu Ser
145             150                 155                 160
Val Thr Glu Gln Asp Ser Lys Asp Ser Thr Tyr Ser Leu Ser Ser Thr
                165                 170                 175
Leu Thr Leu Ser Lys Ala Asp Tyr Glu Lys His Lys Val Tyr Ala Cys
            180                 185                 190
Glu Val Thr His Gln Gly Leu Ser Ser Pro Val Thr Lys Ser Phe Asn
            195                 200                 205
Arg Gly Glu Cys
        210
```

```
<210>  102
<211>  5
<212>  PRT
<213>  Artificial Sequence

<220>
<223>  Hu95-VH5 HCDR1


<400>  102
Asn Tyr Ala Ile His
1               5


<210>  103
<211>  17
<212>  PRT
<213>  Artificial Sequence

<220>
<223>  Hu95-VH5 HCDR2


<400>  103
Leu Val Tyr Pro Tyr Thr Gly Gly Thr Ala Tyr Asn Gln Lys Phe Lys
1               5                   10                  15
Asp


<210>  104
<211>  13
<212>  PRT
<213>  Artificial Sequence

<220>
<223>  Hu95-VH5 HCDR3


<400>  104
Trp Gly Met Ile Pro Gly Thr Asn Ser Tyr Phe Asp Val
1               5                   10
```

111